# EUROPEAN PATENT APPLICATION

(11) **EP 1 491 213 A1**
(43) Date of publication of application: **29.12.2004**
(21) Application number: 03715671.8
(22) Date of filing: 31.03.2003
(51) Int. Cl.: A61K 45/00, A61K 38/17, A61P 25/00

(54) **C-JUN PHOSPHORYLATION INHIBITORS**

(30) Priority: 29.03.2002 JP 2002095291; 29.03.2002 JP 2002095390; 29.03.2002 JP 2002095442; 29.03.2002 JP 2002095486
(71) Applicant: Celestar Lexico-Sciences, Inc., Chiba-shi, Chiba 261-8501 (JP); DAIICHI PHARMACEUTICAL CO., LTD., Chuo-ku, Tokyo 103-8234 (JP)
(72) Inventor: DOI, Hirofumi, c/o Celestar Lexico-Sciences, Inc., Chiba-shi, Chiba 261-8501 (JP); WADA, Naoya, c/o Daiichi Pharmaceutical Co., Ltd., Tokyo 134-8630 (JP); NAKAJIMA, Hiroto, c/o DAIICHI PHARMA. CO., LTD., Tokyo 134-8630 (JP)
(74) Representative: Forstmeyer, Dietmar, Dr. rer. nat., Dipl.-Chem.
(86) International application number: PCT/JP2003/004120
(87) International publication number: WO 2003/086462

(57) **Abstract**

In the present invention, BMAL1, BPL1, KIAA1491 complete (SEQ ID NO: 1), and KIAA0596CT (SEQ ID NO:2), have a function for interacting with JNK3, wherein these peptides, as well as peptides derived therefrom can be used to inhibit c-Jun from being phosphorylated and exhibiting its functions (such as transcription activation activity). Inhibition of the phosphorylation of c-Jun suppresses apoptosis, such as nerve cell apoptosis. Thus, the present invention also provides a drug, a pharmaceutical composition and a method for use in preventing and/or treating a neurodegenerative disease (such as polyglutamine disease and Alzheimer's disease).

SEQUENCE LISTING

<110> CELESTAR LEXICO-SCIENCES, INC.
DAIICHI PHARMACEUTICAL CO., LTD.

<120> c-Jun phosphorylation inhibitor

<130> GP03-1004PCT

<150> JP P2002-095291
<151> 2002-03-29

<150> JP P2002-095390
<151> 2002-03-29

<150> JP P2002-095442
<151> 2002-03-29

<150> JP P2002-095486
<151> 2002-03-29

<160> 90

<170> PatentIn version 3.1

<210> 1
<211> 786
<212> PRT
<213> Homo sapiens

<400> 1

Met Ala Pro Gly Thr Gly Ser Ser Thr Ala Val Asn Ser Cys Ser Pro
1               5                   10                  15

Gln Ser Leu Ser Ser Val Leu Gly Ser Gly Phe Gly Glu Leu Ala Pro
            20                  25                  30

Pro Lys Met Ala Asn Ile Thr Ser Ser Gln Ile Leu Asp Gln Leu Lys
        35                  40                  45

Ala Pro Ser Leu Gly Gln Phe Thr Thr Thr Pro Ser Thr Gln Gln Asn
    50                  55                  60

Ser Thr Ser His Pro Thr Thr Thr Thr Ser Trp Asp Leu Lys Pro Pro
65                  70                  75                  80

Thr Ser Gln Ser Ser Val Leu Ser His Leu Asp Phe Lys Ser Gln Pro
                85                  90                  95

Glu Pro Ser Pro Val Leu Ser Gln Leu Ser Gln Arg Gln Gln His Gln
            100                 105                 110

Ser Gln Ala Val Thr Val Pro Pro Pro Gly Leu Glu Ser Phe Pro Ser
        115                 120                 125

Gln Ala Lys Leu Arg Glu Ser Thr Pro Gly Asp Ser Pro Ser Thr Val
    130                 135                 140

Asn Lys Leu Leu Gln Leu Pro Ser Thr Thr Ile Glu Asn Ile Ser Val
145                 150                 155                 160

Ser Val His Gln Pro Gln Pro Lys His Ile Lys Leu Ala Lys Arg Arg
                165                 170                 175

Ile Pro Pro Ala Ser Lys Ile Pro Ala Ser Ala Val Glu Met Pro Gly
            180                 185                 190

Ser Ala Asp Val Thr Gly Leu Asn Val Gln Phe Gly Ala Leu Glu Phe
        195                 200                 205

Gly Ser Glu Pro Ser Leu Ser Glu Phe Gly Ser Ala Pro Ser Ser Glu
    210                 215                 220

Asn Ser Asn Gln Ile Pro Ile Ser Leu Tyr Ser Lys Ser Leu Ser Glu
225                 230                 235                 240

Pro Leu Asn Thr Ser Leu Ser Met Thr Ser Ala Val Gln Asn Ser Thr
                245                 250                 255

Tyr Thr Thr Ser Val Ile Thr Ser Cys Ser Leu Thr Ser Ser Ser Leu
            260                 265                 270

Asn Ser Ala Ser Pro Val Ala Met Ser Ser Ser Tyr Asp Gln Ser Ser
        275                 280                 285

Val His Asn Arg Ile Pro Tyr Gln Ser Pro Val Ser Ser Ser Glu Ser
    290                 295                 300

Ala Pro Gly Thr Ile Met Asn Gly His Gly Gly Gly Arg Ser Gln Gln
305                 310                 315                 320

Thr Leu Asp Thr Pro Lys Thr Thr Gly Pro Pro Ser Ala Leu Pro Ser
                325                 330                 335

Val Ser Ser Leu Pro Ser Thr Thr Ser Cys Thr Ala Leu Leu Pro Ser
            340                 345                 350

Thr Ser Gln His Thr Gly Asp Leu Thr Ser Ser Pro Leu Ser Gln Leu
        355                 360                 365

Ser Ser Ser Leu Ser Ser His Gln Ser Ser Leu Ser Ala His Ala Ala
    370                 375                 380

Leu Ser Ser Ser Thr Ser His Thr His Ala Ser Val Glu Ser Ala Ser
385                 390                 395                 400

Ser His Gln Ser Ser Ala Thr Phe Ser Thr Ala Ala Thr Ser Val Ser
                405                 410                 415

Ser Ser Ala Ser Ser Gly Val Ser Leu Ser Ser Ser Met Asn Thr Ala
            420                 425                 430

Asn Ser Leu Cys Leu Gly Gly Thr Pro Ala Ser Ala Ser Ser Ser Ser
        435                 440                 445

Ser Arg Ala Ala Pro Leu Val Thr Ser Gly Lys Ala Pro Pro Asn Leu
    450                 455                 460

Pro Gln Gly Val Pro Pro Leu Leu His Asn Gln Tyr Leu Val Gly Pro
465                 470                 475                 480

Gly Gly Leu Leu Pro Ala Tyr Pro Ile Tyr Gly Tyr Asp Glu Leu Gln
                485                 490                 495

Met Leu Gln Ser Arg Leu Pro Val Asp Tyr Tyr Gly Ile Pro Phe Ala
            500                 505                 510

Ala Pro Thr Ala Leu Ala Ser Arg Asp Arg Ser Leu Ala Asn Asn Pro
        515                 520                 525

Tyr Pro Gly Asp Val Thr Lys Phe Gly Arg Gly Asp Ser Ala Ser Pro
    530                 535                 540

Ala Pro Ala Thr Thr Pro Ala Gln Pro Gln Gln Ser Gln Ser Gln Thr
545                 550                 555                 560

His His Thr Ala Gln Gln Pro Phe Val Asn Pro Ala Leu Pro Pro Gly
                565                 570                 575

Tyr Ser Tyr Thr Gly Leu Pro Tyr Tyr Thr Gly Met Pro Ser Ala Phe
            580                 585                 590

Gln Tyr Gly Pro Thr Met Phe Val Pro Pro Ala Ser Ala Lys Gln His
        595                 600                 605

Gly Val Asn Leu Ser Thr Pro Thr Pro Pro Phe Gln Gln Ala Ser Gly
    610                 615                 620

Tyr Gly Gln His Gly Tyr Ser Thr Gly Tyr Asp Asp Leu Thr Gln Gly

625                 630                 635                 640

Thr Ala Ala Gly Asp Tyr Ser Lys Gly Gly Tyr Ala Gly Ser Ser Gln
                645                 650                 655

Ala Pro Asn Lys Ser Ala Gly Ser Gly Pro Gly Lys Gly Val Ser Val
            660                 665                 670

Ser Ser Ser Thr Thr Gly Leu Pro Asp Met Thr Gly Ser Val Tyr Asn
        675                 680                 685

Lys Thr Gln Thr Phe Asp Lys Gln Gly Phe His Ala Gly Thr Pro Pro
    690                 695                 700

Pro Phe Ser Leu Pro Ser Val Leu Gly Ser Thr Gly Pro Leu Ala Ser
705                 710                 715                 720

Gly Ala Ala Pro Gly Tyr Ala Pro Pro Pro Phe Leu His Ile Leu Pro
                725                 730                 735

Ala His Gln Gln Pro His Ser Gln Leu Leu His His His Leu Pro Gln
            740                 745                 750

Asp Ala Gln Ser Gly Ser Gly Gln Arg Ser Gln Pro Ser Ser Leu Gln
        755                 760                 765

Pro Lys Ser Gln Ala Ser Lys Pro Ala Tyr Gly Asn Ser Pro Tyr Trp
    770                 775                 780

Thr Asn
785

<210>  2
<211>  579
<212>  PRT
<213>  Homo sapiens

<400>  2

Leu Ser Gln Glu Glu Gly Val Phe Ala Gln Asp Leu Glu Pro Ala Pro
1               5                   10                  15

Ile Glu Asp Gly Ile Val Tyr Pro Glu Pro Ser Asp Asn Pro Thr Met
            20                  25                  30

Asp Thr Ser Glu Phe Gln Val Gln Ala Pro Ala Arg Gly Thr Leu Gly
        35                  40                  45

Arg Val Tyr Pro Gly Ser Arg Ser Ser Glu Lys His Ser Pro Asp Ser

    50                  55                  60

Ala Cys Ser Val Asp Tyr Ser Ser Ser Cys Leu Ser Ser Pro Glu His
65                  70                  75                  80

Pro Thr Glu Asp Ser Glu Ser Thr Glu Pro Leu Ser Val Asp Gly Ile
                85                  90                  95

Ser Ser Asp Leu Glu Glu Pro Ala Glu Gly Asp Glu Glu Glu Glu Glu
            100                 105                 110

Glu Glu Gly Gly Met Gly Pro Tyr Gly Leu Gln Glu Gly Ser Pro Gln
        115                 120                 125

Thr Pro Asp Gln Glu Gln Phe Leu Lys Gln His Phe Glu Thr Leu Ala
    130                 135                 140

Ser Gly Ala Ala Pro Gly Ala Pro Val Gln Val Pro Glu Arg Ser Glu
145                 150                 155                 160

Ser Arg Ser Ile Ser Ser Arg Phe Leu Leu Gln Val Gln Thr Arg Pro
                165                 170                 175

Leu Arg Glu Pro Ser Pro Ser Ser Ser Ser Leu Ala Leu Met Ser Arg
            180                 185                 190

Pro Ala Gln Val Pro Gln Ala Ser Gly Glu Gln Pro Arg Gly Asn Gly
        195                 200                 205

Ala Asn Pro Pro Gly Ala Pro Pro Glu Val Glu Pro Ser Ser Gly Asn
    210                 215                 220

Pro Ser Pro Gln Gln Ala Ala Ser Val Leu Leu Pro Arg Cys Arg Leu
225                 230                 235                 240

Asn Pro Asp Ser Ser Trp Ala Pro Lys Arg Val Ala Thr Ala Ser Pro
                245                 250                 255

Phe Ser Gly Leu Gln Lys Ala Gln Ser Val His Ser Leu Val Pro Gln
            260                 265                 270

Glu Arg His Glu Ala Ser Leu Gln Ala Pro Ser Pro Gly Ala Leu Leu
        275                 280                 285

Ser Arg Glu Ile Glu Ala Gln Asp Gly Leu Gly Ser Leu Pro Pro Ala
    290                 295                 300

Asp Gly Pro Pro Ser Arg Pro His Ser Tyr Gln Asn Pro Thr Thr Ser
305                 310                 315                 320

Ser Met Ala Lys Ile Ser Arg Ser Ile Ser Val Gly Glu Asn Leu Gly
                325                 330                 335

Leu Val Ala Glu Pro Gln Ala His Ala Pro Ile Arg Val Ser Pro Leu
            340                 345                 350

Ser Lys Leu Ala Leu Pro Ser Arg Ala His Leu Val Leu Asp Ile Pro
        355                 360                 365

Lys Pro Leu Pro Asp Arg Pro Thr Leu Ala Ala Phe Ser Pro Val Thr
    370                 375                 380

Lys Gly Arg Ala Pro Gly Glu Ala Glu Lys Pro Gly Phe Pro Val Gly
385                 390                 395                 400

Leu Gly Lys Ala His Ser Thr Thr Glu Arg Trp Ala Cys Leu Gly Glu
                405                 410                 415

Gly Thr Thr Pro Lys Pro Arg Thr Glu Cys Gln Ala His Pro Gly Pro
            420                 425                 430

Ser Ser Pro Cys Ala Gln Gln Leu Pro Val Ser Ser Leu Phe Gln Gly
        435                 440                 445

Pro Glu Asn Leu Gln Pro Pro Pro Pro Glu Lys Thr Pro Asn Pro Met
    450                 455                 460

Glu Cys Thr Lys Pro Gly Ala Ala Leu Ser Gln Asp Ser Ala Val Ser
465                 470                 475                 480

Leu Glu Gln Cys Glu Gln Leu Val Ala Glu Leu Arg Gly Ser Val Arg
                485                 490                 495

Gln Ala Val Arg Leu Tyr His Ser Val Ala Gly Cys Lys Met Pro Ser
            500                 505                 510

Ala Glu Gln Ser Arg Ile Ala Gln Leu Leu Arg Asp Thr Phe Ser Ser
        515                 520                 525

Val Arg Gln Glu Leu Glu Ala Val Ala Gly Ala Val Leu Ser Ser Pro
    530                 535                 540

Gly Ser Ser Pro Gly Ala Val Gly Ala Glu Gln Thr Gln Ala Leu Leu
545                 550                 555                 560

Glu Gln Tyr Ser Glu Leu Leu Leu Arg Ala Val Glu Arg Arg Met Glu
                565                 570                 575

Arg Lys Leu

<210> 3
<211> 1217
<212> PRT
<213> Homo sapiens

<400> 3

Ser Asn Lys Val Ser Ser Arg Val Thr Ala Val Ser Phe Ser Glu Asp
1               5                   10                  15

Cys Ser Tyr Phe Val Thr Ala Gly Asn Arg His Ile Lys Phe Trp Tyr
            20                  25                  30

Leu Asp Asp Ser Lys Thr Ser Lys Val Asn Ala Thr Val Pro Leu Leu
        35                  40                  45

Gly Arg Ser Gly Leu Leu Gly Glu Leu Arg Asn Asn Leu Phe Thr Asp
    50                  55                  60

Val Ala Cys Gly Arg Gly Lys Lys Ala Asp Ser Thr Phe Cys Ile Thr
65                  70                  75                  80

Ser Ser Gly Leu Leu Cys Glu Phe Ser Asp Arg Arg Leu Leu Asp Lys
                85                  90                  95

Trp Val Glu Leu Arg Val Tyr Pro Glu Val Lys Asp Ser Asn Gln Ala
            100                 105                 110

Cys Leu Pro Pro Ser Ser Phe Ile Thr Cys Ser Ser Asp Asn Thr Ile
        115                 120                 125

Arg Leu Trp Asn Thr Glu Ser Ser Gly Val His Gly Ser Thr Leu His
    130                 135                 140

Arg Asn Ile Leu Ser Ser Asp Leu Ile Lys Ile Ile Tyr Val Asp Gly
145                 150                 155                 160

Asn Thr Gln Ala Leu Leu Asp Thr Glu Leu Pro Gly Gly Asp Lys Ala
                165                 170                 175

Asp Ala Ser Leu Leu Asp Pro Arg Val Gly Ile Arg Ser Val Cys Val
            180                 185                 190

Ser Pro Asn Gly Gln His Leu Ala Ser Gly Asp Arg Met Gly Thr Leu
        195                 200                 205

Arg Val His Glu Leu Gln Ser Leu Ser Glu Met Leu Lys Val Glu Ala
    210                 215                 220

His Asp Ser Glu Ile Leu Cys Leu Glu Tyr Ser Lys Pro Asp Thr Gly
225                 230                 235                 240

Leu Lys Leu Leu Ala Ser Ala Ser Arg Asp Arg Leu Ile His Val Leu
                245                 250                 255

Asp Ala Gly Arg Glu Tyr Ser Leu Gln Gln Thr Leu Asp Glu His Ser
            260                 265                 270

Ser Ser Ile Thr Ala Val Lys Phe Ala Ala Ser Asp Gly Gln Val Arg
        275                 280                 285

Met Ile Ser Cys Gly Ala Asp Lys Ser Ile Tyr Phe Arg Thr Ala Gln
    290                 295                 300

Lys Ser Gly Asp Gly Val Gln Phe Thr Arg Thr His His Val Val Arg
305                 310                 315                 320

Lys Thr Thr Leu Tyr Asp Met Asp Val Glu Pro Ser Trp Lys Tyr Thr
                325                 330                 335

Ala Ile Gly Cys Gln Asp Arg Asn Ile Arg Ile Phe Asn Ile Ser Ser
            340                 345                 350

Gly Lys Gln Lys Lys Leu Phe Lys Gly Ser Gln Gly Glu Asp Gly Thr
        355                 360                 365

Leu Ile Lys Val Gln Thr Asp Pro Ser Gly Ile Tyr Ile Ala Thr Ser
    370                 375                 380

Cys Ser Asp Lys Asn Leu Ser Ile Phe Asp Phe Ser Ser Gly Glu Cys
385                 390                 395                 400

Val Ala Thr Met Phe Gly His Ser Glu Ile Val Thr Gly Met Lys Phe
                405                 410                 415

Ser Asn Asp Cys Lys His Leu Ile Ser Val Ser Gly Asp Ser Cys Ile
            420                 425                 430

Phe Val Trp Arg Leu Ser Ser Glu Met Thr Ile Ser Met Arg Gln Arg
        435                 440                 445

Leu Ala Glu Leu Arg Gln Arg Gln Arg Gly Gly Lys Gln Gln Gly Pro
    450                 455                 460

Ser Ser Pro Gln Arg Ala Ser Gly Pro Asn Arg His Gln Ala Pro Ser
465                 470                 475                 480

Met Leu Ser Pro Gly Pro Ala Leu Ser Ser Asp Ser Asp Lys Glu Gly
                485                 490                 495

Glu Asp Glu Gly Thr Glu Glu Glu Leu Pro Ala Leu Pro Val Leu Ala
            500                 505                 510

Lys Ser Thr Lys Lys Ala Leu Ala Ser Val Pro Ser Pro Ala Leu Pro
        515                 520                 525

Arg Ser Leu Ser His Trp Glu Met Ser Arg Ala Gln Glu Ser Val Gly
    530                 535                 540

Phe Leu Asp Pro Ala Pro Ala Ala Asn Pro Gly Pro Arg Arg Arg Gly
545                 550                 555                 560

Arg Trp Val Gln Pro Gly Val Glu Leu Ser Val Arg Ser Met Leu Asp
                565                 570                 575

Leu Arg Gln Leu Glu Thr Leu Ala Pro Ser Leu Gln Asp Pro Ser Gln
            580                 585                 590

Asp Ser Leu Ala Ile Ile Pro Ser Gly Pro Arg Lys His Gly Gln Glu
        595                 600                 605

Ala Leu Glu Thr Ser Leu Thr Ser Gln Asn Glu Lys Pro Pro Arg Pro
    610                 615                 620

Gln Ala Ser Gln Pro Cys Ser Tyr Pro His Ile Ile Arg Leu Leu Ser
625                 630                 635                 640

Gln Glu Glu Gly Val Phe Ala Gln Asp Leu Glu Pro Ala Pro Ile Glu
                645                 650                 655

Asp Gly Ile Val Tyr Pro Glu Pro Ser Asp Asn Pro Thr Met Asp Thr
            660                 665                 670

Ser Glu Phe Gln Val Gln Ala Pro Ala Arg Gly Thr Leu Gly Arg Val
        675                 680                 685

Tyr Pro Gly Ser Arg Ser Ser Glu Lys His Ser Pro Asp Ser Ala Cys

690                 695                 700

Ser Val Asp Tyr Ser Ser Ser Cys Leu Ser Ser Pro Glu His Pro Thr
705                 710                 715                 720

Glu Asp Ser Glu Ser Thr Glu Pro Leu Ser Val Asp Gly Ile Ser Ser
                725                 730                 735

Asp Leu Glu Glu Pro Ala Glu Gly Asp Glu Glu Glu Glu Glu Glu Glu
            740                 745                 750

Gly Gly Met Gly Pro Tyr Gly Leu Gln Glu Gly Ser Pro Gln Thr Pro
        755                 760                 765

Asp Gln Glu Gln Phe Leu Lys Gln His Phe Glu Thr Leu Ala Ser Gly
    770                 775                 780

Ala Ala Pro Gly Ala Pro Val Gln Val Pro Glu Arg Ser Glu Ser Arg
785                 790                 795                 800

Ser Ile Ser Ser Arg Phe Leu Leu Gln Val Gln Thr Arg Pro Leu Arg
                805                 810                 815

Glu Pro Ser Pro Ser Ser Ser Ser Leu Ala Leu Met Ser Arg Pro Ala
            820                 825                 830

Gln Val Pro Gln Ala Ser Gly Glu Gln Pro Arg Gly Asn Gly Ala Asn
        835                 840                 845

Pro Pro Gly Ala Pro Pro Glu Val Glu Pro Ser Ser Gly Asn Pro Ser
    850                 855                 860

Pro Gln Gln Ala Ala Ser Val Leu Leu Pro Arg Cys Arg Leu Asn Pro
865                 870                 875                 880

Asp Ser Ser Trp Ala Pro Lys Arg Val Ala Thr Ala Ser Pro Phe Ser
                885                 890                 895

Gly Leu Gln Lys Ala Gln Ser Val His Ser Leu Val Pro Gln Glu Arg
            900                 905                 910

His Glu Ala Ser Leu Gln Ala Pro Ser Pro Gly Ala Leu Leu Ser Arg
        915                 920                 925

Glu Ile Glu Ala Gln Asp Gly Leu Gly Ser Leu Pro Pro Ala Asp Gly
    930                 935                 940

Pro Pro Ser Arg Pro His Ser Tyr Gln Asn Pro Thr Thr Ser Ser Met
945                 950                 955                 960

Ala Lys Ile Ser Arg Ser Ile Ser Val Gly Glu Asn Leu Gly Leu Val
                965                 970                 975

Ala Glu Pro Gln Ala His Ala Pro Ile Arg Val Ser Pro Leu Ser Lys
            980                 985                 990

Leu Ala Leu Pro Ser Arg Ala His Leu Val Leu Asp Ile Pro Lys Pro
        995                 1000                1005

Leu Pro Asp Arg Pro Thr Leu Ala Ala Phe Ser Pro Val Thr Lys
    1010                1015                1020

Gly Arg Ala Pro Gly Glu Ala Glu Lys Pro Gly Phe Pro Val Gly
    1025                1030                1035

Leu Gly Lys Ala His Ser Thr Thr Glu Arg Trp Ala Cys Leu Gly
    1040                1045                1050

Glu Gly Thr Thr Pro Lys Pro Arg Thr Glu Cys Gln Ala His Pro
    1055                1060                1065

Gly Pro Ser Ser Pro Cys Ala Gln Gln Leu Pro Val Ser Ser Leu
    1070                1075                1080

Phe Gln Gly Pro Glu Asn Leu Gln Pro Pro Pro Pro Glu Lys Thr
    1085                1090                1095

Pro Asn Pro Met Glu Cys Thr Lys Pro Gly Ala Ala Leu Ser Gln
    1100                1105                1110

Asp Ser Ala Val Ser Leu Glu Gln Cys Glu Gln Leu Val Ala Glu
    1115                1120                1125

Leu Arg Gly Ser Val Arg Gln Ala Val Arg Leu Tyr His Ser Val
    1130                1135                1140

Ala Gly Cys Lys Met Pro Ser Ala Glu Gln Ser Arg Ile Ala Gln
    1145                1150                1155

Leu Leu Arg Asp Thr Phe Ser Ser Val Arg Gln Glu Leu Glu Ala
    1160                1165                1170

Val Ala Gly Ala Val Leu Ser Ser Pro Gly Ser Ser Pro Gly Ala
    1175                1180                1185

Val Gly Ala Glu Gln Thr Gln Ala Leu Leu Glu Gln Tyr Ser Glu
    1190                1195                1200

Leu Leu Leu Arg Ala Val Glu Arg Arg Met Glu Arg Lys Leu
    1205                1210                1215

<210> 4
<211> 626
<212> PRT
<213> Homo sapiens

<400> 4

Met Ala Asp Gln Arg Met Asp Ile Ser Ser Thr Ile Ser Asp Phe Met
1               5                   10                  15

Ser Pro Gly Pro Thr Asp Leu Leu Ser Ser Ser Leu Gly Thr Ser Gly
            20                  25                  30

Val Asp Cys Asn Arg Lys Arg Lys Gly Ser Ser Thr Asp Tyr Gln Glu
        35                  40                  45

Ser Met Asp Thr Asp Lys Asp Asp Pro His Gly Arg Leu Glu Tyr Thr
    50                  55                  60

Glu His Gln Gly Arg Ile Lys Asn Ala Arg Glu Ala His Ser Gln Ile
65                  70                  75                  80

Glu Lys Arg Arg Arg Asp Lys Met Asn Ser Phe Ile Asp Glu Leu Ala
                85                  90                  95

Ser Leu Val Pro Thr Cys Asn Ala Met Ser Arg Lys Leu Asp Lys Leu
            100                 105                 110

Thr Val Leu Arg Met Ala Val Gln His Met Lys Thr Leu Arg Gly Ala
        115                 120                 125

Thr Asn Pro Tyr Thr Glu Ala Asn Tyr Lys Pro Thr Phe Leu Ser Asp
    130                 135                 140

Asp Glu Leu Lys His Leu Ile Leu Arg Ala Ala Asp Gly Phe Leu Phe
145                 150                 155                 160

Val Val Gly Cys Asp Arg Gly Lys Ile Leu Phe Val Ser Glu Ser Val
                165                 170                 175

Phe Lys Ile Leu Asn Tyr Ser Gln Asn Asp Leu Ile Gly Gln Ser Leu
            180                 185                 190

Phe Asp Tyr Leu His Pro Lys Asp Ile Ala Lys Val Lys Glu Gln Leu
        195                 200                 205

Ser Ser Ser Asp Thr Ala Pro Arg Glu Arg Leu Ile Asp Ala Lys Thr
    210                 215                 220

Gly Leu Pro Val Lys Thr Asp Ile Thr Pro Gly Pro Ser Arg Leu Cys
225                 230                 235                 240

Ser Gly Ala Arg Arg Ser Phe Phe Cys Arg Met Lys Cys Asn Arg Pro
                245                 250                 255

Ser Val Lys Val Glu Asp Lys Asp Phe Pro Ser Thr Cys Ser Lys Lys
            260                 265                 270

Lys Ala Asp Arg Lys Ser Phe Cys Thr Ile His Ser Thr Gly Tyr Leu
        275                 280                 285

Lys Ser Trp Pro Pro Thr Lys Met Gly Leu Asp Glu Asp Asn Glu Pro
    290                 295                 300

Asp Asn Glu Gly Cys Asn Leu Ser Cys Leu Val Ala Ile Gly Arg Leu
305                 310                 315                 320

His Ser His Val Val Pro Gln Pro Val Asn Gly Glu Ile Arg Val Lys
                325                 330                 335

Ser Met Glu Tyr Val Ser Arg His Ala Ile Asp Gly Lys Phe Val Phe
            340                 345                 350

Val Asp Gln Arg Ala Thr Ala Ile Leu Ala Tyr Leu Pro Gln Glu Leu
        355                 360                 365

Leu Gly Thr Ser Cys Tyr Glu Tyr Phe His Gln Asp Asp Ile Gly His
    370                 375                 380

Leu Ala Glu Cys His Arg Gln Val Leu Gln Thr Arg Glu Lys Ile Thr
385                 390                 395                 400

Thr Asn Cys Tyr Lys Phe Lys Ile Lys Asp Gly Ser Phe Ile Thr Leu
                405                 410                 415

Arg Ser Arg Trp Phe Ser Phe Met Asn Pro Trp Thr Lys Glu Val Glu
            420                 425                 430

Tyr Ile Val Ser Thr Asn Thr Val Val Leu Ala Asn Val Leu Glu Gly
        435                 440                 445

Gly Asp Pro Thr Phe Pro Gln Leu Thr Ala Ser Pro His Ser Met Asp
450                 455                 460

Ser Met Leu Pro Ser Gly Glu Gly Gly Pro Lys Arg Thr His Pro Thr
465                 470                 475                 480

Val Pro Gly Ile Pro Gly Gly Thr Arg Ala Gly Ala Gly Lys Ile Gly
                485                 490                 495

Arg Met Ile Ala Glu Glu Ile Met Glu Ile His Arg Ile Arg Gly Ser
            500                 505                 510

Ser Pro Ser Ser Cys Gly Ser Ser Pro Leu Asn Ile Thr Ser Thr Pro
        515                 520                 525

Pro Pro Asp Ala Ser Ser Pro Gly Gly Lys Lys Ile Leu Asn Gly Gly
    530                 535                 540

Thr Pro Asp Ile Pro Ser Ser Gly Leu Leu Ser Gly Gln Ala Gln Glu
545                 550                 555                 560

Asn Pro Gly Tyr Pro Tyr Ser Asp Ser Ser Ser Ile Leu Gly Glu Asn
                565                 570                 575

Pro His Ile Gly Ile Asp Met Ile Asp Asn Asp Gln Gly Ser Ser Ser
            580                 585                 590

Pro Ser Asn Asp Glu Ala Ala Met Ala Val Ile Met Ser Leu Leu Glu
        595                 600                 605

Ala Asp Ala Gly Leu Gly Gly Pro Val Asp Phe Ser Asp Leu Pro Trp
    610                 615                 620

Pro Leu
625

<210>  5
<211>  726
<212>  PRT
<213>  Homo sapiens

<400>  5

<210>  6
<211>  757
<212>  PRT
<213>  Homo sapiens

<400>  6

Leu Ala Pro Pro Lys Met Ala Asn Ile Thr Ser Ser Gln Ile Leu Asp
1               5                   10                  15

Gln Leu Lys Ala Pro Ser Leu Gly Gln Phe Thr Thr Thr Pro Ser Thr
            20                  25                  30

Gln Gln Asn Ser Thr Ser His Pro Thr Thr Thr Thr Ser Trp Asp Leu
        35                  40                  45

Lys Pro Pro Thr Ser Gln Ser Ser Val Leu Ser His Leu Asp Phe Lys
    50                  55                  60

Ser Gln Pro Glu Pro Ser Pro Val Leu Ser Gln Leu Ser Gln Arg Gln
65                  70                  75                  80

Gln His Gln Ser Gln Ala Val Thr Val Pro Pro Pro Gly Leu Glu Ser
                85                  90                  95

Phe Pro Ser Gln Ala Lys Leu Arg Glu Ser Thr Pro Gly Asp Ser Pro
            100                 105                 110

Ser Thr Val Asn Lys Leu Leu Gln Leu Pro Ser Thr Thr Ile Glu Asn
        115                 120                 125

Ile Ser Val Ser Val His Gln Pro Gln Pro Lys His Ile Lys Leu Ala
    130                 135                 140

Lys Arg Arg Ile Pro Pro Ala Ser Lys Ile Pro Ala Ser Ala Val Glu
145                 150                 155                 160

Met Pro Gly Ser Ala Asp Val Thr Gly Leu Asn Val Gln Phe Gly Ala
                165                 170                 175

Leu Glu Phe Gly Ser Glu Pro Ser Leu Ser Glu Phe Gly Ser Ala Pro
            180                 185                 190

Ser Ser Glu Asn Ser Asn Gln Ile Pro Ile Ser Leu Tyr Ser Lys Ser
        195                 200                 205

Leu Ser Glu Pro Leu Asn Thr Ser Leu Ser Met Thr Ser Ala Val Gln
    210                 215                 220

Asn Ser Thr Tyr Thr Thr Ser Val Ile Thr Ser Cys Ser Leu Thr Ser
225                 230                 235                 240

Ser Ser Leu Asn Ser Ala Ser Pro Val Ala Met Ser Ser Ser Tyr Asp
                245                 250                 255

Gln Ser Ser Val His Asn Arg Ile Pro Tyr Gln Ser Pro Val Ser Ser
            260                 265                 270

Ser Glu Ser Ala Pro Gly Thr Ile Met Asn Gly His Gly Gly Gly Arg
        275                 280                 285

Ser Gln Gln Thr Leu Asp Thr Pro Lys Thr Thr Gly Pro Pro Ser Ala
    290                 295                 300

Leu Pro Ser Val Ser Ser Leu Pro Ser Thr Thr Ser Cys Thr Ala Leu
305                 310                 315                 320

Leu Pro Ser Thr Ser Gln His Thr Gly Asp Leu Thr Ser Ser Pro Leu
                325                 330                 335

Ser Gln Leu Ser Ser Ser Leu Ser Ser His Gln Ser Ser Leu Ser Ala
            340                 345                 350

His Ala Ala Leu Ser Ser Ser Thr Ser His Thr His Ala Ser Val Glu
        355                 360                 365

Ser Ala Ser Ser His Gln Ser Ser Ala Thr Phe Ser Thr Ala Ala Thr
    370                 375                 380

Ser Val Ser Ser Ser Ala Ser Ser Gly Val Ser Leu Ser Ser Ser Met
385                 390                 395                 400

Asn Thr Ala Asn Ser Leu Cys Leu Gly Gly Thr Pro Ala Ser Ala Ser
                405                 410                 415

Ser Ser Ser Ser Arg Ala Ala Pro Leu Val Thr Ser Gly Lys Ala Pro
            420                 425                 430

Pro Asn Leu Pro Gln Gly Val Pro Pro Leu Leu His Asn Gln Tyr Leu
        435                 440                 445

Val Gly Pro Gly Gly Leu Leu Pro Ala Tyr Pro Ile Tyr Gly Tyr Asp
    450                 455                 460

Glu Leu Gln Met Leu Gln Ser Arg Leu Pro Val Asp Tyr Tyr Gly Ile
465                 470                 475                 480

Pro Phe Ala Ala Pro Thr Ala Leu Ala Ser Arg Asp Gly Ser Leu Ala
                485                 490                 495

Asn Asn Pro Tyr Pro Gly Asp Val Thr Lys Phe Gly Arg Gly Asp Ser
            500                 505                 510

Ala Ser Pro Ala Pro Ala Thr Thr Pro Ala Gln Pro Gln Gln Ser Gln
        515                 520                 525

Ser Gln Thr His His Thr Ala Gln Gln Pro Phe Val Asn Pro Ala Leu
    530                 535                 540

Pro Pro Gly Tyr Ser Tyr Thr Gly Leu Pro Tyr Tyr Thr Gly Met Pro
545                 550                 555                 560

Ser Ala Phe Gln Tyr Gly Pro Thr Met Phe Val Pro Pro Ala Ser Ala
                565                 570                 575

Lys Gln His Gly Val Asn Leu Ser Thr Pro Thr Pro Pro Phe Gln Gln
            580                 585                 590

Ala Ser Gly Tyr Gly Gln His Gly Tyr Ser Thr Gly Tyr Asp Asp Leu
        595                 600                 605

Thr Gln Gly Thr Ala Ala Gly Asp Tyr Ser Lys Gly Gly Tyr Ala Gly
    610                 615                 620

Ser Ser Gln Ala Pro Asn Lys Ser Ala Gly Ser Gly Pro Gly Lys Gly
625                 630                 635                 640

Val Ser Val Ser Ser Ser Thr Thr Gly Leu Pro Asp Met Thr Gly Ser
                645                 650                 655

Val Tyr Asn Lys Thr Gln Thr Phe Asp Lys Gln Gly Phe His Ala Gly
            660                 665                 670

Thr Pro Pro Pro Phe Ser Leu Pro Ser Val Leu Gly Ser Thr Gly Pro
        675                 680                 685

Leu Ala Ser Gly Ala Ala Pro Gly Tyr Ala Pro Pro Pro Phe Leu His
    690                 695                 700

Ile Leu Pro Ala His Gln Gln Pro His Ser Gln Leu Leu His His His
705                 710                 715                 720

Leu Pro Gln Asp Ala Gln Ser Gly Ser Gly Gln Arg Ser Gln Pro Ser
                725                 730                 735

Ser Leu Gln Pro Lys Ser Gln Ala Ser Lys Pro Ala Tyr Gly Asn Ser
            740                 745                 750

Pro Tyr Trp Thr Asn
        755

<210> 7

<211> 3006
<212> DNA
<213> Homo sapiens

<400> 7
atggcaccag ggactggcag ctccactgcc gtcaactcct gttctcctca gagcctgtca 60

tccgtccttg gctcaggatt tggagagctt gcaccaccaa aaatggcaaa catcaccagc 120

tcccagattt tggaccagtt gaaagctccg agtttgggcc agtttaccac caccccaagt 180

acacagcaga atagtacaag tcaccctaca actactactt cttgggacct caagccccca 240

acatcccagt cctcagtcct cagtcatctt gacttcaaat ctcaacctga gccatcccca 300

gttcttagcc agttgagcca gcgacaacag caccagagcc aggcagtcac tgttcctcct 360

cctggtttgg agtcctttcc ttcccaggca aaacttcgag aatcaacacc tggagacagt 420

ccctccactg tgaacaagct tttgcagctt cccagcacga ccattgaaaa tatctctgtg 480

tctgtccacc agccacagcc caaacacatc aaacttgcta agcggcggat acccccagct 540

tctaagatcc cagcttctgc agtggaaatg cctggttcag cagatgtcac aggattaaat 600

gtgcagtttg gggctctgga atttgggtca gaaccttctc tctctgaatt tggatcagct 660

ccaagcagtg aaaatagtaa tcagattccc atcagcttgt attcgaagtc tttaagtgag 720

cctttgaata catctttatc aatgaccagt gcagtacaga actccacata tacaacttcc 780

gtcattacct cctgcagtct gacaagctca tcactgaatt ctgctagtcc agtagcaatg 840

tcttcctctt atgaccagag ttctgtgcat aacaggatcc cataccaaag ccctgtgagt 900

tcatcagagt cagctccagg aaccatcatg aatggacatg gtggtggtcg aagtcagcag 960

acactagaca ctccaaagac aacaggccct ccctctgccc tcccgtctgt gagctccctg 1020

cccagcacca cctcctgcac tgcacttctg ccgtccacat cccagcacac tggcgacctg 1080

actagcagcc ctctctctca gcttagcagt tcgctctcca gccaccagag cagcctctct 1140

gcacatgcag ccctctcctc gagcacgtca cacacacatg ccagtgtgga gagcgcctct 1200

tcccaccagt cctcagccac cttctccacg gcagcgacct ccgtctcaag ttccgcatcc 1260

tcaggcgtca gcctgtccag tagcatgaac accgcgaaca gcctctgtct gggtgggacc 1320

cccgcgagtg catccagcag cagtagcagg gccgcgccct tggtgacctc aggcaaagca 1380

cccccaaact tacctcaggg ggtgcctccc ctgctgcaca accagtacct cgtaggtccc 1440

ggaggactgc ttcctgccta cccgatctat ggctatgacg agctccagat gctgcagtca 1500

cggctgccag tggactacta tggaattccc tttgctgcac ccacagcgct tgccagccga 1560

gatgggagcc tagctaataa tccatatcca ggtgatgtca caaagtttgg ccgtggggac 1620

tctgcatccc ctgcacccgc taccacacca gctcagccac agcagagcca atcacagacc 1680

caccacacag cccagcagcc cttcgtgaat cctgcactgc cacctggcta tagctacact 1740

ggtcttccct actacacagg catgcccagt gccttccagt atggccccac catgtttgtc 1800

cctccagcct cagccaagca acatggggtg aacctcagca ctcccacacc tcccttccag 1860

caggccagtg gttatggcca gcacggctac agtacaggtt atgacgacct gacccagggg 1920

acagcagcag gagactactc caaaggtggc tatgctggat catcgcaggc accaaacaag 1980

tctgcaggtt ctgggcctgg caaaggagta tcagtgtctt caagcaccac tggtctacct 2040

gatatgactg gttctgtcta caataagaca cagacttttg acaagcaggg atttcatgca 2100

gggacgcctc cacctttcag cctgccctcg gtcttgggct ccactgggcc cctggcctcg 2160

ggagcggccc ctggctatgc acccccacca ttcctacaca tcttgccagc ccaccagcag 2220

ccccactcac agctgctgca ccaccacctt ccgcaggatg cacagagtgg ctcgggtcag 2280

cgcagccagc ccagctccct gcagcccaag tctcaagcct ccaaacctgc ctacggcaac 2340

tctccatact ggacaaacta aacccagaag agaggggtgg gctggggcaa ggcttatcct 2400

gggcaggaga gaacacacga gcacgtattt gggagcccag tgccctttcc tagaattccc 2460

gacatgtgtc agccatgcct ctgtggggag tctgcctccc agactggcta ctgtatgtaa 2520

tgtatttatg tatgtatttg taaatgtgat agaagtctgg gggggagttg ggggatggcg 2580

gcagatgtta gccaggtctg ccctccccat tcaagcccct tctccactgt agcaaaataa 2640

gcacccccac cccatctgcc ttcaggtctt cttcacagcc tgcactgccc agtgggccac 2700

taggggcagt ctctggaagg gctggttcaa ggctgtttgg gtataggggt caggtaccaa 2760

tgaagaatca cgacttgtct cactcctttg gaaattgttt tctttcctgt gtaattactt 2820

catacctctg tttttgagaa actgttccgt ttgtcatctg tcatggtctc cttccaccaa 2880

atcttcatct gggaatagca gcggtatccc tccacccaag tatggccacc tgtttgtctt 2940

catatagaac aggggcttct ggtctggtca tgtccctaga gacttactag agactggctg 3000

accatg 3006

<210> 8
<211> 3655
<212> DNA
<213> Homo sapiens

<400> 8
ctccaacaag gtgtccagtc gggtgacagc agtgtccttc tctgaggatt gcagctactt 60

tgtcactgca ggcaaccgac acatcaaatt ctggtatctc gatgacagca agacctcaaa 120

ggtgaatgcc actgtgccct tgctgggccg ctcagggctg ctgggagagc tacggaacaa 180

cctattcact gatgtggcct gtggcagagg aaaaaaggcg gacagtacct tctgcatcac 240

gtcctcaggg ctgctgtgcg agttcagtga tcgaaggctt ttggacaagt gggtggagct 300

gagggtctac cccgaggtga aggatagtaa ccaggcctgc ctgcccccca gttcctttat 360

tacctgctcc tcagacaaca ccatccgcct gtggaacaca gagagctccg gggtgcatgg 420
ctccaccctc caccgaaaca tcctcagcag tgacctcatt aaaatcatct atgtggatgg 480
gaacacccag gccctgctgg acacagagct gcctggagga gacaaagctg atgcatccct 540
gttggatccc cgcgtgggca tccgctcggt gtgtgtcagc cccaatggac agcatctagc 600
atcaggggac cgtatgggca cacttagggt gcacgaactt cagtccctga gtgagatgct 660
gaaggtggag gcccatgact ctgagattct gtgcctggag tattctaagc cagacacagg 720
tctgaaactg ctagcatcgg cgagccggga ccggctgatc catgtgctgg atgccgggcg 780
ggagtacagc ctacagcaga cgctggacga acactcatcc tccatcactg ctgtcaagtt 840
tgcagccagt gatgggcaag tccgcatgat cagctgtgga gcagacaaga gcatctactt 900
ccgcactgcg cagaagtctg gagatggagt gcagttcaca cggacacacc acgtggtgcg 960
gaagacgacc ctctatgaca tggatgtgga gcccagctgg aagtacacgg ctatcggctg 1020
ccaggaccga aatattcgga tatttaacat cagcagtgga aagcagaaga agctgtttaa 1080
agggtcacag ggtgaggacg gcacactcat taaggtgcag acagacccct cagggatcta 1140
cattgccacc agctgttctg acaagaatct ctccattttt gacttctcct caggcgagtg 1200
cgtggccacc atgtttggcc actcagagat tgtcactggc atgaaattta gtaatgattg 1260
taaacatctc atctctgtgt ctggggacag ctgcatattt gtgtggcgcc tgagctctga 1320
gatgaccatc agcatgaggc agcgtctggc cgagttgcgc cagcgtcagc ggggcggcaa 1380
gcagcaagga ccatcctctc cccaaagggc ttctggaccc aaccggcacc aggccccatc 1440
aatgctgtct cctggaccgg ctctctcatc agacagtgac aaggagggag aagatgaggg 1500
gactgaagaa gaacttccag cactgcccgt ccttgccaag agtaccaaga aggcactggc 1560
ctcggtcccc agcccagctt tgccccgaag cctgtcccac tgggagatga gtcgggcaca 1620
ggagtccgtg gggttcctgg acccagctcc tgcagccaac ccaggaccca gaagaagagg 1680
gcgctgggtt cagccaggtg tggaactgag cgttagatcc atgctggatc tgcggcagct 1740
ggaaacactg gccccaagcc tgcaggaccc tagccaggac tcgctggcca tcatcccatc 1800
tggtcccagg aagcatgggc aggaggccct tgagacttca ctcactagcc agaatgaaaa 1860
gccccctcgg cctcaggctt cccaaccttg ttcctatccc catattatcc gattattgtc 1920
acaagaggaa ggggtctttg cccaagatct ggaacctgca cccattgaag atggtattgt 1980
ctacccggag ccgagtgaca accccaccat ggataccagt gagttccaag tgcaggctcc 2040
agcccgggga actctgggaa gagtgtaccc aggcagcagg agctcagaaa agcacagccc 2100
tgacagtgcc tgctctgtgg attacagcag cagctgcctt tccagcccgg agcaccccac 2160
tgaagactct gagagcacgg agcccctcag tgtggatggc atctcctcag accttgaaga 2220
gccagctgag ggtgatgaag aagaggaaga agaggaggga ggcatgggcc cctatgggct 2280

acaggagggc agcccccaga ctccagacca ggagcagttt ctaaaacagc actttgagac 2340
tctggccagt ggagctgctc caggggcccc agtgcaggtc ccagagaggt cagagtctcg 2400
gagtatctct tcacgattcc tgttgcaagt acagacccgc ccactcaggg aaccatcccc 2460
atcctcctca agcctggcac tgatgtcgag accagcccag gtgccacagg catctggtga 2520
gcagccgaga ggcaatggtg ccaatccccc tggagcaccc ccggaggtgg aaccgtcctc 2580
tggcaacccc agcccccagc aggcagcctc tgtgctgttg ccacgatgcc gtctcaaccc 2640
tgacagcagc tgggctccca agagagtggc cacagccagc cccttttctg gactccagaa 2700
ggcccagtct gtgcacagtc tggtgccaca ggaaagacat gaggccagtc tgcaggcccc 2760
ttcaccaggc gcactgctgt ctcgggagat cgaagctcag gatggtctgg gctccctgcc 2820
cccagctgat ggccctccgt ctcggcctca ctcctatcag aaccccacca ccagttccat 2880
ggccaagata tcccgcagta tctctgttgg ggagaacctg ggcctggtgg ctgaacctca 2940
agctcatgcc cccatccgag tctcaccact cagcaagctg gccctgccca gccgggctca 3000
cctggtcctg gacatcccca aaccactgcc tgaccgtcct accctggctg cattctctcc 3060
tgtcaccaaa ggccgggccc ctggcgaggc agaaaagcct ggcttcccgg tgggcctagg 3120
aaaagctcac agtacaactg agagatgggc ctgtttgggg gagggcacca ctcccaagcc 3180
taggacagag tgccaggctc atcctgggcc cagcagcccc tgtgcccagc aactgccagt 3240
cagcagcctc ttccaaggcc ctgaaaactt gcagccccca ccccctgaga agactcccaa 3300
ccccatggaa tgcaccaagc caggggcagc cctgagccag gactcagcgg tgagcctgga 3360
gcagtgtgag cagctggtgg cagagctccg cggcagcgtg cgccaggcag tgcggctcta 3420
ccactcggtg gctggctgca agatgccctc agcagagcaa agtcggattg cccagctcct 3480
cagagacacc ttctcttcag tgcgacagga gctggaagct gtggctgggg cagtgctgtc 3540
cagcccaggc agcagccctg ggctgtgggg agccgagcag acacaggccc tgctggagca 3600
atactcagaa ctgttgcttc gagccgtgga acggcgtatg gaacgcaaac tctga 3655

<210> 9
<211> 1881
<212> DNA
<213> Homo sapiens

<400> 9
atggcagacc agagaatgga catttcttca accatcagtg atttcatgtc cccgggcccc 60
accgacctgc tttccagctc tcttggtacc agtggtgtgg attgcaaccg caaacggaaa 120
ggcagctcca ctgactacca agaaagcatg gacacagaca aagatgaccc tcatggaagg 180
ttagaatata cagaacacca aggaaggata aaaaatgcaa gggaagctca cagtcagatt 240
gaaaagcggc gtcgggataa aatgaacagt tttatagatg aattggcttc tttggtacca 300

acatgcaacg caatgtccag gaaattagat aaacttactg tgctaaggat ggctgttcag 360
cacatgaaaa cattaagagg tgccaccaat ccatacacag aagcaaacta caaaccaact 420
tttctatcag acgatgaatt gaaacacctc attctcaggg cagcagatgg atttttgttt 480
gtcgtaggat gtgaccgagg gaagatactc tttgtctcag agtctgtctt caagatcctc 540
aactacagcc agaatgatct gattggtcag agtttgtttg actacctgca tcctaaagat 600
attgccaaag tcaaggagca gctctcctcc tctgacaccg caccccggga gcggctcata 660
gatgcaaaaa ctggacttcc agttaaaaca gatataaccc ctgggccatc tcgattatgt 720
tctggagcac gacgttcttt cttctgtagg atgaagtgta acaggccttc agtaaaggtt 780
gaagacaagg acttcccctc tacctgctca aagaaaaaag cagatcgaaa aagcttctgc 840
acaatccaca gcacaggcta tttgaaaagc tggccaccca caaagatggg gctggatgaa 900
gacaacgaac cagacaatga ggggtgtaac ctcagctgcc tcgtcgcaat tggacgactg 960
cattctcatg tagttccaca accagtgaac ggggaaatca gggtgaaatc tatggaatat 1020
gtttctcggc acgcgataga tggaaagttt gtttttgtag accagagggc aacagctatt 1080
ttggcatatt taccacaaga acttctaggc acatcgtgtt atgaatattt tcaccaagat 1140
gacataggac atcttgcaga atgtcatagg caagttttac agacgagaga aaaaattaca 1200
actaattgct ataaatttaa aatcaaagat ggttctttta tcacactacg gagtcgatgg 1260
ttcagtttca tgaacccttg gaccaaggaa gtagaatata ttgtctcaac taacactgtt 1320
gttttagcca acgtcctgga aggcggggac ccaaccttcc cacagctcac agcatccccc 1380
cacagcatgg acagcatgct gccctctgga gaaggtggcc caaagaggac ccaccccact 1440
gttccaggga ttccaggggg aacccgggct ggggcaggaa aaataggccg aatgattgct 1500
gaggaaatca tggaaatcca caggataaga gggtcatcgc cttctagctg tggctccagc 1560
ccattgaaca tcacgagtac gcctccccct gatgcctctt ctccaggagg caagaagatt 1620
ttaaatggag ggactccaga cattccttcc agtggcctac tatcaggcca ggctcaggag 1680
aacccaggtt atccatattc tgatagttct tctattcttg gtgagaaccc ccacataggt 1740
atagacatga ttgacaacga ccaaggatca agtagtccca gtaatgatga ggcagcaatg 1800
gctgtcatca tgagcctctt ggaagcagat gctggactgg gtggccctgt tgactttagt 1860
gacttgccat ggccgctgta a 1881

<210> 10
<211> 2181
<212> DNA
<213> Homo sapiens

<400> 10
atggaagata gactccacat ggataatgga ctggtacccc aaaagattgt gtcggtgcac 60

ttgcaggact ccactctgaa ggaagttaag gatcaggtct caaacaagca agcccagatc 120
ctagagccga agcctgaacc ttctcttgag attaagcctg agcaggacgg tatggagcat 180
gttggcagag atgacccaaa ggctcttggt gaagaaccca aacaaaggag aggcagtgcc 240
tctgggagtg agcctgctgg ggacagtgac aggggagggg gccccgttga gcattatcac 300
ctccatctgt ctagttgcca cgagtgtctg gaacttgaga acagcaccat tgagtcagtc 360
aagtttgcgt ctgccgagaa cattccagac cttccctacg attatagcag cagtttggag 420
agtgttgctg atgagacctc ccccgaaaga gaagggagga gagtcaacct cacgggaaag 480
gcacccaaca tcctcctcta tgtgggctcc gactcccagg aagccctcgg ccggttccac 540
gaggtccggt ctgtgctggc cgactgtgtg gacattgaca gttatattct ctaccacctg 600
ctggaggaca gtgctctcag agacccgtgg acggacaact gtctgctgtt ggtcattgct 660
accagggagt ccattcccga agacctgtac cagaagttca tggcctatct ttctcaggga 720
gggaaggtgt tgggcctgtc ttcatccttc acctttggtg gctttcaggt gacaagcaag 780
ggtgcactgc acaagacagt ccagaacttg gttttctcca aggctgacca gagcgaggtg 840
aagctcagcg tcttgagcag tggctgcagg taccaggaag gccccgtccg gctcagcccc 900
ggcaggctcc agggccacct ggagaatgag gacaaggaca ggatgattgt gcatgtgcct 960
tttggaactc gcgggggaga agctgttctt tgccaggtgc acttagaact acctcccagc 1020
tccaacatag tgcaaactcc agaagatttt aacttgctca agtcaagcaa ttttagaaga 1080
tacgaagtcc ttagagagat tctgacaacc cttggcctca gctgtgacat gaaacaagtt 1140
cctgccttaa ctcctcttta cttgctgtca gctgcggagg aaatcaggga tcctcttatg 1200
cagtggcttg ggaaacatgt ggactccgag ggagaaataa aatccggcca gctctctctt 1260
agatttgttt catcctacgt gtctgaagta gaaataaccc catcttgtat acctgtggtg 1320
accaacatgg aggccttctc atcagaacat ttcaacttag agatctatcg ccaaaatctg 1380
cagaccaagc agttggggaa agtaattttg tttgccgaag tgacccccac aacgatgcgt 1440
ctcctggatg ggctgatgtt tcagacaccg caggaaatgg gcttaatagt gatcgcggcc 1500
cggcagaccg agggcaaagg acggggaggg aatgtgtggc tgagccctgt gggatgtgct 1560
ctttctactc tgctcatctc cattccactg agatcccagc tgggacagag gatcccgttt 1620
gtccagcatc tgatgtccgt ggctgtcgtg gaagcagtga ggtccattcc cgagtatcag 1680
gatatcaact tacgagtgaa gtggcccaac gatatttatt acagtgacct catgaagatc 1740
ggcggagttc tggttaactc aacactcatg ggagaaacat tttatatact tattggctgt 1800
ggatttaatg tgactaacag taaccctacc atctgcatca acgacctcat cacagaatac 1860
aataaacaac acaaggcaga actgaagccc ttaagagccg attatctcat cgccagagtc 1920

gtgactgtgc tggagaaact gatcaaagag tttcaggaca aagggcccaa cagcgtcctt   1980

cccctttatt accgatactg ggtccacagt ggtcagcaag tccatctggg cagcgcagag   2040

ggaccaaagg tgtccatcgt tggcctggac gattctggct tcctccaggt tcaccaggag   2100

ggcggcgagg ttgtgactgt gcacccggac ggcaactcct tcgacatgct gagaaacctc   2160

atcctcccca aacggcggta a                                             2181


<210>  11
<211>  6
<212>  PRT
<213>  Homo sapiens

<220>
<221>  MISC_FEATURE
<223>  Partial oligopeptide of JNK3 showing high score in the local alig
       nment between JNK3 and KIAA1491(SEQ ID NO:6)


<400>  11

Gln Gly Phe Asp Lys Gln
1               5


<210>  12
<211>  6
<212>  PRT
<213>  Homo sapiens

<220>
<221>  MISC_FEATURE
<223>  Partial oligopeptide of KIAA1491(SEQ ID NO:6) showing high score
       in the local alignment between JNK3 and KIAA1491


<400>  12

Gln Thr Phe Asp Lys Gln
1               5


<210>  13
<211>  6
<212>  PRT
<213>  Homo sapiens

<220>
<221>  MISC_FEATURE
<223>  a peptide consisting of sequential 6 amino acid residues which ex
       ists in an amino acid sequence of JNK3


<400>  13

Ser Leu Phe Pro Ala Asp
1               5


<210>  14

<211>  6
<212>  PRT
<213>  Homo sapiens

<220>
<221>  MISC_FEATURE
<223>  a partial peptide of KIAA0596(SEQ ID NO:3), which shows high homo
       logy with the peptide of SEQ ID NO:8


<400>  14

Ser Leu Pro Pro Ala Asp
1               5


<210>  15
<211>  6
<212>  PRT
<213>  Homo sapiens

<220>
<221>  MISC_FEATURE
<223>  a peptide consisting of sequential 6 amino acid residues which ex
       ists in an amino acid sequence of JNK3


<400>  15

Lys Val Ile Glu Gln Leu
1               5


<210>  16
<211>  6
<212>  PRT
<213>  Homo sapiens

<220>
<221>  MISC_FEATURE
<223>  a partial peptide of BMAL1 or BMAL2, which shows high homology wi
       th the peptide of SEQ ID NO:10


<400>  16

Lys Val Lys Glu Gln Leu
1               5


<210>  17
<211>  6
<212>  PRT
<213>  Homo sapiens

<220>
<221>  MISC_FEATURE
<223>  a peptide consisting of sequential 6 amino acid residues which ex
       ists in an amino acid sequence of JNK3


<400>  17

Leu Pro Pro Ser Ser Ser
1               5


<210>  18
<211>  6
<212>  PRT
<213>  Homo sapiens

<220>
<221>  MISC_FEATURE
<223>  a peptide consisting of sequential 6 amino acid residues which ex
       ists in an amino acid sequence of JNK3


<400>  18

Ala Asn Leu Cys Gln Val
1               5


<210>  19
<211>  6
<212>  PRT
<213>  Homo sapiens

<220>
<221>  MISC_FEATURE
<223>  a partial peptide of BPL1, which shows high homology with the pep
       tide of SEQ ID NO:12


<400>  19

Leu Pro Pro Ser Ser Asn
1               5


<210>  20
<211>  6
<212>  PRT
<213>  Homo sapiens

<220>
<221>  MISC_FEATURE
<223>  a partial peptide of BPL1, which shows high homology with the pep
       tide of SEQ ID NO:13


<400>  20

Ala Val Leu Cys Gln Val
1               5


<210>  21
<211>  33
<212>  PRT
<213>  Homo sapiens

<220>
<221>  MISC_FEATURE
<223>  Partial oligopeptide of JNK3 showing high score in the local alig

nment between JNK3 and KIAA1491(SEQ ID NO:6)

<400> 21

Gln Pro Ser Pro Ser Gly Ala Ala Val Asn Ser Ser Glu Ser Leu Pro
1               5                   10                  15

Pro Ser Ser Ser Val Asn Asp Ile Ser Ser Met Ser Thr Asp Gln Thr
            20                  25                  30

Leu

<210> 22
<211> 33
<212> PRT
<213> Homo sapiens

<220>
<221> MISC_FEATURE
<223> Partial oligopeptide of KIAA1491(SEQ ID NO:6) showing high score
      in the local alignment between JNK3 and KIAA1491

<400> 22

Gln Ser Ser Ala Thr Phe Ser Thr Ala Ala Thr Ser Val Ser Ser Ser
1               5                   10                  15

Ala Ser Ser Gly Val Ser Leu Ser Ser Ser Met Asn Thr Ala Asn Ser
            20                  25                  30

Leu

<210> 23
<211> 43
<212> PRT
<213> Homo sapiens

<220>
<221> MISC_FEATURE
<223> Partial oligopeptide of JNK3 showing high score in the local alig
      nment between JNK3 and KIAA1491(SEQ ID NO:6)

<400> 23

Ser Pro Ser Gly Ala Ala Val Asn Ser Ser Glu Ser Leu Pro Pro Ser
1               5                   10                  15

Ser Ser Val Asn Asp Ile Ser Ser Met Ser Thr Asp Gln Thr Leu Ala
            20                  25                  30

Ser Asp Thr Asp Ser Ser Leu Glu Ala Ser Ala
35                  40

<210>  24
<211>  43
<212>  PRT
<213>  Homo sapiens

<220>
<221>  MISC_FEATURE
<223>  Partial oligopeptide of KIAA1491(SEQ ID NO:6) showing high score
       in the local alignment between JNK3 and KIAA1491

<400>  24

Ala Ala Thr Ser Val Ser Ser Ser Ala Ser Ser Gly Val Ser Leu Ser
1               5                   10                  15

Ser Ser Met Asn Thr Ala Asn Ser Leu Cys Leu Gly Gly Thr Pro Ala
            20                  25                  30

Ser Ala Ser Ser Ser Ser Ser Arg Ala Ala Pro
        35                  40

<210>  25
<211>  4
<212>  PRT
<213>  Homo sapiens

<220>
<221>  MISC_FEATURE
<223>  Partial sequence identical in the sequences of JNK3 and KIAA1491(
       SEQ ID NO:6)

<400>  25

Phe Asp Lys Gln
1

<210>  26
<211>  14
<212>  PRT
<213>  Homo sapiens

<220>
<221>  MISC_FEATURE
<223>  Partial oligopeptide of JNK3 showing high score in the local alig
       nment between JNK3 and KIAA1491(SEQ ID NO:6)

<400>  26

His Ser Ala Gly Ile Ile His Arg Asp Leu Lys Pro Ser Asn
1               5                   10

<210> 27
<211> 14
<212> PRT
<213> Homo sapiens

<220>
<221> MISC_FEATURE
<223> Partial oligopeptide of KIAA1491(SEQ ID NO:6) showing high score
      in the local alignment between JNK3 and KIAA1491


<400> 27

His Pro Thr Thr Thr Thr Ser Trp Asp Leu Lys Pro Pro Thr
1               5                   10


<210> 28
<211> 4
<212> PRT
<213> Homo sapiens

<220>
<221> MISC_FEATURE
<223> Partial sequence identical in the sequences of JNK3 and KIAA1491(
      SEQ ID NO:6)


<400> 28

Asp Leu Lys Pro
1


<210> 29
<211> 30
<212> PRT
<213> Homo sapiens

<220>
<221> MISC_FEATURE
<223> Partial oligopeptide of JNK3 showing high score in the local alig
      nment between JNK3 and KIAA1491(SEQ ID NO:6)


<400> 29

Pro Ser Gly Ala Ala Val Asn Ser Ser Glu Ser Leu Pro Pro Ser Ser
1               5                   10                  15


Ser Val Asn Asp Ile Ser Ser Met Ser Thr Asp Gln Thr Leu
            20                  25                  30


<210> 30
<211> 29
<212> PRT
<213> Homo sapiens

<220>
<221> MISC_FEATURE
<223> Partial oligopeptide of KIAA1491(SEQ ID NO:6) showing high score

in the local alignment between JNK3 and KIAA1491

<400> 30

Pro Tyr Gln Ser Pro Val Ser Ser Ser Glu Ser Ala Pro Gly Thr Ile
1               5                   10                  15

Met Asn Gly His Gly Gly Gly Arg Ser Gln Gln Thr Leu
            20                  25

<210> 31
<211> 4
<212> PRT
<213> Homo sapiens

<220>
<221> MISC_FEATURE
<223> Partial sequence identical in the sequences of JNK3 and KIAA1491(
      SEQ ID NO:6)

<400> 31

Ser Ser Glu Ser
1

<210> 32
<211> 33
<212> PRT
<213> Homo sapiens

<220>
<221> MISC_FEATURE
<223> Partial oligopeptide of JNK3 showing high score in the local alig
      nment between JNK3 and KIAA1491(SEQ ID NO:6)

<400> 32

Ser Gly Ala Ala Val Asn Ser Ser Glu Ser Leu Pro Pro Ser Ser Ser
1               5                   10                  15

Val Asn Asp Ile Ser Ser Met Ser Thr Asp Gln Thr Leu Ala Ser Asp
            20                  25                  30

Thr

<210> 33
<211> 33
<212> PRT
<213> Homo sapiens

<220>
<221> MISC_FEATURE
<223> Partial oligopeptide of KIAA1491(SEQ ID NO:6) showing high score

in the local alignment between JNK3 and KIAA1491

<400> 33

Thr Gly Asp Leu Thr Ser Ser Pro Leu Ser Gln Leu Ser Ser Ser Leu
1               5                   10                  15

Ser Ser His Gln Ser Ser Leu Ser Ala His Ala Ala Leu Ser Ser Ser
            20                  25                  30

Thr

<210> 34
<211> 44
<212> PRT
<213> Homo sapiens

<220>
<221> MISC_FEATURE
<223> Partial oligopeptide of JNK3 showing high score in the local alig
      nment between JNK3 and KIAA1491(SEQ ID NO:6)

<400> 34

Pro Ser Pro Ser Gly Ala Ala Val Asn Ser Ser Glu Ser Leu Pro Pro
1               5                   10                  15

Ser Ser Ser Val Asn Asp Ile Ser Ser Met Ser Thr Asp Gln Thr Leu
            20                  25                  30

Ala Ser Asp Thr Asp Ser Ser Leu Glu Ala Ser Ala
        35                  40

<210> 35
<211> 45
<212> PRT
<213> Homo sapiens

<220>
<221> MISC_FEATURE
<223> Partial oligopeptide of KIAA1491(SEQ ID NO:6) showing high score
      in the local alignment between JNK3 and KIAA1491

<400> 35

Ser Ser Pro Leu Ser Gln Leu Ser Ser Ser Leu Ser Ser His Gln Ser
1               5                   10                  15

Ser Leu Ser Ala His Ala Ala Leu Ser Ser Ser Thr Ser His Thr His
            20                  25                  30

Ala Ser Val Glu Ser Ala Ser Ser His Gln Ser Ser Ala
35                  40                  45

<210>  36
<211>  31
<212>  PRT
<213>  Homo sapiens

<220>
<221>  MISC_FEATURE
<223>  Partial oligopeptide of JNK3 showing high score in the local alig
       nment between JNK3 and KIAA0596(SEQ ID NO:3)

<400>  36

Gln Pro Ser Pro Ser Gly Ala Ala Val Asn Ser Ser Glu Ser Leu Pro
1               5                   10                  15

Pro Ser Ser Ser Val Asn Asp Ile Ser Ser Met Ser Thr Asp Gln
            20                  25                  30

<210>  37
<211>  31
<212>  PRT
<213>  Homo sapiens

<220>
<221>  MISC_FEATURE
<223>  Partial oligopeptide of  KIAA0596(SEQ ID NO:3) showing high score
       in the local alignment between JNK3 and KIAA0596

<400>  37

Gln Pro Pro Pro Pro Glu Lys Thr Pro Asn Pro Met Glu Cys Thr Lys
1               5                   10                  15

Pro Gly Ala Ala Leu Ser Gln Asp Ser Ala Val Ser Leu Glu Gln
            20                  25                  30

<210>  38
<211>  9
<212>  PRT
<213>  Homo sapiens

<220>
<221>  MISC_FEATURE
<223>  Partial oligopeptide of JNK3 showing high score in the local alig
       nment between JNK3 and KIAA0596(SEQ ID NO:3)

<400>  38

Phe Thr Pro Gln Lys Thr Leu Glu Glu
1               5

<210> 39
<211> 9
<212> PRT
<213> Homo sapiens

<220>
<221> MISC_FEATURE
<223> Partial oligopeptide of KIAA0596(SEQ ID NO:3) showing high score
in the local alignment between JNK3 and KIAA0596

<400> 39

Tyr Ser Leu Gln Gln Thr Leu Asp Glu
1               5

<210> 40
<211> 20
<212> PRT
<213> Homo sapiens

<220>
<221> MISC_FEATURE
<223> Partial oligopeptide of JNK3 showing high score in the local alig
nment between JNK3 and KIAA0596(SEQ ID NO:3)

<400> 40

Ser Asp Cys Thr Leu Lys Ile Leu Asp Phe Gly Leu Ala Arg Thr Ala
1               5                   10                  15

Gly Thr Ser Phe
            20

<210> 41
<211> 20
<212> PRT
<213> Homo sapiens

<220>
<221> MISC_FEATURE
<223> Partial oligopeptide of KIAA0596(SEQ ID NO:3) showing high score
in the local alignment between JNK3 and KIAA0596

<400> 41

Ser Asp Lys Asn Leu Ser Ile Phe Asp Phe Ser Ser Gly Glu Cys Val
1               5                   10                  15

Ala Thr Met Phe
            20

<210> 42
<211> 15
<212> PRT
<213> Homo sapiens

<220>
<221> MISC_FEATURE
<223> Partial oligopeptide of JNK3 showing high score in the local alig
nment between JNK3 and KIAA0596(SEQ ID NO:3)

<400> 42

Lys Leu Lys Ala Ser Gln Ala Arg Asp Leu Leu Ser Lys Met Leu
1               5                   10                  15

<210> 43
<211> 15
<212> PRT
<213> Homo sapiens

<220>
<221> MISC_FEATURE
<223> Partial oligopeptide of KIAA0596(SEQ ID NO:3) showing high score
in the local alignment between JNK3 and KIAA0596

<400> 43

Lys Leu Leu Ala Ser Ala Ser Arg Asp Arg Leu Ile His Val Leu
1               5                   10                  15

<210> 44
<211> 23
<212> PRT
<213> Homo sapiens

<220>
<221> MISC_FEATURE
<223> Partial oligopeptide of JNK3 showing high score in the local alig
nment between JNK3 and KIAA0596(SEQ ID NO:3)

<400> 44

Ser Leu Phe Pro Ala Asp Ser Glu His Asn Lys Leu Lys Ala Ser Gln
1               5                   10                  15

Ala Arg Asp Leu Leu Ser Lys
            20

<210> 45
<211> 23
<212> PRT
<213> Homo sapiens

<220>
<221> MISC_FEATURE
<223> Partial oligopeptide of KIAA0596(SEQ ID NO:3) showing high score
in the local alignment between JNK3 and KIAA0596

<400> 45

Ser Leu Val Pro Gln Glu Arg His Glu Ala Ser Leu Gln Ala Pro Ser
1               5                   10                  15

Pro Gly Ala Leu Leu Ser Arg
            20

<210>  46
<211>  17
<212>  PRT
<213>  Homo sapiens

<220>
<221>  MISC_FEATURE
<223>  Partial oligopeptide of JNK3 showing high score in the local alig
       nment between JNK3 and KIAA0596(SEQ ID NO:3)

<400>  46

Ile Glu Glu Trp Lys Glu Leu Ile Tyr Lys Glu Val Met Asn Ser Glu
1               5                   10                  15

Glu

<210>  47
<211>  18
<212>  PRT
<213>  Homo sapiens

<220>
<221>  MISC_FEATURE
<223>  Partial oligopeptide of  KIAA0596(SEQ ID NO:3) showing high score
       in the local alignment between JNK3 and KIAA0596

<400>  47

Leu Asp Lys Trp Val Glu Leu Arg Val Tyr Pro Glu Val Lys Asp Ser
1               5                   10                  15

Asn Gln

<210>  48
<211>  14
<212>  PRT
<213>  Homo sapiens

<220>
<221>  MISC_FEATURE
<223>  Partial oligopeptide of JNK3 showing high score in the local alig
       nment between JNK3 and KIAA0596(SEQ ID NO:3)

<400>  48

Ser Ser Met Ser Thr Asp Gln Thr Leu Ala Ser Asp Thr Asp
1               5                   10

<210>  49
<211>  14
<212>  PRT
<213>  Homo sapiens

<220>
<221>  MISC_FEATURE
<223>  Partial oligopeptide of  KIAA0596(SEQ ID NO:3) showing high score
       in the local alignment between JNK3 and KIAA0596

<400>  49

Ser Met Leu Ser Pro Gly Pro Ala Leu Ser Ser Asp Ser Asp
1               5                   10

<210>  50
<211>  23
<212>  PRT
<213>  homo sapiens

<220>
<221>  misc_feature
<223>  Partial oligopeptide of JNK3 showing high score in the local alig
       nment between JNK3 and BMAL1

<400>  50

Ser Lys Ser Lys Val Asp Asn Gln Phe Tyr Ser Val Glu Val Gly Asp
1               5                   10                  15

Ser Thr Phe Thr Val Leu Lys
            20

<210>  51
<211>  23
<212>  PRT
<213>  homo sapiens

<220>
<221>  misc_feature
<223>  Partial oligopeptide of BMAL1 showing high score in the local ali
       gnment between JNK3 and BMAL1

<400>  51

Thr Arg Glu Lys Ile Thr Thr Asn Cys Tyr Lys Phe Lys Ile Lys Asp
1               5                   10                  15

Gly Ser Phe Ile Thr Leu Arg
            20

<210> 52
<211> 14
<212> PRT
<213> homo sapiens

<220>
<221> misc_feature
<223> Partial oligopeptide of JNK3 showing high score in the local alig
nment between JNK3 and BMAL1

<400> 52

Val Gly Asp Ser Thr Phe Thr Val Leu Lys Arg Tyr Gln Asn
1               5                   10

<210> 53
<211> 14
<212> PRT
<213> homo sapiens

<220>
<221> misc_feature
<223> Partial oligopeptide of BMAL1 showing high score in the local ali
gnment between JNK3 and BMAL1

<400> 53

Val Ser Glu Ser Val Phe Lys Ile Leu Asn Tyr Ser Gln Asn
1               5                   10

<210> 54
<211> 10
<212> PRT
<213> homo sapiens

<220>
<221> misc_feature
<223> Partial oligopeptide of JNK3 showing high score in the local alig
nment between JNK3 and BMAL1

<400> 54

Glu Gln Leu Gly Thr Pro Cys Pro Glu Phe
1               5                   10

<210> 55
<211> 10
<212> PRT
<213> homo sapiens

<220>
<221> misc_feature
<223> Partial oligopeptide of BMAL1 showing high score in the local ali
gnment between JNK3 and BMAL1

<400> 55

Glu Leu Leu Gly Thr Ser Cys Tyr Glu Tyr
1               5                   10


<210> 56
<211> 22
<212> PRT
<213> homo sapiens

<220>
<221> misc_feature
<223> Partial oligopeptide of JNK3 showing high score in the local alig
      nment between JNK3 and BMAL1


<400> 56

Ser Ser Met Ser Thr Asp Gln Thr Leu Ala Ser Asp Thr Asp Ser Ser
1               5                   10                  15


Leu Glu Ala Ser Ala Gly
            20


<210> 57
<211> 22
<212> PRT
<213> homo sapiens

<220>
<221> misc_feature
<223> Partial oligopeptide of BMAL1 showing high score in the local ali
      gnment between JNK3 and BMAL1


<400> 57

Ser Ser Pro Ser Asn Asp Glu Ala Ala Met Ala Val Ile Met Ser Leu
1               5                   10                  15


Leu Glu Ala Asp Ala Gly
            20


<210> 58
<211> 10
<212> PRT
<213> homo sapiens

<220>
<221> misc_feature
<223> Partial oligopeptide of JNK3 showing high score in the local alig
      nment between JNK3 and BMAL1


<400> 58

Ser Asp Cys Thr Leu Lys Ile Leu Asp Phe
1               5                   10

<210>  59
<211>  10
<212>  PRT
<213>  homo sapiens

<220>
<221>  misc_feature
<223>  Partial oligopeptide of BMAL1 showing high score in the local ali
      gnment between JNK3 and BMAL1


<400>  59

Ser Glu Ser Val Phe Lys Ile Leu Asn Tyr
1               5                   10


<210>  60
<211>  10
<212>  PRT
<213>  homo sapiens

<220>
<221>  misc_feature
<223>  Partial oligopeptide of JNK3 showing high score in the local alig
      nment between JNK3 and BMAL1


<400>  60

Tyr Ile Asp Gln Trp Asn Lys Val Ile Glu
1               5                   10


<210>  61
<211>  10
<212>  PRT
<213>  homo sapiens

<220>
<221>  misc_feature
<223>  Partial oligopeptide of BMAL1 showing high score in the local ali
      gnment between JNK3 and BMAL1


<400>  61

Phe Met Asn Pro Trp Thr Lys Glu Val Glu
1               5                   10


<210>  62
<211>  11
<212>  PRT
<213>  homo sapiens

<220>
<221>  misc_feature
<223>  Partial oligopeptide of JNK3 showing high score in the local alig
      nment between JNK3 and BMAL1

<400>  62

Val Lys Gly Gln Pro Ser Pro Ser Gly Ala Ala
1               5                   10


<210>  63
<211>  11
<212>  PRT
<213>  homo sapiens

<220>
<221>  misc_feature
<223>  Partial oligopeptide of BMAL1 showing high score in the local ali
       gnment between JNK3 and BMAL1


<400>  63

Val Lys Glu Gln Leu Ser Ser Ser Asp Thr Ala
1               5                   10


<210>  64
<211>  31
<212>  PRT
<213>  homo sapiens

<220>
<221>  misc_feature
<223>  Partial oligopeptide of JNK3 showing high score in the local alig
       nment between JNK3 and BMAL2


<400>  64

Glu Glu Lys Thr Lys Asn Gly Val Val Lys Gly Gln Pro Ser Pro Ser
1               5                   10                  15


Gly Ala Ala Val Asn Ser Ser Glu Ser Leu Pro Pro Ser Ser Ser
            20                  25                  30


<210>  65
<211>  31
<212>  PRT
<213>  homo sapiens

<220>
<221>  misc_feature
<223>  Partial oligopeptide of BMAL2 showing high score in the local ali
       gnment between JNK3 and BMAL2


<400>  65

Asp Asp Ser Ser Pro Thr Gly Leu Met Lys Asp Thr His Thr Val Asn
1               5                   10                  15


Cys Arg Ser Met Ser Asn Lys Glu Leu Phe Pro Pro Ser Pro Ser

            20                  25                  30

<210> 66
<211> 10
<212> PRT
<213> homo sapiens

<220>
<221> misc_feature
<223> Partial oligopeptide of JNK3 showing high score in the local alig
nment between JNK3 and BMAL2

<400> 66

Glu Gln Leu Gly Thr Pro Cys Pro Glu Phe
1               5                   10

<210> 67
<211> 10
<212> PRT
<213> homo sapiens

<220>
<221> misc_feature
<223> Partial oligopeptide of BMAL2 showing high score in the local ali
gnment between JNK3 and BMAL2

<400> 67

Glu Leu Leu Gly Thr Ser Cys Tyr Glu Tyr
1               5                   10

<210> 68
<211> 23
<212> PRT
<213> homo sapiens

<220>
<221> misc_feature
<223> Partial oligopeptide of JNK3 showing high score in the local alig
nment between JNK3 and BMAL2

<400> 68

Ser Lys Ser Lys Val Asp Asn Gln Phe Tyr Ser Val Glu Val Gly Asp
1               5                   10                  15

Ser Thr Phe Thr Val Leu Lys
            20

<210> 69
<211> 23
<212> PRT
<213> homo sapiens

<220>
<221> misc_feature
<223> Partial oligopeptide of BMAL2 showing high score in the local ali
      gnment between JNK3 and BMAL2

<400> 69

Ser Lys Glu Lys Ile Leu Thr Asp Ser Tyr Lys Phe Arg Ala Lys Asp
1               5                   10                  15

Gly Ser Phe Val Thr Leu Lys
            20

<210> 70
<211> 12
<212> PRT
<213> homo sapiens

<220>
<221> misc_feature
<223> Partial oligopeptide of JNK3 showing high score in the local alig
      nment between JNK3 and BMAL2

<400> 70

Ser Lys Ser Lys Val Asp Asn Gln Phe Tyr Ser Val
1               5                   10

<210> 71
<211> 12
<212> PRT
<213> homo sapiens

<220>
<221> misc_feature
<223> Partial oligopeptide of BMAL2 showing high score in the local ali
      gnment between JNK3 and BMAL2

<400> 71

Ser Lys Lys Lys Glu His Arg Lys Phe Tyr Thr Ile
1               5                   10

<210> 72
<211> 8
<212> PRT
<213> homo sapiens

<220>
<221> MISC_FEATURE
<223> Partial oligopeptide of JNK3 showing high score in the local alig
      nment between JNK3 and BPL1

<400> 72

Ser Tyr Leu Leu Tyr Gln Met Leu
1               5


<210>  73
<211>  8
<212>  PRT
<213>  homo sapiens

<220>
<221>  MISC_FEATURE
<223>  Partial oligopeptide of BPL1 showing high score in the local alig
       nment between JNK3 and BPL1


<400>  73

Ser Tyr Ile Leu Tyr His Leu Leu
1               5


<210>  74
<211>  8
<212>  PRT
<213>  homo sapiens

<220>
<221>  MISC_FEATURE
<223>  Partial oligopeptide of JNK3 showing high score in the local alig
       nment between JNK3 and BPL1


<400>  74

Ser Glu Pro Thr Leu Asp Val Lys
1               5


<210>  75
<211>  8
<212>  PRT
<213>  homo sapiens

<220>
<221>  MISC_FEATURE
<223>  Partial oligopeptide of BPL1 showing high score in the local alig
       nment between JNK3 and BPL1


<400>  75

Pro Glu Pro Ser Leu Glu Ile Lys
1               5


<210>  76
<211>  14
<212>  PRT
<213>  homo sapiens

<220>
<221>  MISC_FEATURE
<223>  Partial oligopeptide of JNK3 showing high score in the local alig

nment between JNK3 and BPL1

<400> 76

Ile His Arg Asp Leu Lys Pro Ser Asn Ile Val Val Lys Ser
1               5                   10

<210> 77
<211> 14
<212> PRT
<213> homo sapiens

<220>
<221> MISC_FEATURE
<223> Partial oligopeptide of BPL1 showing high score in the local alig
nment between JNK3 and BPL1

<400> 77

Val His Leu Glu Leu Pro Pro Ser Ser Asn Ile Val Gln Thr
1               5                   10

<210> 78
<211> 9
<212> PRT
<213> homo sapiens

<220>
<221> MISC_FEATURE
<223> Partial oligopeptide of JNK3 showing high score in the local alig
nment between JNK3 and BPL1

<400> 78

Gln Lys Thr Leu Glu Glu Phe Gln Asp
1               5

<210> 79
<211> 9
<212> PRT
<213> homo sapiens

<220>
<221> MISC_FEATURE
<223> Partial oligopeptide of BPL1 showing high score in the local alig
nment between JNK3 and BPL1

<400> 79

Glu Lys Leu Ile Lys Glu Phe Gln Asp
1               5

<210> 80
<211> 4
<212> PRT

<213>  homo sapiens

<220>
<221>  MISC_FEATURE
<223>  Partial sequence identical in the sequences of JNK3 and BPL1


<400>  80

Glu Phe Gln Asp
1


<210>  81
<211>  12
<212>  PRT
<213>  homo sapiens

<220>
<221>  MISC_FEATURE
<223>  Partial oligopeptide of JNK3 showing high score in the local alig
      nment between JNK3 and BPL1


<400>  81

Asp Ala Asn Leu Cys Gln Val Ile Gln Met Glu Leu
1               5                   10


<210>  82
<211>  11
<212>  PRT
<213>  homo sapiens

<220>
<221>  MISC_FEATURE
<223>  Partial oligopeptide of BPL1 showing high score in the local alig
      nment between JNK3 and BPL1


<400>  82

Glu Ala Val Leu Cys Gln Val His Leu Glu Leu
1               5                   10


<210>  83
<211>  4
<212>  PRT
<213>  homo sapiens

<220>
<221>  MISC_FEATURE
<223>  Partial sequence identical in the sequences of JNK3 and BPL1


<400>  83

Leu Cys Gln Val
1

<210> 84
<211> 7
<212> PRT
<213> homo sapiens

<220>
<221> MISC_FEATURE
<223> Partial oligopeptide of JNK3 showing high score in the local alig
      nment between JNK3 and BPL1


<400> 84

Leu Pro Pro Ser Ser Ser Val
1               5


<210> 85
<211> 7
<212> PRT
<213> homo sapiens

<220>
<221> MISC_FEATURE
<223> Partial oligopeptide of BPL1 showing high score in the local alig
      nment between JNK3 and BPL1


<400> 85

Leu Pro Pro Ser Ser Asn Ile
1               5


<210> 86
<211> 5
<212> PRT
<213> homo sapiens

<220>
<221> MISC_FEATURE
<223> Partial sequence identical in the sequences of JNK3 and BPL1


<400> 86

Leu Pro Pro Ser Ser
1               5


<210> 87
<211> 15
<212> PRT
<213> homo sapiens

<220>
<221> MISC_FEATURE
<223> Partial oligopeptide of JNK3 showing high score in the local alig
      nment between JNK3 and BPL1


<400> 87

Pro Gln Lys Thr Leu Glu Glu Phe Gln Asp Val Tyr Leu Val Met
1               5                   10                  15

<210>  88
<211>  15
<212>  PRT
<213>  homo sapiens

<220>
<221>  MISC_FEATURE
<223>  Partial oligopeptide of BPL1 showing high score in the local alig
       nment between JNK3 and BPL1

<400>  88

Ser Gln Glu Ala Leu Gly Arg Phe His Glu Val Arg Ser Val Leu
1               5                   10                  15

<210>  89
<211>  11
<212>  PRT
<213>  homo sapiens

<220>
<221>  MISC_FEATURE
<223>  Partial oligopeptide of JNK3 showing high score in the local alig
       nment between JNK3 and BPL1

<400>  89

Lys Thr Leu Glu Glu Phe Gln Asp Val Tyr Leu
1               5                   10

<210>  90
<211>  11
<212>  PRT
<213>  homo sapiens

<220>
<221>  MISC_FEATURE
<223>  Partial oligopeptide of BPL1 showing high score in the local alig
       nment between JNK3 and BPL1

<400>  90

Arg Ser Ile Pro Glu Tyr Gln Asp Ile Asn Leu
1               5                   10

## Description

### TECHNICAL FIELD

The present invention relates to an agent for inhibiting the phosphorylation of c-Jun, wherein the agent comprises a peptide that has a function for interacting with c-Jun N-terminal kinase 3 (hereinafter also referred to as "JNK3"), and a method for inhibiting the phosphorylation of c-Jun, wherein the method comprises using the peptide. The present invention also relates to an agent for inhibiting the ability of c-Jun to activate transcription, wherein the agent comprises the peptide, and a method for inhibiting the ability of c-Jun to activate transcription, wherein the method comprises using the peptide. The present invention further relates to the peptide, a polynucleotide encoding the peptide, a recombinant vector comprising the polynucleotide, a transformant transfected with the recombinant vector, and an antibody to the peptide. The present invention also relates to a method of producing the peptide, wherein the method comprises using the transformant, a method of identifying a compound that promotes or inhibits the interaction of the peptide with JNK3, and a method of identifying a compound that mediates or inhibits the expression of a polynucleotide encoding the peptide. The present invention also relates to a pharmaceutical composition containing the peptide, the polynucleotide, the vector, the transformant, the antibody, the agent for inhibiting the phosphorylation of c-Jun, or the inhibitor of the ability of c-Jun to activate transcription, and a method for preventing and/or treating disease(s) caused by the phosphorylation of c-Jun comprising using these.

### BACKGROUND OF THE INVENTION

c-Jun N-terminal kinase (hereafter also referred to as "JNK") is a protein phosphoenzyme belonging to the MAP kinase superfamily. A MAP kinase is an enzyme that plays an important role in an intracellular signaling pathway for regulating cellular proliferation and differentiation, and is activated, for example, by proliferative stimulus. However, unlike a classical MAP kinase (ERK), JNK is not substantially activated. Rather, JNK is activated by a cellular stress such as DNA damage, ultraviolet rays, high osmotic pressure, ER stress, and active oxygen, as well as by an inflammatory cytokine such as a tumor necrosis factor or interleukin 1.

It has been known that JNK is activated in response to stress to play a role in apoptosis because JNK activation is observed in the cell death of the nerve-derived cell line PC12 from nerve growth factor removal. Recently, it has been reported that JNK controls caspase dependent apoptosis and causes caspase independent apoptosis. Furthermore, it has been revealed that many cell deaths that occur pathologically (as in neurodegenerative disease) in mammals are independent of caspase (non-Patent Reference).

Apoptosis is classified into transcription dependent cell death (for example, sympathetic cell death by removal of nerve nutritive factors and death by DNA damage) and transcription independent apoptosis (for example, death from exposure to ultraviolet rays and death induced by Fas). It has been suggested that JNK plays a role in both forms of apoptosis. For transcription dependent apoptosis, it is recognized that JNK phosphorylates the serines (S) at the 63 and 73 positions in the amino acid sequence of c-Jun to activate the c-Jun. For example, death of neurocytes from kainic acid administration is inhibited in a knockin mouse that is a mutant (A63, A73) produced by substituting the phosphorylation sites of c-Jun with alanines (non Patent Reference 2). c-Jun is a transactivator that has an activity for transcription activation, that is, an activity to bind to a site for regulating gene expression to promote gene transcription. Therefore, c-Jun is presumed to induce the transcription of apoptosis promoting factors such as Bim and Fast in its downstream.

Three kinds of JNK genes, that is, the JNK 1 gene, the JNK 2 gene, and the JNK 3 gene are known to be found in mammals. It is known that the JNK 3 gene is expressed specifically in cranial nerves, for example. The JNK3 gene is known to be expressed at a stage of shock such as an anoxic condition to bring about damage to cerebral functions. It is also reported that the excitatory death of neurocytes from kainic acid administration is inhibited by a JNK 3 gene knockout (non-Patent Reference 3).

The references cited hereinafter are listed below.
Patent document 1: International Publication No. WO 01/67299.
Non-Patent Reference 1: Kitanaka, C. et al., Molecular medicine (2000)37: 408-418.
Non-Patent Reference 2: Behrens, A. et al., Nature Genetics (1999)21: 326-329.
Non-Patent Reference 3: Yang, D.D. et al., Nature (1997) 389:865-870.
Non-Patent Reference 4: Koyano, S. et al., FEBS Letters (1999) 457:385-388.
Non-Patent Reference 5: Gekakis N. et al., Science (1998) 280:1564-1569.
Non-Patent Reference 6: Bunger M.K. et al., Cell (2000) 103:1009-1017.
Non-Patent Reference 7: Nippon Rinsho (1996) 54(1):259-267.
Non-Patent Reference 8: Sambruck et al., "Molecular Cloning, A laboratory manual, 2^{nd} version" Cold Spring Harbor Laboratory, 1989.
Non-Patent Reference 9: Muramatsu Masami ed., "Labomanual Gene Technology" Maruzen KK., 1988.
Non-Patent Reference 10: Ehrlich H.E, ed. "PCR technology, Principle and Application of DNA amplification" Stockton Press, 1989.
Non-Patent Reference 11: Ulmer, Science (1983) 219:666-.
Non-Patent Reference 12: Ikeda, M. et al., Biochemical and Biophysical Research Communications (1997) 233: 258-264.
Non-Patent Reference 13: Suzuki et al., Nature Genetics (1994) 8(2): 122-128.
Non-Patent Reference 14: Yasuda, S. et al., Genes to Cells (1999) 4:734-756.
Non-Patent Reference 15: Ura Seiji, et al., Jikken Igaku (2001) 19:1839-1844.
Non-Patent Reference 16: Nature (1957) 179:160-161.

### SUMMARY OF THE INVENTION

It is an object of the present invention to find a substance for inhibiting a signaling pathway in which JNK3 plays a role, and to provide a means for preventing and/or treating disease(s) caused by the abnormal signaling pathway including disease(s) caused by cell apoptosis, such as a neurodegenerative disease.

The present inventors conducted concentrated research to achieve the above-mentioned object and have found proteins BMAL1 and BPL1, as well as proteins comprised of amino acid sequences represented by SEQ ID NO:1, SEQ ID NO:2, and SEQ No:3 in the Sequence List, which interact with JNK3. They also have clarified that BMAL1 and the protein comprised of the amino acid sequence represented by SEQ ID NO:2 bind to JNK3. Furthermore, they have demonstrated that all the proteins inhibit the phosphorylation of c-Jun by JNK3.

Accordingly, an aspect of the present invention is an agent for inhibiting the phosphorylation of c-Jun wherein the agent comprises one or more peptides selected from the following peptide group as active ingredients:
(i) BMAL1,
(ii) BPL1,
(iii) a peptide comprised of the amino acid sequence represented by SEQ ID NO:1 in the Sequence List,
(iv) a peptide comprised of the amino acid sequence represented by SEQ ID NO:2 in the Sequence List,
(v) a peptide comprised of the amino acid sequence represented by SEQ ID NO:3 in the Sequence List,
(vi) a peptide comprising at least one peptide of the above (i) to (v), wherein the peptide has a function for inhibiting the phosphorylation of c-Jun by JNK3,
(vii) a peptide comprised of at least 5 consecutive amino acid residues found in an amino acid sequence of at least one peptide of (i) to (v), wherein the peptide has a function for inhibiting the phosphorylation of c-Jun by JNK3,
(viii) a peptide having mutations of one to several amino acid residues in an amino acid sequence of at least one peptide of (i) to (v), wherein the peptide has a function for inhibiting the phosphorylation of c-Jun by JNK3,
(ix) a peptide having a homology of 70% or more to at least one peptide of (i) to (v), wherein the peptide has a function for inhibiting the phosphorylation of c-Jun by JNK3,
(x) a peptide comprised of the amino acid sequence represented by SEQ ID NO:4 in the Sequence List, and
(xi) a peptide comprised of the amino acid sequence represented by SEQ ID NO:5 in the Sequence List.

Furthermore, an aspect of the present invention is an agent for inhibiting the phosphorylation of c-Jun, wherein the agent is a peptide group consisting of (i), (ii), (iii), (iv), (x), or (xi).

In addition, an aspect of the present invention is a method for inhibiting the phosphorylation of c-Jun, wherein the method comprises putting one or more peptides selected from the peptide group having a function for interacting with JNK3 in coexistence with JNK3.

Furthermore, an aspect of the present invention is a method for inhibiting the phosphorylation of c-Jun, wherein the peptide group consists of (i), (ii), (iii), (iv), (x), or (xi).

In addition, an aspect of the present invention is an agent for inhibiting the ability of c-Jun to activate transcription, wherein the agent comprises one or more peptides selected from the peptide group having a function for interacting with JNK3 as active ingredients.

In addition, an aspect of the present invention is a method for inhibiting the ability of c-Jun to activate transcription, wherein the method comprises putting one or more peptides selected from the above peptide group having a function for interacting with JNK3 in coexistence with JNK3.

In addition, an aspect of the present invention is a pharmaceutical composition comprising an effective dose of the agent for inhibiting the phosphorylation of c-Jun or for inhibiting the ability of c-Jun to activate transcription.

In addition, an aspect of the present invention is a pharmaceutical composition, wherein the pharmaceutical composition is an agent for preventing and/or treating disease(s) caused by the phosphorylation of c-Jun by JNK3.

In addition, an aspect of the present invention is a pharmaceutical composition comprising an effective dose of one or more peptides selected from the above peptide group having a function for interacting with JNK3, wherein the pharmaceutical composition is an agent for preventing and/or treating disease(s) caused by the phosphorylation of c-Jun by JNK3.

In addition, an aspect of the present invention is a pharmaceutical composition comprising an effective dose of one or more polynucleotides encoding at least one peptide selected from the above peptide group having a function for interacting with JNK3, wherein the pharmaceutical composition is an agent for preventing and/or treating disease(s) caused by the phosphorylation of c-Jun by JNK3.

In addition, an aspect of the present invention is a pharmaceutical composition described above, wherein the disease caused by the phosphorylation of c-Jun by JNK3 is a neurodegenerative disease.

Furthermore, an aspect of the present invention is a pharmaceutical composition, wherein the neurodegenerative disease is polyglutamine disease, Huntington's disease, spinocerebellar ataxia, bulbospinal muscular atrophy, dentatorubropallidoluysian atrophy, Alzheimer's disease, Down's disease, Parkinson's disease, Lewy body dementia, multiple system atrophy, familial amyotrophic lateral sclerosis, progressive supranuclear palsy, corticobasal degeneration, Pick's disease, familial British dementia, Creutzfeldt-Jakob disease, Gerstmann-Stranssler syndrome, mad cow disease (bovine spongiform encephalopathy) (BSE), or familial dementia accompanying neuroserpin inclusion bodies.

In addition, an aspect of the present invention is a method for preventing and/or treating disease(s) caused by the phosphorylation of c-Jun by JNK3, wherein the method comprises putting one or more peptides selected from the peptide group having a function for interacting with JNK3 in coexistence with JNK3.

In addition, an aspect of the present invention is a method for preventing and/or treating disease(s) caused by the phosphorylation of c-Jun by JNK3, wherein the method comprises using one or more polynucleotides encoding at least one peptide selected from the peptide group having a function for interacting with JNK3 to inhibit the phosphorylation of c-Jun by JNK3 by expressing a peptide encoded by the polynucleotides.

In addition, an aspect of the present invention is a method for preventing and/or treating disease(s) caused by the phosphorylation of c-Jun by JNK3, wherein the method comprises using the pharmaceutical composition.

In addition, an aspect of the present invention is a method for preventing and/or treating disease(s), wherein the disease caused by the phosphorylation of c-Jun by JNK3 is a neurodegenerative disease.

Furthermore, an aspect of the present invention is a method for preventing and/or treating disease(s), wherein the neurodegenerative disease is polyglutamine disease, Huntington's disease, spinocerebellar ataxia, bulbospinal muscular atrophy, dentatorubropallidoluysian atrophy, Alzheimer's disease, Down's disease, Parkinson's disease, Lewy body dementia, multiple system atrophy, familial amyotrophic lateral sclerosis, progressive supranuclear palsy, corticobasal degeneration, Pick's disease, familial British dementia, Creutzfeldt-Jakob disease, Gerstmann-Stranssler syndrome, mad cow disease (Bovine Spongiform Encephalopathy) (BSE), or familial dementia accompanying neuroserpin inclusion bodies.

In addition, an aspect of the present invention is at least one peptide selected from the following peptide group:
(i) a peptide comprised of the amino acid sequence represented by SEQ ID NO: 1 in the Sequence List,
(ii) a peptide comprising a peptide comprised of the amino acid sequence represented by SEQ ID NO: 1 in the Sequence List,
(iii) a peptide comprised of at least 5 consecutive amino acid residues in the amino acid sequence represented by SEQ ID NO:1 in the Sequence List, and
(iv) a peptide having mutations of one to several amino acids found in at least one peptide of (i) to (iii).

Furthermore, an aspect of the present invention is the above peptide having a function for interacting with JNK3.

In addition, a further aspect of the present invention relates to a polynucleotide comprising a nucleotide sequence encoding the peptide or a complementary sequence thereof.

In addition, an aspect of the present invention relates to a polynucleotide comprised of a nucleotide represented by SEQ ID NO:7 in the Sequence List.

In addition, an aspect of the present invention is a polynucleotide that hybridizes with the polynucleotide under stringent conditions.

In addition, an aspect of the present invention is a recombinant vector comprising the polynucleotide.

Furthermore, an aspect of the present invention is a recombinant vector described above, wherein the recombinant vector is a recombinant expression vector.

In addition, an aspect of the present invention is a transformant transfected with the recombinant vector.

In addition, an aspect of the present invention relates to a method for producing the peptide, wherein the method comprises a process for culturing the transformant transfected with the recombinant vector.

Furthermore, an aspect of the present invention is an antibody that immunologically recognizes the peptide.

In addition, an aspect of the present invention is a method of identifying a compound that promotes or inhibits the interaction of the peptide with JNK3, comprising using at least one of the following: the peptide, a polynucleotide encoding the peptide, a recombinant vector comprising the polynucleotide, a transformant transfected with the recombinant vector, or an antibody that immunologically recognizes the peptide.

In addition, an aspect of the present invention is a method of identifying a compound that promotes or inhibits the expression of a polynucleotide encoding the peptide, wherein the method comprises using at least one of the following: a polynucleotide encoding the peptide, a recombinant vector comprising the polynucleotide, a transformant transfected with the recombinant vector, and an antibody that immunologically recognizes the peptide.

Furthermore, an aspect of the present invention is a pharmaceutical composition comprising an effective dose of at least one of the following: the peptide, a polynucleotide encoding the peptide, a recombinant vector comprising the polynucleotide, a transformant transfected with the recombinant vector, or an antibody that immunologically recognizes the peptide.

### BRIEF DESCRIPTION OF DRAWINGS

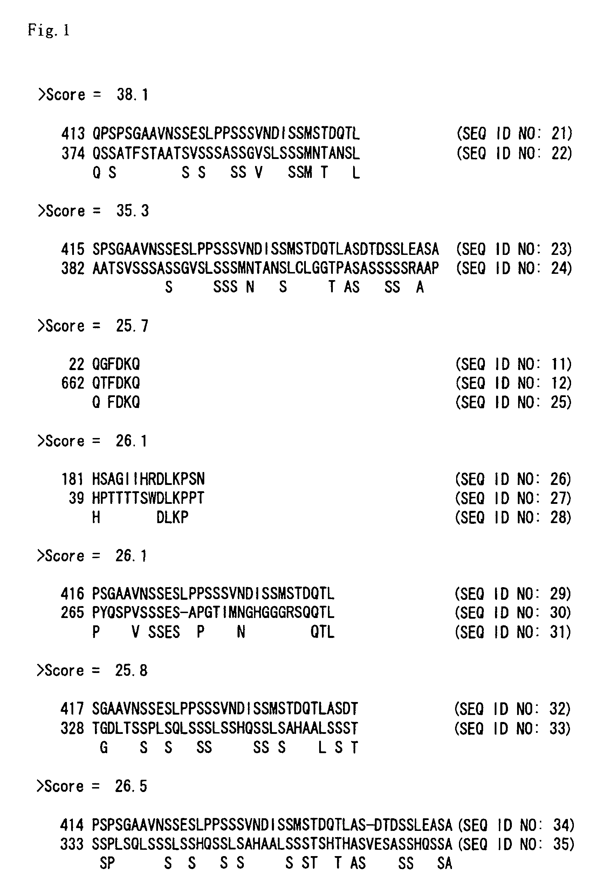

Figure 1 illustrates the result of the *in silico* prediction of the interaction between JNK3 and KIAA1491 (SEQ ID NO:6). Local alignment between JNK3 and KIAA1491 was conducted, and regions with high scores are shown. The upper and lower rows indicate sequences present in JNK3 and sequences present in KIAA1491, respectively.

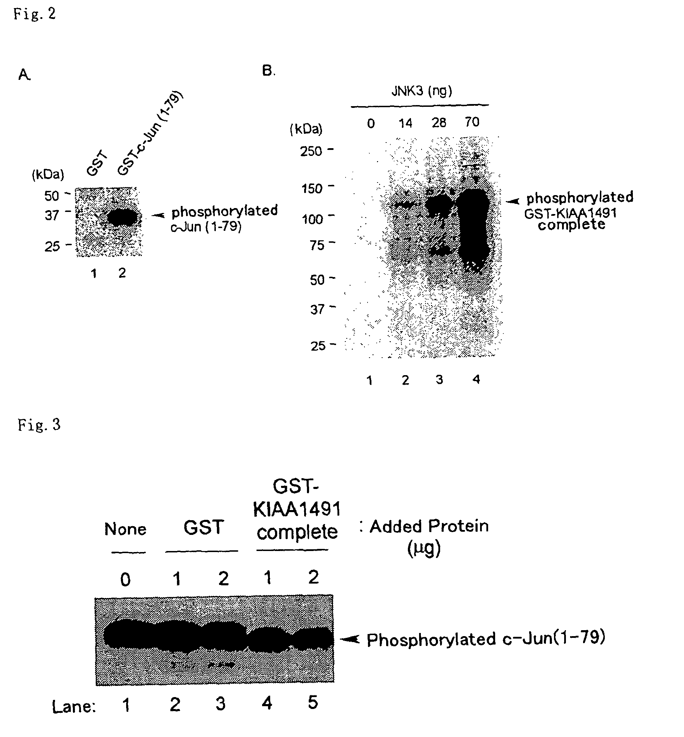

Figure 2A and Figure 2B show that c-Jun and KIAA1491 complete (SEQ ID NO: 1) were phosphorylated *in vitro* by JNK3. Figure 2A shows that although GST-c-Jun (1-79) (a fusion protein of glutathione S-transferase (GST) and a 79-amino acid region at the N-terminus of c-Jun) was phosphorylated by JNK3, GST was not phosphorylated. Figure 2B shows that a fusion protein of GST and KIAA1491 complete (GST-KIAA1491 complete) was dose-dependently phosphorylated by JNK3. The numerical values on the left indicate molecular weight.
Figure 3 shows that KIAA1491 complete (SEQ ID NO:1) inhibited the phosphorylation of c-Jun by JNK3. Lane 1 shows the result when another protein was not added to the reaction for the phosphorylation of c-Jun (1-79) by JNK3, lanes 2 and 3 show the results when 1 µg and 2 µg of GST were added thereto, respectively, and lanes 4 and 5 show the results when 1 µg and 2 µg of GST-KIAA1491 were added thereto, respectively. The arrowhead indicates phosphorylated c-Jun (1-79).

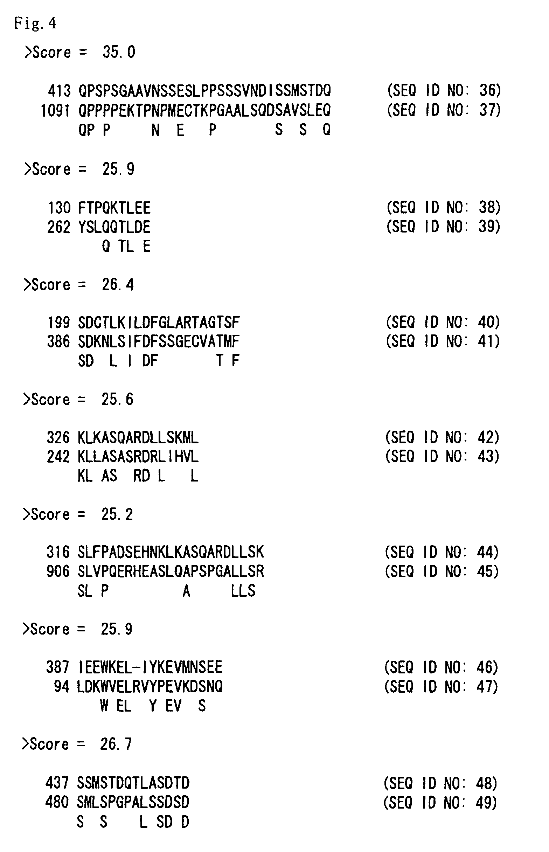

Figure 4 illustrates the result of the *in silico* prediction of the interaction between JNK3 and KIAA0596 (SEQ ID NO: 3). Local alignment between JNK3 and KIAA0596 was conducted, and regions with high scores are shown. The upper and lower rows indicate sequences present in JNK3 and sequences present in KIAA0596, respectively.

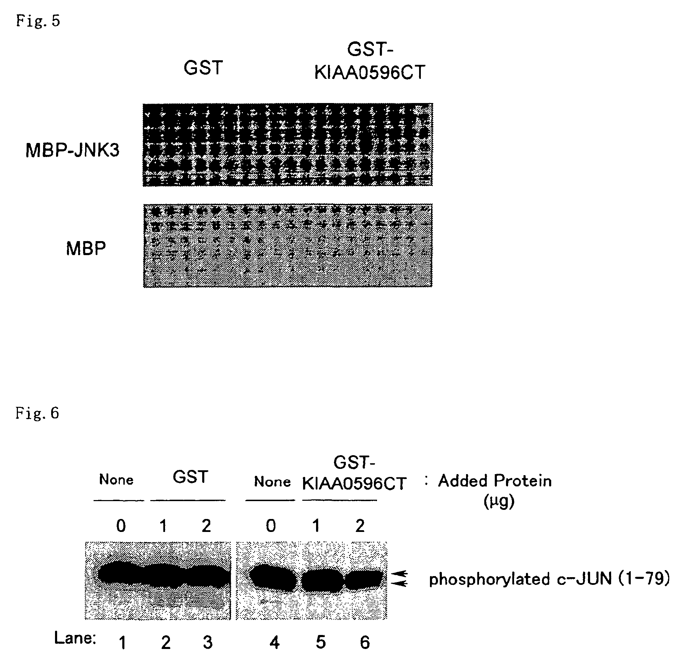

Figure 5 shows that KIAA0596CT (a peptide comprising the amino acid sequence from amino acid 639 to amino acid 1217 of the amino acid sequence of KIAA0596 (SEQ ID NO: 2)) bound with JNK3. The upper figure shows results detected the binding between GST-KIAA0596CT (fusion protein of KIAA0596CT and GST) and MBP-JNK3 (fusion protein of JNK3 and maltose binding protein (MBP)) by a far-Western method. The lower figure shows results detected the binding between GST-KIAA0596CT and MBP as a negative control by far-Western method.
Figure 6 shows that KIAA0596CT (SEQ ID NO: 2) inhibited the phosphorylation of c-Jun by JNK3. Lanes 1 and 4 show the results when another protein was not added to the reaction for the phosphorylation of c-Jun (1-79) by JNK3, lanes 2 and 3 show the results when 1 µg and 2 µg of GST were added thereto, respectively, and lanes 5 and 6 show the results when 1 µg and 2 µg of GST-KIAA0596CT were added thereto, respectively. The arrowheads indicate phosphorylated c-Jun (1-79).

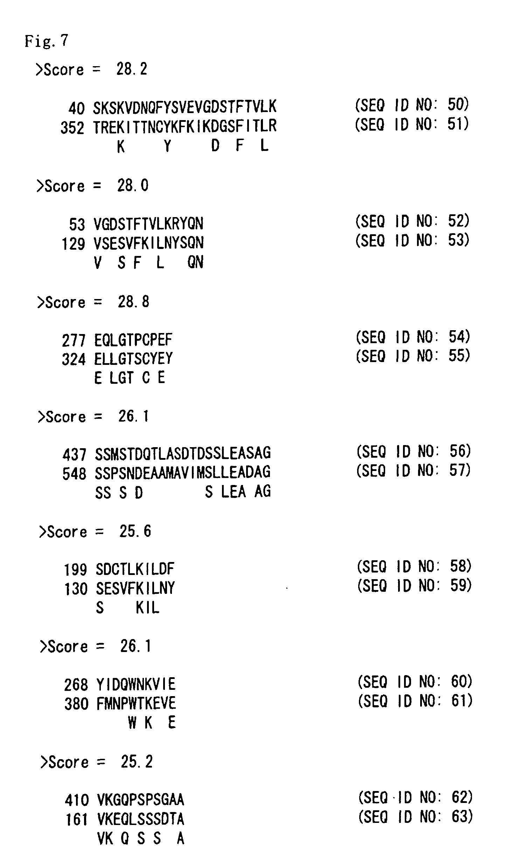

Figure 7 illustrates the result of the *in silico* prediction of the interaction between JNK3 and BMAL1. Local alignment between JNK3 and BMAL1 was conducted, and regions with high scores are shown. The upper and lower rows indicate sequences present in JNK3 and sequences present in BMAL1, respectively.

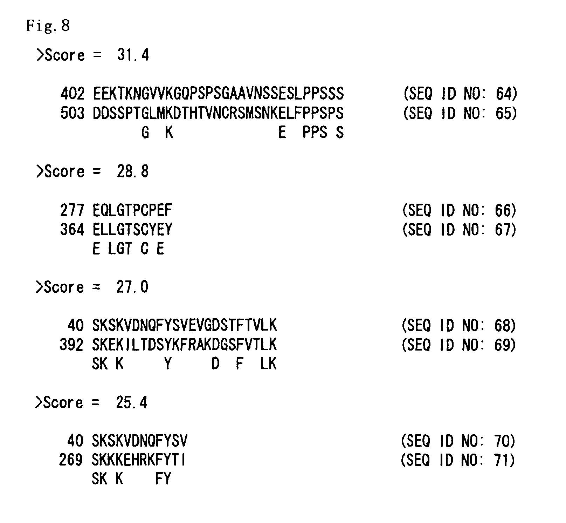

Figure 8 illustrates the result of the *in silico* prediction of the interaction between JNK3 and BMAL2. Local alignment between JNK3 and BMAL2 was conducted, and regions with high scores are shown. The upper and lower rows indicate sequences present in JNK3 and sequences present in BMAL2, respectively.

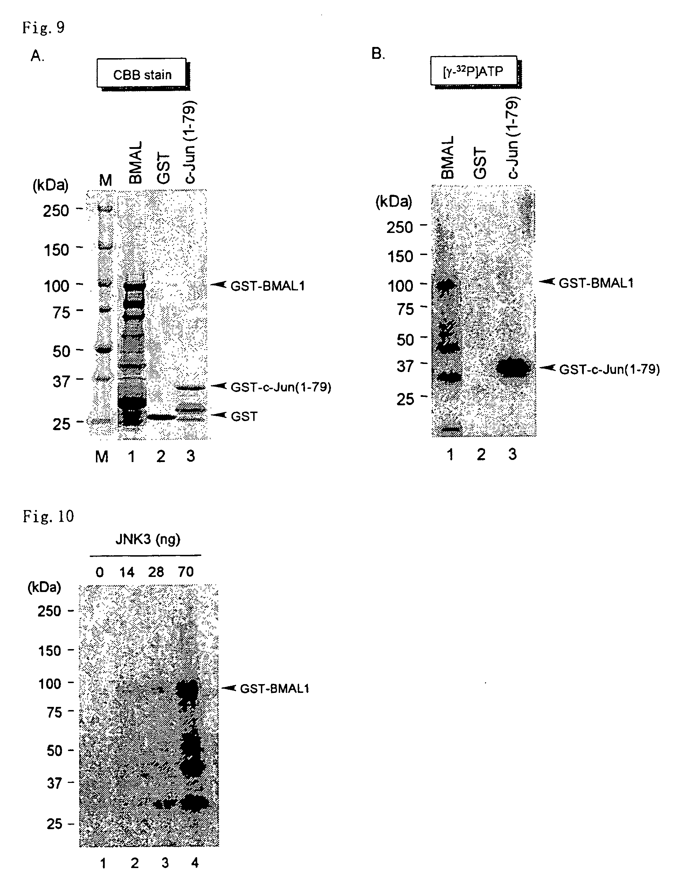

Figure 9A and Figure 9B show that BMAL1 was phosphorylated *in vitro* by JNK3. Figure 9A shows the positions to which each of the purified GST fusion proteins (lane 1: GST-BMAL1, lane 2: GST, lane 3: GST-c-Jun (1-79)) migrated in sodium dodecyl sulfate-polyacrylamide gel electrophoresis (SDS-PAGE) (arrowheads) detected by Coomassie brilliant blue (CBB) staining. Lane M is a molecular weight marker. The numerical values on the left of the figure indicate molecular weight. Figure 9B shows that although GST-BMAL1 (lane 1) and GST-c-Jun (1-79) (lane 3) were phosphorylated by JNK3, GST (lane 2) was not phosphorylated. The arrowheads indicate phosphorylated GST-BMAL1 and GST-c-Jun (1-79).
Figure 10 shows that BMAL1 was dose-dependently phosphorylated by JNK3. Lane 1, lane 2, lane 3, and lane 4 show the results obtained using no JNK3 (BMAL1 alone), 14 ng of JNK3, 28 ng of JNK3, and 70 ng of JNK3, respectively. The arrowhead indicates phosphorylated GST-BMAL1. The numerical values on the left of the figure indicate molecular weight of molecular weight markers.

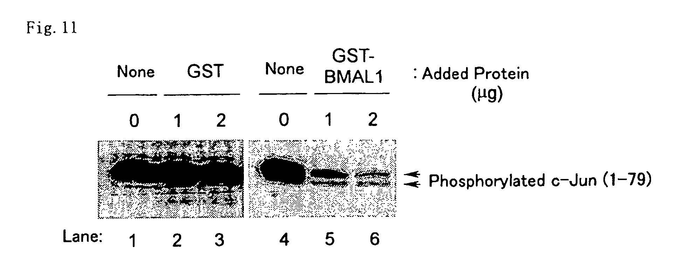

Figure 11 shows that BMAL1 inhibited the phosphorylation of c-Jun by JNK3. Lanes 1 and 4 show the results obtained when another protein was not added to the reaction for the phosphorylation of c-Jun (1-79) by JNK3, lanes 2 and 3 show the results when 1 µg and 2 µg of GST were added thereto, respectively, and lanes 5 and 6 show the results when 1 µg and 2 µg of GST-BMAL1 were added thereto, respectively. The arrowheads indicate phosphorylated c-Jun (1-79).

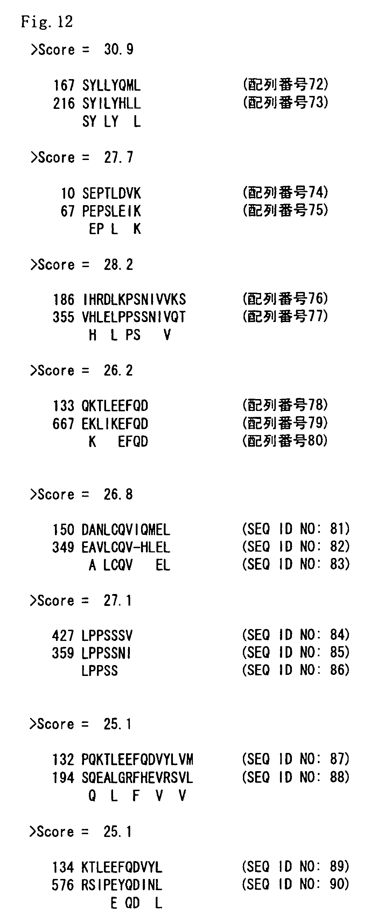

Figure 12 illustrates the result of the *in silico* prediction of the interaction between JNK3 and BPL1. Local alignment between JNK3 and BPL1 was conducted, and regions with high scores are shown. The upper and lower rows indicate sequences present in JNK3 and sequences present in BPL1, respectively.

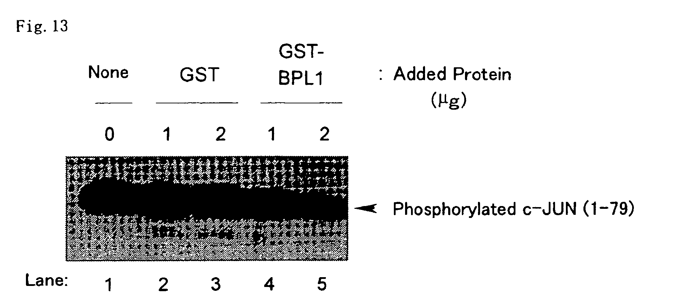

Figure 13 shows that BPL1 inhibited the phosphorylation of c-Jun by JNK3. Lane 1 shows the result obtained when another protein was not added to the reaction for the phosphorylation of c-Jun (1-79) by JNK3, lanes 2 and 3 show the results obtained when 1 µg and 2 µg of GST were added thereto, respectively, and lanes 4 and 5 show the results when 1 µg and 2 µg of GST-BPL1 were added thereto, respectively. The arrowhead indicates phosphorylated c-Jun (1-79).

### DETAILED DESCRIPTION OF THE INVENTION

The present invention claims the benefit of priority from Japanese Patent Application Nos. 2002-95291, 2002-95390, 2002-95442 and 2002-95486, which are incorporated herein by reference.

Technical and scientific terms used herein have the meanings as normally understood by those skilled in the art, unless otherwise defined. Various methods that are well known to those skilled in the art are referenced herein. These reference materials, such as published materials disclosing known methods cited herein, are incorporated herein by reference in their entirety.

Embodiments of the present invention are explained in more detail below. However, the detailed description below is exemplary and only for the purpose of explanation, and is not intended to limit the scope of the present invention.

In the present invention, proteins that interact with JNK3 were predicted according to a method described in International Publication No. WO 01/67299 (Patent Document 1), and as a result the following proteins were found: BPL1, BMAL1, KIAA1491 (SEQ ID NO:6) and KIAA0596 (SEQ ID NO:3). Further, a nucleotide sequence (SEQ ID NO:7) encoding the full length of KIAA1491 (hereunder, referred to as "KIAA1491 complete") was obtained by predicting *in silico* a region on the N-terminal side of KIAA1491 that was considered to be deleted, whereby KIAA1491 complete (SEQ ID NO:1) was acquired using a genetic engineering technique. It was clarified by experimentation that a protein KIAA0596CT (SEQ ID NO:2) comprised of an amino acid sequence of the C-terminal region of KIAA0596 (SEQ ID NO:3) and BPL1 bind with JNK3. It was also found that each of the proteins BPL1, BMAL1, KIAA1491 complete (SEQ ID NO:1) and KIAA0596CT (SEQ ID NO:2) inhibit the phosphorylation of c-Jun by JNK3.

In the present specification, the term "peptide" is used as a generic term to refer to an isolated or synthetic full-length protein, an isolated or synthetic full-length polypeptide, or an isolated or synthetic full-length oligopeptide, and in this specification the minimum size of a protein, a polypeptide or an oligopeptide is two amino acids. Amino acids may be designated by one-letter codes or three-letter abbreviations.

Further, in the present specification, the term "peptide having a function of interacting with JNK3" refers to a peptide that specifically interacts with JNK3, and specifically refers to, for example, a peptide that specifically contacts or binds with JNK3 as one of its functions. More specifically, it refers to a peptide that inhibits the ability of JNK3 to phosphorylate as one of the functions thereof, and preferably to a peptide that exhibits a competitively inhibiting function in the coexistence of an effective dose of the peptide and JNK3.

It is reported that the phosphorylation of c-Jun by JNK3 is involved in apoptosis, for example, in the death of neurocytes (Non-patent Document 2). Further, c-Jun is a transactivator that has the ability to activate transcription whereby it binds to a gene expression regulatory site to promote transcription of the gene, and induce transcription of an apoptosis promoter such as, for example, Bim or FasL, downstream of the signaling pathway thereof.

Therefore, by inhibiting the phosphorylation of c-Jun by JNK3 using the aforementioned peptides that interact with JNK3, the ability of c-Jun to activate transcription can be inhibited. Further, it is also possible to suppress physiological phenomenon caused by the phosphorylation of c-Jun by JNK3, such as apoptosis, and therefore to inhibit and/or treat disease(s) caused by the phosphorylation of c-Jun by JNK3, such as a neurodegenerative disease.

A peptide KIAA1491 (SEQ ID NO:6) that was predicted to interact with JNK3 is a peptide (GenBank accession number: AB040924) that was found in the HUGE database of Kazusa DNA Research Institute.The functions of this peptide had not been previously reported. Since it was considered that a region on the N-terminal side of KIAA1491 was deleted, the deletion region was predicted *in silico,* and it was found that a cDNA sequence encoding the region corresponds to a sequence of QV1-MT0132-201100-498-b05 MTO132 Homo sapiens cDNA (GenBank accession number: BF894928). Thus, a nucleotide sequence (SEQ ID NO:7) encoding the full length of KIAA1491 (KIAA1491 complete) was obtained. A full-length open reading frame (ORF) of the KIAA1491 complete gene is comprised of 2361 base pairs, and a gene product of the gene is comprised of 786 amino acid residues (SEQ ID NO:1). There have been no reports to date concerning this gene or the gene product thereof. Further, it was also clarified that mRNA corresponding to KIAA1491 complete cDNA is present in the brain, as shown by a reverse transcription polymerase chain reaction (RT-PCR) using poly-A RNA derived from human brain.

KIAA1491 complete was obtained from cDNA of human brain by a genetic engineering technique. As the result of experimental studies concerning the interaction of KIAA1491 complete with JNK3, it was found that KIAA1491 complete is phosphorylated by JNK3, and also that KIAA1491 complete inhibits the phosphorylation of c-Jun by JNK3. That is, it is considered that KIAA1491 complete interacts with JNK3 to inhibit the phosphorylation of c-Jun by JNK3.

KIAA0596 (SEQ ID NO:3) (GenBank accession number: AB011168) is another of the aforementioned peptides. There had been no report regarding the functions of KIAA0596. It was clarified that KIAA0596 has a homology of 74% to mouse JNK-binding protein 1 (JNKBP1) (GenBank accession number: AB029482)), which has been reported as binding with JNK3 (Non-patent Document 4). Thus, it was predicted that KIAA0596 is a human homolog of JNKBP1. There has been no report to date concerning JNKBP1 in humans. Mouse JNKBP1 is an activator protein of the JNK signaling pathway, which promotes the activity of JNK3 for the phosphorylation of c-Jun. There is a possibility that Mouse JNKBP1 plays a similar role to JIP-1, which is a scaffold protein in the JNK signaling pathway (Non-patent Document 4). Since it was kriown that the C-terminal region of mouse JNKBP1 is required for binding with JNK3, we prepared a peptide (SEQ ID NO:2, hereinafter referred to as "KIAA0596CT") comprised of a C-terminal region (amino acid sequence from amino acid 639 to 1217) of KIAA0596. We found that KIAA0596CT binds with JNK3 and dose-dependently inhibits the phosphorylation of c-Jun by activated JNK3. More specifically, it is supposed that KIAA0596CT binds with JNK3 to inhibit the activity of JNK3 for the phosphorylation of c-Jun, to thereby inhibit the phosphorylation of c-Jun. In addition, it is supposed that KIAA0596 (SEQ ID NO:3), which comprises KIAA0596CT, binds to JNK3 in the same manner as KIAA0596CT to inhibit the phosphorylation of c-Jun by JNK3.

BMAL1, still another of the aforementioned peptides, interacted with JNK3 to inhibit the phosphorylation of c-Jun by JNK3, although it was likewise phosphorylated. Specifically, it is supposed that BMAL1 interacts with JNK3 to inhibit the activity of JNK3 for phosphorylation of c-Jun, to thereby inhibit the phosphorylation of c-Jun.

BMAL1 is a transcription factor that promotes transcription of the PERIOD gene (PER), a circadian rhythm-associated gene. BMAL1 also forms a heterodimer with the CLOCK protein to bind to an E-box sequence that is present in a PER gene promoter (Non-patent Document 5 and Non-patent Document 6). In knockout mice of MOP3, an isoform of BMAL1, a circadian rhythm abnormality is observed.

BPL1, still another of the aforementioned peptides, bound to JNK3 and dose-dependently inhibited the phosphorylation of c-Jun by JNK3. That is, it is supposed that BPL1 binds with JNK3 to inhibit the activity of JNK3 for the phosphorylation of c-Jun, to thereby inhibit the phosphorylation of c-Jun.

BPL1 is a holocarboxylase synthetase, and it has a function for binding biotin with carboxylase, in particular, four kinds of metabolism-related carboxylases: methylcrotonyl-CoA carboxylase, acetyl-CoA carboxylase, pyruvate carboxylase, and propionyl-CoA carboxylase to activate the carboxylase (Non-patent Document 7). A shortage of the enzyme causes holocarboxylase synthetase deficiency. Holocarboxylase synthetase deficiency is an autosomal recessive inborn error of metabolism indicated by a diversity of biochemical abnormalities and clinical symptoms, which reflect a decline in the activity of a plurality of carboxylases (Non-patent Document 7).

Based on the above findings, the present invention provides an agent for inhibiting the phosphorylation of c-Jun and an agent for inhibiting the ability of c-Jun to activate transcription comprising a peptide that interacts with JNK3 as an active ingredient. The present invention also provides a method for inhibiting the phosphorylation of c-Jun and a method for inhibiting the ability of c-Jun to activate transcription, in one respect, comprising putting peptides that interact with JNK3 in coexistence with JNK3. As used herein, the term "agent for inhibiting the ability of c-Jun to activate transcription" refers to a substance that acts on a mechanism of transcription activation that comprises c-Jun, in some manner including binding of c-Jun to a specific DNA sequence or binding to another factor that conducts transcription in cooperation with c-Jun, ultimately to inhibit transcriptional activity that involves c-Jun.

Preferably, a peptide that interacts with JNK3 is KIAA1491 complete (SEQ ID NO: 1), KIAA0596CT (SEQ ID NO: 2), KIAA0596 (SEQ ID NO: 3), BMAL1 or BPL1. Although BMAL1 and BPL1 are known peptides, it has not been reported that they inhibit the phosphorylation of c-Jun by JNK3. Several isoforms of BMAL1 are known, for example, BMAL1a and BMAL1b, and they can each be used in the present invention. Preferably, BMAL1b can be used. Peptides that have a function for inhibiting the phosphorylation of c-Jun and are derived from any of these peptides are also included in the scope of the present invention. A peptide derived from KIAA1491 complete (SEQ ID NO: 1), KIAA0596CT (SEQ ID NO: 2), KIAA0596 (SEQ ID NO: 3), BMAL1 or BPL1 is, for example, a peptide comprising any of these five peptides, or a partial peptide of any of these five peptides. Alternatively, the peptide may be a peptide having a mutation in the amino acid sequence thereof relative to an amino acid sequence of any of these five peptides, or a peptide having a homology of 70% or more to any of these five peptides. The agent for inhibiting the phosphorylation of c-Jun and the agent for inhibiting the ability of c-Jun to activate transcription according to the present invention comprise one or more peptides selected from the above peptides as active ingredients. Further, the method for inhibiting the phosphorylation of c-Jun and the method for inhibiting the ability of c-Jun to activate transcription according to the present invention comprise, in one respect, putting one or more peptides selected from the above peptides in coexistence with JNK3. Coexistence of the peptide(s) with JNK3 can be carried out *in vitro* or *in vivo.*

As used herein, the term "partial peptide" refers to a peptide comprised of at least 5, preferably 8 or more, more preferably 12 or more, and even more preferably 15 or more consecutive amino acid residues in the amino acid sequence of the peptide comprising the partial peptide. More preferably, a peptide that comprises a specifically acting site or binding site, such as a contact site between JNK3 and a peptide that interacts with JNK3, is desirable. Since it is supposed that the partial peptide can, for example, competitively inhibit the phosphorylation of c-Jun by JNK3, the partial peptide is suitable for inhibiting the phosphorylation of c-Jun by JNK3.

The term "a peptide having mutation in the amino acid sequence thereof relative to an amino acid sequence of any of the above peptides" refers to a peptide comprised of an amino acid sequence having mutation such as a deletion, substitution, addition or insertion of one or more amino acids, for example, 1 to 100, preferably 1 to 30, more preferably 1 to 20, even more preferably 1 to 10, and most preferably 1 to several amino acids in the amino acid sequence in question. A peptide having the mutation may be a natural peptide or a peptide in which a mutation is introduced. Methods for introducing mutations are well known. For example, methods involving site-specific mutagenesis, genetic homologous-recombination, primer extension, polymerase chain reaction (PCR) or the like can be used alone or in suitable combinations thereof. The above methods can be carried out, for example, according to methods described in publications (Non-patent Document 8, Non-patent Document 9 and Non-patent Document 10), or using a modified method thereof. For example, the Ulmer's technique (Non-patent Document 11) may be utilized. In the introduction of the mutation, in order to avoid a change in the fundamental properties (physical properties, function, physiological activity, immunological activity, and the like) of the peptide, for example, mutual substitution among related amino acids (polar amino acids, non-polar amino acids, hydrophobic amino acids, hydrophilic amino acids, positive-charged amino acids, negative-charged amino acids and aromatic amino acids or the like) may be considered. In addition, with respect to these available peptides, an amino group or carboxyl group constituting the peptides can be altered, for example, by amidation or the like, to the extent that doing so does not cause a remarkable change in their function.

KIAA1491 complete (SEQ ID NO: 1), KIAA0596CT (SEQ ID NO: 2), KIAA0596 (SEQ ID NO: 3), BMAL1 and BPL1 can be obtained by common genetic engineering techniques. For example, using suitable primers designed by known methods based on the information of polynucleotides encoding the peptides, it is possible to amplify a gene of interest using a human-brain derived cDNA library as a template. Thereafter, it is possible to obtain cells that express the peptides by inserting the obtained gene into an expression vector, and then introducing the vector into a host cell. As a further example, it is also possible to synthesize the peptides utilizing a known cell-free protein synthesis system.

As a polynucleotide encoding KIAA1491 complete, for example, the polynucleotide described in SEQ ID NO: 7 can be used. As a polynucleotide encoding KIAAOS96CT (SEQ ID NO: 2) and KIAA0596 (SEQ ID NO: 3), for example, the polynucleotide described in SEQ ID NO: 8 can be used. BMAL1 is a known peptide (Non-patent Document 12) and a polynucleotide encoding BMAL1 has been disclosed. It is possible to acquire BMAL1 by utilizing the sequence information thereof. For example, a polynucleotide encoding a peptide comprised of an amino acid sequence described in SEQ ID NO: 4, such as a polynucleotide comprised of the nucleotide sequence described in SEQ ID NO: 9, can be used. The cDNA of human BPL1 has already been cloned (Non-patent Document 13), and an amino acid sequence thereof has been disclosed. For example, BPL1 can be obtained by a known genetic engineering technique using a polynucleotide encoding the peptide comprised of the amino acid sequence described in SEQ ID NO: 5, such as a polynucleotide comprised of the nucleotide sequence described in SEQ ID NO: 10.

Peptides derived from KIAA1491 complete (SEQ ID NO: 1), KIAA0596CT (SEQ ID NO: 2), KIAA0596 (SEQ ID NO: 3), BMAL1 or BPL1 can be obtained by designing the peptides on the basis of the respective amino acid sequences and performing synthesis according to known methods. For example, solid phase synthesis and liquid phase synthesis and the like are known methods for the chemical synthesis of a peptide, and any of these methods may be used. Further, peptides may also be obtained by common methods using genetic engineering techniques. Alternatively, partial peptides of the above peptides can be obtained by cleaving the above peptides using a suitable peptidase. Preferably, a peptide that interacts with JNK3 may be further selected from the obtained peptides. For example, for peptides derived from KIAA0596CT (SEQ ID NO: 2), KIAA0596 (SEQ ID NO: 3) or BPL1, a peptide having a function for binding with JNK3 may be selected by employing binding with JNK3 as an indicator. Alternatively, a peptide that interacts with JNK3 to inhibit the phosphorylation of c-Jun may be selected. Inhibition of the phosphorylation of c-Jun by a peptide can be assessed by, for example, putting a peptide derived from KIAA1491 complete (SEQ ID NO: 1), KIAA0596CT (SEQ ID NO: 2), KIAA0596 (SEQ ID NO: 3), BMAL1 or BPL1 in coexistence with JNK3 under the conditions where c-Jun is phosphorylated by JNK3, and determining whether the phosphorylation is inhibited or not. Detection of phosphorylation of c-Jun can be carried out by a common method for detecting a phosphorylated protein.

KIAA1491 complete (SEQ ID NO: 1), KIAA0596CT (SEQ ID NO: 2), KIAA0596 (SEQ ID NO: 3), BMAL1 and BPL1, or a peptide derived from any of them, have a function for interacting with JNK3 and exhibit an inhibitory action for the phosphorylation of c-Jun by JNK3.

In recent years, it has been reported that the JNK signaling pathway is activated in a polyglutamine related aggregation in a model system of cell death caused by a polyglutamine disease. It has also been reported that MKK4 (MAP kinase kinase 4, which is located upstream of JNK in JNK signaling and phosphorylates JNK to activate) or a dominant-negative mutant of c-Jun inhibits cell death caused by polyglutamine (Non-patent Document 14). Data has also been disclosed that indicates that JNK is involved in cell death caused by amyloid β, which is a cleavage product of amyloid precursor protein (APP), a causative gene of Alzheimer's disease (Non-patent Document 15).

It is therefore believed that by inhibiting the phosphorylation of c-Jun by JNK3 using KIAA1491 complete (SEQ ID NO: 1), KIAA0596CT (SEQ ID NO: 2), KIAA0596 (SEQ ID NO: 3), BMAL1 or BPL1, or a peptide derived from any of these, it is possible to resolve as well as inhibit and/or treat disease(s) caused by apoptosis involving the phosphorylation of c-Jun by JNK3 such as, for example, a neurodegenerative disease.

Examples of neurodegenerative disease include, but are not limited to, polyglutamine disease (for example, Huntington's disease, spinocerebellar ataxia, bulbospinal muscular atrophy, and dentaterubropallidoluysian atrophy), Alzheimer's disease, Down's disease, Parkinson's disease, Lewy bodies dementia, multiple system atrophy, familial ALS (amyotrophic lateral sclerosis), progressive supranuclear palsy, corticobasal degeneration, Pick's disease, familial British dementia, Creutzfeldt-Jakob disease, Gerstmann-Stranssler syndrome, mad cow disease (bovine spongiform encephalopathy) (BSE), and familial dementia accompanying neuroserpin inclusion bodies.

By utilizing KIAA1491 complete (SEQ ID NO: 1), KIAA0596CT (SEQ ID NO: 2), KIAA0596 (SEQ ID NO: 3), BMAL1 or BPL1 or a peptide derived from any of them, independently or in a combination of a plurality thereof, the present invention provides a pharmaceutical composition, which comprises at least one of the peptides as an active ingredient. A pharmaceutical composition comprising an effective dose of the agent for inhibiting the phosphorylation of c-Jun or the agent for inhibiting the ability of c-Jun to activate transcription according to the present invention is also included within the scope of the present invention. The pharmaceutical composition according to the present invention can be used as a preventing and/or treating agent for disease(s) caused by the phosphorylation of c-Jun by JNK3, such as a neurodegenerative disease.

It is also possible to inhibit and/or treat the aforementioned diseases by expressing *in vivo* a polynucleotide encoding any member of KIAA1491 complete (SEQ ID NO: 1), KIAA0596CT (SEQ ID NO: 2), KIAA0596 (SEQ ID NO: 3), BMAL1 or BPL1 or a peptide derived from any of them using a vector for protein expression or a transporter, to inhibit the phosphorylation of c-Jun by JNK3. Expression of these peptides *in vivo* can be carried out utilizing known methods. For example, *in vivo* expression can be performed by obtaining a polynucleotide encoding one of the peptides, then inserting it into a nucleic acid vector such as a replication-defective retrovirus vector, and delivering it into a target cell. Accordingly, a pharmaceutical composition comprising at least one of the polynucleotides encoding at least one of KIAA1491 complete (SEQ ID NO: 1), KIAA0596CT (SEQ ID NO: 2), KIAA0596 (SEQ ID NO: 3), BMAL1 or BPL1 or a peptide derived from any of them as an active ingredient, that is, the agent for preventing and/or treating the disease caused by the phosphorylation of c-Jun by JNK3, is also included within the scope of this invention.

The present invention also provides a method for preventing and/or treating the disease caused by the phosphorylation of c-Jun by JNK3, which comprises putting KIAA1491 complete (SEQ ID NO: 1), KIAA0596CT (SEQ ID NO: 2), KIAA0596 (SEQ ID NO: 3), BMAL1 or BPL1 or a peptide derived from any of them in coexistence with JNK3. Further, the method for preventing and/or treating the disease caused by the phosphorylation of c-Jun by JNK3 that uses the agent for inhibiting the phosphorylation of c-Jun or the agent for inhibiting the ability of c-Jun to activate transcription according to this invention is also included within the scope of this invention. In addition, the present invention also provides a method for preventing and/or treating disease(s) caused by the phosphorylation of c-Jun by JNK3 which comprises using at least one polynucleotide encoding at least one of KIAA1491 complete (SEQ ID NO: 1), KIAA0596CT (SEQ ID NO: 2), KIAA0596 (SEQ ID NO: 3), BMAL1 or BPL1 or the peptide derived from any of them and expressing the peptide encoded by the polynucleotide to inhibit the phosphorylation of c-Jun by JNK3.

A formulation of the pharmaceutical composition of the present invention preferably includes a suitable pharmaceutical carrier. The formulation comprises a therapeutically effective dose of at least one of the group consisting of KIAA1491 complete (SEQ ID NO: 1), KIAA0596CT (SEQ ID NO: 2), KIAA0596 (SEQ ID NO: 3), BMAL1, BPL1, peptides derived from these pepides, polynucleotides encoding the peptides, and vectors comprising the polynucleotides, and a pharmaceutically acceptable carrier or excipient. Examples of the carrier include, but are not limited to, a physiological saline, buffered physiological saline, dextrose, water, glycerol, ethanol, and a mixture thereof. The formulation may be selected in accordance with the suitability thereof to an administration route, and the formulations are well known to those skilled in the art. At the time of formulation, it may be used alone or in combination with other compounds or medicine necessary for treatment.

The formulation may be for systemic administration or for local administration. A preferred mode of systemic administration is injection, such as, for example, intravenous injection. Other injection routes, such as subcutaneous, intramuscular and intraperitoneal injection, can also be used. Another mode of administration can be peroral administration, if an enteric-coated formulation or capsule formulation is suitably formulated. In addition, permucosal administration or percutaneous administration using a penetrating agent such as bile acid salt, fusidic acid, or other surfactants can also be used. Topical administration can be in the form of a plaster, paste, gel, etc.

The range of dosage required can be determined according to the effectiveness of at least one selected from the aforementioned peptides, polynucleotides, and vectors in a therapeutically effective dose; the administration route; the properties of the formulation; the nature of the symptoms to be treated; and the judgment of the doctor in attendance. Specifically, an adequate dose may, for example, be in the range of 0.1 µg to 100 µg per 1 kg of body weight of subject. However, the dose can be modified by means of common conventional experiments for optimization, which means are well known in the art.

In the terms of pharmaceutical preparation, well-known methods of pharmaceutical preparation may be introduced according to the properties of the various targets such as peptides, polynucleotides, oligonucleotides or the like. Specifically, known methods for preparing pharmaceutical compositions such as powdered drugs, pills, tablets, capsules, aqueous solutions, ethanol solutions, liposome preparations, fat emulsions, or clathrates(such as those of cyclodextrin) can be used.

Powder drugs, pills, capsules and tablets can be prepared using excipients such as lactose, glucose, sucrose or mannitol; disintegrants such as starch or sodium alginate; lubricants such as magnesium stearate and talc; binders such as polyvinyl alcohol, hydroxypropyl cellulose and gelatin; surfactants such as fatty acid esters; and plasticizers such as glycerin. For the preparation of a tablet or a capsule, a solid pharmaceutical carrier is used.

A suspension can be produced using water, sugars such as sucrose, sorbitol and fructose, glycols such as polyethylene glycol (PEG), and oils.

Injectable solutions can be prepared using a saline solution, a glucose solution or a carrier comprising a mixture of saline water and glucose solution.

Inclusion into liposomes can be performed, for instance, by adding a solution that is prepared by dissolving the substance of interest in a solvent (such as ethanol) to a solution that is prepared by dissolving phospholipid in an organic solvent (such as chloroform), then removing the solvent by evaporation, then adding a phosphate-buffered solution thereto, followed by agitating and sonicating thereof, and finally, centrifuging thereof to obtain the supernatant and filtering it for recovery.

Fat emulsification can, for example, be prepared by mixing the substance of interest, oil ingredients (vegetable oils such as bean oil, sesame oil and olive oil as well as MCT and the like), emulsifying agents (such as phospholipids) and the like, followed by heating to obtain a solution, then adding a necessary amount of water, and emulsifying/homogenizing with homogenizer (for example, high-pressure-spray type, sonicating type or the like). The thus obtained product may also be subjected to freezing and drying. Moreover, when emulsifying fat, an auxiliary emulsifier may be added. Examples of the auxiliary emulsifier include glycerin and sugars (such as glucose, sorbitol, or fructose).

Cyclodextrin clathrates can, for example, be prepared by adding a solution that is prepared by dissolving cyclodextrin in water or the like by heating to a solution that is prepared by dissolving the substance of interest in a solvent (such as ethanol), then cooling and filtering the precipitate, and dry-sterilizing. At this point, the cyclodextrin to be used can be suitably selected from cyclodextrins with different void diameters (α, β, or γ type) according to the size of the substance.

The present invention further provides the KIAA1491 complete peptide that is comprised of the amino acid sequence described in SEQ ID NO: 1 in Sequence List. The scope of the present invention also includes a peptide that is derived from KIAA1491 complete, such as a peptide comprising the amino acid sequence described in SEQ ID NO: 1 or a peptide (partial peptide) comprised of at least 5 consecutive amino acid residues in the amino acid sequence. Based on the amino acid sequence of KIAA1491 complete or a peptide derived from KIAA1491 complete, the present invention also provides a peptide comprised of an amino acid sequence having mutation such as a deletion, substitution, addition or insertion of one or more amino acids, for example, 1 to 100, preferably 1 to 30, more preferably 1 to 20, more preferably 1 to 10, and most preferably 1 or several amino acids, by employing interaction with JNK3, for example, the phosphorylation by JNK3 or inhibition of the phosphorylation of c-Jun by JNK3, as an indicator. A peptide having the mutation may be a natural peptide or a peptide in which a mutation is introduced. Introduction of a mutation such as a deletion, substitution, addition or insertion can be carried out according to the methods described above.

KIAA1491 complete or a peptide derived therefrom may be prepared from a cell in which they are expressed by a genetic engineering technique, or may be obtained as the synthesized product of a cell-free system, a chemically synthesized product, or a substance prepared from a cell or a sample from a living organism, as well as the further purified products of these. Further, as long as there is no influence on the property or function thereof, for example, interaction with JNK3, such as inhibition of the phosphorylation of c-Jun by JNK3 or the phosphorylation by JNK3, KIAA1491 complete or the peptide derived therefrom may be labeled. The labeling may be performed by adding another protein or peptide or the like at the N-terminal or C-terminal thereof directly, or indirectly through a linker peptide or the like using a genetic engineering technique. Preferably, the labeling is conducted in a manner that does not inhibit the fundamental properties of KIAA1491 complete or the peptide derived therefrom. Examples of a protein or peptide or the like that may be added include, but are not limited to, an enzyme such as glutathione S-transferase, β-galactosidase, horseradish peroxidase (HRP) or alkaline phosphatase; a tag peptide such as His-tag, Myc-tag, HA-tag, FLAG-tag, or Xpress-tag; a fluorescent substance such as green fluorescent protein, fluorescein isothiocyanate or phycoerythrin, a maltose binding protein, Fc fragment of immunoglobulin, biotin and a radioisotope. At the time of labeling, the proteins and peptides and the like may be added independently or a plurality thereof may be combined and added. By measuring the protein or peptide, or the like that is used in the above labeling, or a function thereof, for example, it is possible to detect interaction between the peptides and JNK3. Detection or purification of the peptide of the present invention is also facilitated.

Of peptides derived from KIAA1491 complete, a peptide that interacts with JNK3 is useful as a substance that inhibits the phosphorylation of c-Jun by JNK3 and is also useful for identifying a compound that regulates interaction between JNK3 and KIAA1491 complete. A peptide that is immunologically recognizable, such as, for example, an epitope peptide, can be used as an antigen to produce an antibody for KIAA1491 complete, either by itself or by binding to a carrier (for example, keyhole limpet hemocyanin or egg albumin), as described hereunder.

The present invention provides a polynucleotide comprising the nucleotide sequence encoding KIAA1491 complete or the peptide derived therefrom, or comprising the complementary sequence thereof For example, the polynucleotide may be the polynucleotide comprising the nucleotide sequence encoding the peptide comprised of the amino acid sequence described in SEQ ID NO:1 in the Sequence List, or the complementary sequence thereof Preferably, the polynucleotide is comprised of a nucleotide sequence described in SEQ ID NO:7 in the Sequence List. The polynucleotides provide useful genetic information, for example, for producing KIAA1491 complete or a peptide derived therefrom. And the polynucleotides can also be used as standard preparations or reagents for the nucleic acids.

The present invension further provides a polynucleotide hybridizing under stringent conditions to a corresponding region of the polynucleotide comprising the nucleotide sequence encoding the amino acid sequence of KIAA1491 complete or the peptide derived therefrom, for example, the amino acid sequence described in SEQ ID NO:1 in the Sequence List, or the complementary sequence thereof. The hybridization conditions can be in accordance with, for example, a method described in publications (Non-patent Documents) or the like. The polynucleotide need not necessarily comprise the complementary sequence of a polynucleotide of interest, as long as it hybridizes to the polynucleotide of interest, such as a polynucleotide comprised of the nucleotide sequence described in SEQ ID NO:7 in the Sequence List or the complementary strand thereof. For example, the polynucleotide is preferably a polynucleotide that exhibits a homology of 85% or more to a nucleotide sequence of the gene encoding KIAA1491 complete or the complementary sequence thereof, more preferably 90% or more, and even more preferably 95% or more. The present invention also includes a polynucleotide or oligonucleotide comprised of 10 or more consecutive nucleotides located in the designated region of the nucleotide sequence of the polynucleotides, preferably 15 or more consecutive nucleotides, and more preferably 20 or more consecutive nucleotides. The polynucleotide may be a polynucleotide that includes a gene added at the 5'-terminal or C'-terminal thereof, such as, for example, a gene for expressing enzymes such as glutathione S-transferase, β-galactosidase, horseradish peroxidase (HRP) or alkaline phosphatase; tag-peptides such as His-tag, Myc-tag, HA-tag, FLAG-tag or Xpress-tag; and the green fluorescent protein, as long as the gene does not inhibit the function for expressing the encoded peptide or the function of an expressed peptide. The polynucleotides can be used for producing KIAA1491 complete or the peptide derived therefrom. They also can be used as probes or primers for detecting mRNA or the gene encoding KIAA1491 complete, or as antisense oligonucleotides or the like for controlling expression of the gene. In this respect, the polynucleotides include not only translation regions but also sequences corresponding to non-translation regions. In order to specifically inhibit the expression of the gene encoding KIAA1491 complete by the antisense oligonucleotide, it is preferable to use a nucleotide sequence of a characteristic region of the gene. The polynucleotide encoding KIAA1491 complete or the peptide derived therefrom can be obtained, for example, by verifying the expressed protein with a known protein expression system, using a physiological activity thereof as an indicator. For example, interaction with JNK3, specifically, inhibition of the phosphorylation of c-Jun by JNK3 or the phosphorylation by JNK3 can be used as an indicator. When a cell-free protein expression system is used as the expression system, for example, a technique using a ribosome system derived from embryo or rabbit reticulocyte or the like can be used (Non-patent Document 16).

Recombinant vectors can be obtained by incorporating the polynucleotides into appropriate vector DNA. The vector DNA may be appropriately selected according to the kind of host and purpose of use. The vector DNA may be a vector extracted from a natural source, or one in which a section of DNA other than a section required for proliferation is partially deleted. Examples of vector DNA include a vector derived from a chromosome, episome or virus, such as a vector derived from a bacteria plasmid, a bacteriophage, a transposon, a yeast episome, an inserted element or a yeast chromosome element, for example, a vector derived from a virus such as baculovirus, papovavirus, SV40, vaccinia virus, adenovirus, fowlpox virus, pseudo-rabies virus or retrovirus, as well as a combined vector thereof, or for example, a vector derived from a genetic element of a plasmid or bacteriophage, such as a cosmid or a phagemid. According to the purpose of use, an expression vector or a cloning vector or the like may be used.

The recombinant vector comprises the genetic sequence of interest and genetic sequences that contain information for duplication and control, such as a promoter, a ribosome-binding region, a terminator, a signal sequence and an enhancer. The recombinant vector is prepared by combining these by a known method. Recombining a polynucleotide into the vector DNA can be carried out in accordance with known methods. For example, a method may be used in which a suitable restriction enzyme is selected and treated to cleave DNA at a specific site, the cleaved DNA is subsequently mixed with vector DNA treated in the same manner to recombine using a ligase. Alternatively, a desired recombinant vector can be obtained by ligating a suitable linker to a polynucleotide of interest and inserting it into a multicloning site of a vector.

A transformant can be obtained by introducing a vector DNA into a known host by known methods. The vector DNA to be introduced into a host may be one single kind of vector DNA or a mixture of two or more kinds of vector DNA. Examples of a host include *Escherichia coli,* yeasts, grass bacillus, insect cells, and animal cells. Introduction of the vector DNA into a host cell may be carried out by applying well-known methods such as standard methods described in publications (Non-patent Document 8). Considering the stability of the gene, introducing vector DNA into a host cell by integration into the chromosome is a more preferred method. However, an autonomous replication system using an extranuclear gene can be used as a simpler method. Specific examples thereof include calcium phosphate transfection, DEAE-dextran mediating transfection, microinjection, positive lipid mediating transfection, electroporation, genetic transduction, scrape loading, ballistic introduction, and infection.

In the examples described hereinafter, an insect cell is used. However, the host is not limited thereto.

It is possible to obtain KIAA1491 complete or the peptide derived therefrom by a known genetic engineering technique based on information relating to the nucleotide sequence thereof, such as, for example, the nucleotide sequence described in SEQ ID NO:7 in the Sequence List. For example, an amplified gene of interest from a human brain-derived cDNA library may be obtained using a designed primer, according to the nucleotide sequence in question, that is synthesized by known methods. After inserting the amplified gene into an expression vector for introduction into a host cell to thereby construct a cell that expresses the peptide, the peptide of interest may be obtained from the cell by known methods for extracting and purifying. A known cell-free protein synthesis system can also be used for synthesizing. In the example described hereinafter, a gene expression system that uses an insect cell is used. KIAA1491 complete is obtained by producing a baculovirus having incorporated therein an ORF part of KIAA1491 complete that was amplified by PCR, and then infecting an insect cell, Sf9, with the baculovirus to induce expression. However, a method of acquiring KIAA1491 complete is not limited to the method described in this example.

For example, an expression vector may be used as vector DNA, and a polynucleotide encoding the peptide of interest may be inserted therein. A transformed cell, into which the obtained vector has been incorporated, is incubated after selecting optimal conditions according to the respective host, and then the peptide of interest is recovered and/or purified from the culture medium or the cell itself. Recovery and/or purification are carried out using a property or function of the peptide as an indicator. For example, inhibiting action on the phosphorylation of c-Jun by JNK3 or the phosphorylation by JNK3 can be employed as an indicator. In the case of KIAA1491 complete or the peptide derived therefrom, the phosphorylated protein of KIAA1491 complete or the peptide itself that is phosphorylated by JNK3 can be employed as an indicator. Examples of methods for recovery and/or purification include a technique for fractionation based on solubility difference using ammonium sulfate or alcohol or the like, gel filtration, ion column chromatography, and affinity chromatography. These methods may be used alone or in combination. Preferably, a method is used in which polyclonal antibodies or monoclonal antibodies specific to the peptides are prepared based on information concerning the amino acid sequences of the peptides, and recovery by specific adsorption is conducted using the antibodies.

Antibodies can be prepared using KIAA1491 complete or the peptide derived therefrom as an antigen. The antigen may be a peptide or fragments thereof. The antigen is composed of at least 8, preferably at least 10, more preferably at least 12, and even more preferably 15 or more amino acids. To prepare an antibody specific to KIAA1491 complete or a peptide derived therefrom, it is preferable to use a region comprised of an amino acid sequence specific to KIAA1491 complete or the peptide. The amino acid sequence of the region in question need not necessarily be homologous or identical to the sequence of KIAA1491 complete or the peptide. A site that is exposed to an outside of the tertiary structure thereof is preferable. Even if an amino acid sequence of the exposed site is discontinuous in the primary structure, the sequence is acceptable as long as the amino acid sequence is continuous in the exposed site. The antibody is not particularly limited as long as it immunologically binds to or recognizes the peptide. The presence or absence of the binding or recognition can be determined by known antigen-antibody binding reactions.

Known antibody production methods can be used to produce the antibody. The antibody can be obtained, for example, by inducing immunity such as a humoral response and/or cellular response after administration to an animal of the antigen with or without an adjuvant, by itself or in a condition where it is bound to a carrier. The carrier is not particularly limited, as long as it does not itself exhibit any adverse action to the host and increase the antigenicity. Examples of carriers include cellulose, polymerized amino acids, albumin, and keyhole limpet hemocyanin. Examples of adjuvants include Freund's complete adjuvant (FCA), Freund's incomplete adjuvant (FIA), Ribi (MPL), Ribi (TDM), Ribi (MPL+TDM), Bordetella pertussis vaccine, muramyl dipeptide (MDP), aluminum adjuvant (ALUM), and combinations thereof. As the immunized animal, a mouse, rat, goat, rabbit, horse or the like is preferably used.

A polyclonal antibody can be obtained by well-known antibody collection methods from the serum of an animal that was treated with an immunizing means. Antibody affinity chromatography is a preferred antibody collection method.

A monoclonal antibody can be produced by collecting antibody-producing cells (i.e., spleen or lymph cells derived from a lymph node) from an animal, that was treated with an immunizing means, and then introducing a well-known means for transforming the cells to permanently proliferating cells (for example, using myeloma cells such as P3-X63-Ag8). For example, the antibody producing cell and permanently proliferating cell are fused together by known methods to form a hybridoma. The hybridoma may be then cloned. After selecting, from the cloned hybridomas, a hybridoma that produces an antibody that specifically recognizes the peptide of interest the antibody may be collected from a culture solution of the selected hybridoma.

The thus-obtained polyclonal antibody or monoclonal antibody can be used as an antibody for purification, a reagent, a labeling marker or the like for the peptide.

A pharmaceutical composition that comprises as an active ingredient at least one member of KIAA1491 complete or peptides derived therefrom, a polynucleotide encoding any of these, a vector comprising the polynucleotide, a transformant transfected with the vector, and an antibody that recognizes any of the peptides, are also included within the scope of the present invention.

The present invention provides an effective means for identifying an agent for inhibiting activity or an agent for promoting activity of KIAA1491 complete, or an agent for inhibiting expression or an agent for promoting expression of KIAA1491 complete, by using KIAA1491 complete or the peptide derived therefrom, the polynucleotide comprising the nucleotide sequence encoding KIAA1491 complete or the complementary nucleotide sequence thereof, a vector comprising the polynucleotide, the transformant formed by introduction of the vector, the antibody that immunologically recognizes KIAA1491 complete or the peptide derived therefrom, and the protein synthesis system using them alone or in combination of a plurality thereof The method for identification can be constructed using well-known screening systems for pharmaceutical preparations. Examples of a test substance include a compound derived from a natural product or a chemical library, or a compound obtained by drug design based on the tertiary structure of KIAA1491 complete or the peptide derived therefrom.

Specifically, a compound that inhibits or promotes the activity of a peptide of the present invention can be identified by selecting conditions that enable specific interaction, such as contact between KIAA1491 complete or the peptide derived therefrom and the test substance, introducing thereto a system using a signal and/or marker that is capable of detecting the presence or absence of the interaction, and then detecting the presence, absence or change of the signal and/or the marker. For example, it is possible to screen for a compound that can regulate the interaction or the phosphorylation, by employing interaction between KIAA1491 complete or the peptide derived therefrom and JNK3, or the phosphorylation by JNK3 as the indicator. Specifically, for example, a test substance is added to coexist with KIAA1491 complete, JNK3 and ATP (adenosine triphosphate) to conduct the phosphorylation, and the resulting amount of phosphorylated KIAA1491 complete is quantified. When the amount of phosphorylated protein decreases in comparison with a result obtained from a reaction to which the test substance was not added, it can be determined that the test substance inhibits interaction between KIAA1491 complete and JNK3. In contrast, when the amount of phosphorylated protein increases, it can be determined that the test substance promotes interaction between KIAA1491 complete and JNK3. As a different example, a method for identification may be mentioned, in which a test substance is added to coexist with KIAA1491 complete, JNK3, c-Jun and ATP to conduct the phosphorylation, and the resulting amount of phosphorylated c-Jun is quantified. When the amount of phosphorylated c-Jun decreases in comparison with a result obtained from a reaction to which the test substance was not added, it can be determined that the test substance promotes interaction between KIAA1491 complete and JNK3. In contrast, when the amount of phosphorylated c-Jun increases, it can be determined that the test substance inhibits interaction between KIAA1491 complete and JNK3.

A compound that promotes or inhibits the expression of the polynucleotide encoding KIAA1491 complete or the peptide derived therefrom can be identified by selecting conditions that enable expression of the polynucleotide and allowing the test substance to be present therein, introducing thereto a system using a signal and/or marker that is capable of detecting the expression of the polynucleotide, and then detecting the presence, absence or change of the signal and/or marker. For example, a compound that influences the expression of a gene of the present invention can be identified by preparing a vector in which a reporter gene is connected in downstream of a promoter region of KIAA1491 complete gene in place of the polynucleotide encoding KIAA1491 complete, then contacting the test substance with a cell, such as an eukaryotic cell into which the vector was introduced, and finally, detecting the presence, absence or change of the reporter gene expression. When the expression amount of the reported gene increases in comparison to a case where the cell was not contacted with the test substance, it can be determined that the test substance promotes the expression of KIAA1491 complete. In contrast, when the expression amount decreases, it can be determined that the test substance inhibits the expression of KIAA1491 complete.

As used herein, the term "signal" refers to a substance that can itself be directly detected by means of the physical or chemical properties thereof, and the term "marker" refers to a substance that can be indirectly detected using the physical or biological properties thereof as an indicator. Examples of signals that can be used herein include known signals such as luciferase, GFP and radioactive isotopes, and examples of markers include known markers such as a reporter gene, for example, β-galactosidase or chloramphenicol acetyl transferase (CAT) gene, or a detection tag such as 6xHis-tag. Methods for detecting these signals or markers are known by those skilled in the art.

### EXAMPLES

Hereunder, the present invention is described in detail based on examples. However, the present invention is not limited to the following examples.

### Example 1

### (In silico search for proteins that interact with JNK3)

Prediction *in silico* of the proteins that interact with JNK3 was conducted according to a prediction method described in International Publication No. WO 01/67299 (Patent Document 1). First, the amino acid sequence of JNK3 was decomposed into oligopeptides having a pre-determined length in order to search in a database for proteins having the amino acid sequences of each of the oligopeptides, or having the homologous amino acid sequences to these amino acid sequences. Next, local alignment between the proteins obtained and JNK3 was conducted based on the prediction that the proteins having a high local alignment score might be those interacting with JNK3. The criterion for the local alignment score was the same as that in the method described in International Publication No. WO 01/67299, which is to say, more than 25.0. Since it is known that JNK3 is a protein that is specifically expressed in the cerebral nervous system and is expressed in a state of shock, such as hypoxia, and imparts damage to cerebral function, the candidate proteins interacting with JNK3 were narrowed down to proteins that are known to be expressed in the brain and are known to have an important function.

As a result, it was found that the oligopeptide QTFDKQ (SEQ ID NO: 12), which has homology to the oligopeptide QGFDKQ (SEQ ID NO: 11) comprising six amino acid residues derived from JNK3, is present in the amino acid sequence of the protein KIAA1491 (SEQ ID NO: 6) with unknown function. Figure 1 shows the results of local alignment between JNK3 (the upper sequences in the figure) and KIAA1491 (the lower sequences in the figure).

### (In-silico determination of full-length KIAA1491 sequence)

The HUGE database of Kazusa DNA Research Institute indicated the possibility that the N-terminus of KIAA1491 (SEQ ID NO: 6) was deleted. Therefore the present inventors carried out an *in-silico* prediction of the cDNA sequence encoding a region on the N-terminal side. First, 120 bases at the 5'-terminus of KIAA1491 were searched using BLASTN, whereby it was found to have 100% homology with 120 bases of QV1-MT0132-201100-498-b05 MTO132 Homo sapiens cDNA (GenBank accession number: BF894928). Since bases 1 to 120 of KIAA1491 corresponded to bases 358 to 477 of BF894928, it was considered that BF894928 was an upstream sequence of KIAA1491. Next, by taking as a first methionine the second methionine from upstream obtained by *in-silico* translation of BF894928 using the same frame as KIAA1491, it was confirmed through RT-PCR using human brain-derived poly-A RNA that the corresponding mRNA was present in brain. The cDNA extending across the upstream region until the methionine was denoted as "KIAA1491 complete cDNA" (SEQ ID NO: 7) and the full-length KIAA1491 that includes the amino acid sequence up to the methionine was denoted as "KIAA1491 complete" (SEQ ID NO: 1).

### (Analysis of action of KIAA1491 complete on the phosphorylation of c-Jun by JNK3)

### <Materials>

KIAA1491 complete was expressed as an N-terminal GST-fusion protein (hereafter, referred to as "GST-KIAA1491 complete") in *Escherichia coli,* and then purified using Glutathione Sepharose 4B (Amersham Pharmacia Biotech) in preparation for use.

Specifically, after amplifying the ORF region of KIAA1491 complete cDNA by PCR using cDNA from human brain as a template, the amplification product was integrated into a pDEST15 vector (Invitrogen Corp.) using the Gateway Cloning Technology (Invitrogen Corp.) cloning system to construct a GST-KIAA1491 complete expression vector for *Escherichia coli.*

After introducing the vector into *E. coli* BL21-SI strain (Invitrogen) and culturing at 37°C in LBON medium (LB medium without NaCl) containing 100 µg/ml of ampicillin, NaCl was added thereto to bring to a final concentration of 0.3 M, followed by further culturing at 25°C to induce expression of GST-KIAA1491 complete. The bacterial cells were recovered and an extract was prepared, and then the GST-KIAA1491 complete was purified using Glutathione Sepharose 4B. The purified protein was dialyzed in preparation for use.

Activated human JNK3 was prepared as a protein with an N-terminal histidine-tag (His-tag) (hereunder, referred to as "His-JNK3"). First, human JNK3 (JNK3α1) cDNA, obtained by RT-PCR using a human hippocampus cDNA library as a template, was inserted into pFastBac HT (Invitrogen Corp.) to produce a recombinant baculovirus for expression of His-JNK3, as instructed by the manufacturer. Next, Sf9 cells were infected with the recombinant virus to express His-JNK3, and the His-JNK3 was purified with Probond Resin (Invitrogen Corp.) and used.

In an *in vitro* kinase assay of c-Jun, a peptide (c-Jun (1-79)) comprising a 79-amino acid region at the N-terminus of c-Jun was used in place of c-Jun. The c-Jun (1-79) was expressed as an N-terminal GST fusion protein (hereunder, referred to as "GST-c-Jun (1-79)") in *E. Coli,* and then purified using Glutathione Sepharose 4B (Amersham Pharmacia Biotech) in preparafion for use.

### <In vitro kinase assay>

The phosphorylation reaction was performed by incubating 1 µg of each of the GST fusion proteins (GST-KIAA1491 complete, GST-c-Jun (1-79), or GST) and activated JNK3 (0, 14, 28, or 70 ng) in a kinase buffer (25 mM Tris-HCl, pH 7.5 / 5 mM β-glycerophosphate / 2 mM dithiothreitol (DTT) / 0.1 mM Na₃VO₄ / 10 mM MgCl₂ / 10 µM ATP) containing 5 µCi of [γ-³²P] ATP (3000 Ci/mmol, NEN Life Science Products) at 30°C for 30 min. After the reaction, an equivalent amount of 2x SDS sample buffer (4% SDS / 125 mM Tris-HCl, pH6.8 / 20% glycerol / 0.01% bromophenol blue (BPB) / 10% β-mercaptoethanol) was added and treated for 5 min at 100°C, and the protein was then separated using 5-20% SDS-PAGE to detect the phosphorylated protein by autoradiography using BAS2000 (Fuji Photo Film Co., Ltd.) (Figure 2A and Figure 2B).

Next, the phosphorylation reaction was performed by incubating 1 µg of GST-c-Jun (1-79) and activated JNK3 (70 ng) in a kinase buffer containing 100 µM of ATP at 30°C for 20 min in the presence or absence of GST or GST-KIAA1491 complete (both consisting of 1 µg or 2 µg). After the reaction, an equivalent amount of 2x SDS sample buffer was added, the resulting mixture was treated for 5 min at 100°C, the supernatant was separated using 10% SDS-PAGE, and phosphorylated GST-c-Jun (1-79) was detected by immunoblotting using anti-Phospho-c-Jun (S63) antibody (New England Biolabs Inc.) as the primary antibody and HRP-conjugated anti-rabbit IgG antibody (Amersham Pharmacia Biotech, Inc.) as the secondary antibody (Figure 3). An ECL Western Blotting Detection Kit (Amersham Pharmacia Biotech, Inc.) was used for the detection. The band strength of the detected phosphorylated GST-c-Jun (1-79) was quantified using image analysis software, Intelligent Quantifier (Bio Image Inc.), and the band strengths in the presence of GST or GST-KIAA1491 complete were shown as relative strengths when the strength in the absence of GST and GST-KIAA1491 complete was taken as 100% (Table 1).

### <Results>

c-Jun and GST-KIAA1491 complete were phosphorylated by JNK3 (Figure 2A and Figure 2B). Since the phosphorylation was dependent on the dose of JNK3 (Figure 2B), it was clarified that the phosphorylation of GST-KIAA1491 complete was not self-phosphorylation, but was caused by JNK3. It was thus determined that JNK3 and KIAA1491 complete interact with each other.

Further, as shown in Figure 3 and Table 1, in the presence of 1 µg or 2 µg of GST-KIAA1491 complete, the phosphorylation of GST-c-Jun (1-79) by JNK3 was inhibited approximately 30%. In contrast, in the presence of GST as a negative control, there was no change in the phosphorylation of GST-c-Jun (1-79) by JNK3. It was thus clarified that the phosphorylation of GST-c-Jun (1-79) by JNK3 is inhibited by KIAA1491 complete.

**Table 1**

| Added Protein | Added Amount (µg) | Relative Strength (%) |
|---|---|---|
| None | | 100 |
| GST | 1.0 | 92.7 |
| | 2.0 | 93.6 |
| GST-KIAA1491 complete | 1.0 | 71.5 |
| | 2.0 | 67.1 |

### Example 2

### (In-silico search for proteins that interact with JNK3)

Predictions of the proteins that interact with JNK3 were conducted *in silico* in the same manner as in Example 1. As a result, it was found that the oligopeptide SLPPAD (SEQ ID NO: 14), which has homology with the oligopeptide SLFPAD (SEQ ID NO: 13) comprising six amino acid residues derived from JNK3 is present in the amino acid sequence of a protein KIAA0596 (GenBank accession number: AB011168) with unknown function. Figure 4 shows the results of local alignment between JNK3 and KIAA0596.

### (Analysis of action of KIAA0596 on the phosphorylation of c-Jun by JNK3)

It was clarified that KIAA0596 exhibits a homology of 74% to mouse JNK-binding protein 1 (JNKBP1, Non-patent Document 4) (GenBank accession number: AB029482), which is reported as binding with JNK3. It is known that the C-terminal region of mouse JNKBP1 is required for the binding thereof to JNK3. Therefore, binding between a C-terminal region of KIAA0596 (amino acid sequence from amino acid 639 to 1217, hereafter referred to as "KIAA0596CT") and JNK3 was analyzed by a far-Western method. Further, the action of KIAA0596CT on the phosphorylation of c-Jun by activated JNK3 was assayed.

### <Materials>

The human JNK3 used in the binding assay was prepared as a fusion protein (MBP-JNK3) with an N-terminal maltose binding protein. First, human JNK3 (JNK3α1) cDNA, obtained by RT-PCR using a human hippocampus cDNA library as a template, was inserted into pMAL-c2 (New England Biolabs Inc.) to construct an MBP-JNK3 expression vector as instructed by the manufacturer. The vector was introduced into *E*. *coli* DH5α strain, and expression of MBP-JNK3 was induced. Bacterial cells were recovered and the extract was prepared, and then the MBP-JNK3 was purified using amylose resin. The purified protein was dialyzed in preparation for use.

The activated human JNK3 used in the kinase assay was prepared as a protein with an N-terminal His-tag (hereunder, referred to as "His-JNK3") in the same manner as in Example 1.

Human KIAA0596CT was expressed in *E*. *coli* as an N-terminal GST fusion protein (hereunder, referred to as "GST-KIAA0596CT") and then purified with Glutathione Sepharose 4B (Amersham Pharmacia Biotech, Inc.) in preparation for use.

Specifically, first, a region from nucleotide 1916 to 3655 in the nucleotide sequence of KIAA0596 was amplified by PCR using a KIAA0596 clone (purchased from Kazusa DNA Research Institute) as a template. Next, the amplification product was integrated into a pDEST15 vector (Invitrogen Corp.) using Gateway Cloning Technology (Invitrogen Corp.) to construct a GST-KIAA0596CT expression vector for E. *coli.*

After introducing the vector into E. *coli* BL21-SI strain (Invitrogen Corp.) and culturing at 37°C in LBON medium (LB medium without NaCl) containing 100 µg/ml of ampicillin, NaCl was added thereto to a final concentration of 0.3 M, followed by further culturing at 25°C to induce expression of GST-KIAA0596CT. Bacterial cells were recovered and the extract was prepared, and then the GST-KIAA0596CT was purified using Glutathione Sepharose 4B. The purified protein was dialyzed in preparation for use.

### <Binding assay>

After spotting 1 µg of GST or GST-KIAA0596CT on nitrocellulose membranes (Schleicher & Schuell BioScience, BA85), the membranes were incubated at room temperature in TBST (10 mM Tris-HCl, pH 7.5 / 0.15M NaCl / 0.05% Tween 20) containing 5% skim milk. After rinsing the membranes with TBST, they were incubated overnight at 4°C in TBST containing 1.0 µg/ml of MBP-JNK3 or MBP. After washing the membranes with TBST, bound MBP-JNK3 was detected by immunoblotting using anti-MBP antiserum (New England Biolabs Inc.) as the primary antibody, and HRP-conjugated anti-rabbit IgG antibody (Amersham Pharmacia Biotech, Inc.) as the secondary antibody (Figure 5). An ECL Western Blotting Detection Kit (Amersham Pharmacia Biotech, Inc.) was used for the detection.

### <In vitro kinase assay>

The phosphorylation reaction was performed by incubating 1 µg of GST-c-Jun (1-79) and activated JNK3 (70 ng) in a kinase buffer (25 mM Tris-HCl, pH 7.5 / 5 mM β-glycerophosphate / 2 mM DTT / 0.1 mM Na₃VO₄ / 10 mM MgCl₂) containing 100 µM of ATP at 30°C for 30 min in the presence or absence of GST or GST-KIAA0596CT (both consisting of 1 µg or 2 µg). After the reaction, an equivalent amount of 2x SDS sample buffer (4% SDS / 125 mM Tris-HCl, pH 6.8 / 20% glycerol / 0.01% BPB / 10% β-mercaptoethanol) was added and treated for 5 min at 100°C, the supernatant was separated using 10% SDS-PAGE, and phosphorylated GST-c-Jun (1-79) was then detected by immunoblotting using anti-Phospho-c-Jun (S63) antibody (New England Biolabs Inc.) as the primary antibody, and HRP-conjugated anti-rabbit IgG antibody (Amersham Pharmacia Biotech, Inc.) as the secondary antibody (Figure 6). An ECL Western Blotting Detection Kit (Amersham Pharmacia Biotech, Inc.) was used for the detection. The band strength of the detected phosphorylated GST-c-Jun (1-79) was quantified using image analysis software, Intelligent Quantifier (Bio Image Inc.), and the band strengths in the presence of GST or GST-KIAA0596CT were shown as relative strengths when the strength in the absence of GST and GST-KIAA0596CT was taken as 100% (Table 2).

### <Results>

As shown in Figure 5, binding was observed between GST-KIAA0596CT and MBP-JNK3. Since binding between GST-KIAA0596CT and MBP and binding between GST and MBP-JNK3 was not observed, it was confirmed that the detected binding between GST-KIAA0596CT and MBP-JNK3 was specific.

Further, as shown in Figure 6 and Table 2, in the presence of 1 µg and 2 µg of KIAA0596CT, the phosphorylation of GST-c-Jun (1-79) by JNK3 was inhibited by approximately 30% and 67%, respectively. In contrast, in the presence of GST as a negative control, there was no change in the phosphorylation of GST-c-Jun (1-79) by JNK3. It was thus clarified that the phosphorylation of GST-c-Jun (1-79) by JNK3 is dose-dependently inhibited by KIAA0596CT.

**Table 2**

| Added Protein | Added Amount (µg) | Relative Strength (%) |
|---|---|---|
| None | | 100 |
| GST | 1.0 | 92.7 |
| | 2.0 | 93.6 |
| GST-KIAA0596CT | 1.0 | 70.6 |
| | 2.0 | 33.5 |

### Example 3

### (In-silico search for proteins that interact with JNK3)

Prediction *in silico* of proteins that interact with JNK3 was conducted in the same manner as in Example 1. As a result, it was found that the oligopeptide KVKEQL (SEQ ID NO: 16), which has homology to the oligopeptide KVIEQL (SEQ ID NO: 15) comprising six amino acid residues derived from JNK3, is present in the amino acid sequence of a protein, BMAL1, that is associated with circadian rhythm. In addition, it was found that the oligopeptide KVKEQL (SEQ ID NO: 16) is also present in the amino acid sequence of BMAL2, a homologous protein of BMAL1. Figures 7 and 8 show the results of local alignment between JNK3 and BMAL1, and JNK3 and BMAL2. Fragments exhibiting a score of more than 25.0 were found in 7 cases in the local alignment between JNK3 and BMAL1, and in 4 cases between JNK3 and BMAL2. It was thus predicted that JNK3 is a protein that interacts more strongly with BMAL1 than with BMAL2.

### (Analysis of action of BMAL1 on the phosphorylation of c-Jun by JNK3)

### <Materials>

Activated human JNK3 was prepared as a protein with an N-terminal histidine-tag (His-JNK3) in the same manner as in Example 1.

c-Jun (1-79) was prepared as an N-terminal GST fusion protein (hereunder, referred to as "GST-c-Jun (1-79)") in the same manner as in Example 1.

Human BMAL1 was expressed in *E*. *coli* as an N-terminal GST fusion protein (hereunder, referred to as "GST-BMAL1") and then purified using Glutathione Sepharose 4B (Amersham Pharmacia Biotech) in preparation for use. In this example, BMAL1b (SEQ ID NO: 4) was used as the BMAL1.

More specifically, after amplifying by PCR the ORF region of BMAL1 from pcDNA3.1-BMAL1/V5-His (Invitrogen Corp.), which is a human BMAL1 expression plasmid with a C-terminal V5/His-tag added, the amplified product was inserted into pGEX-4T (Amersham Pharmacia Biotech, Inc.) to construct a GST-BMAL1 expression vector for *E*. *coli*. The obtained nucleotide sequence (SEQ ID NO: 9) was confirmed by direct sequencing according to a common method.

Next, after culturing *E*. *coli* BL21 strain, into which the above expression vector was introduced, at 37°C in LB medium containing 100 µg/ml of ampicillin, isopropyl-1-thio-β-D-galactopyronoside (IPTG) was added to a final concentration of 0.3 mM, followed by further culturing at 25°C before recovering bacterial cells. The extract was prepared from these bacterial cells using TGEDS buffer (50 mM Tris-HCl, pH 8.0 /0.2 mM ethylenediaminetetraacetic acid (EDTA) / 1 mM DTT / 150 mM NaCl / 10 % glycerol) containing a protease inhibitor cocktail, and the GST-BMAL1 was purified using Glutathione Sepharose 4B. The purified GST-BMAL1 was used after dialysis with TGEDS buffer / 0.1% Triton X-100.

### <In vitro kinase assay>

The phosphorylation reaction was performed by incubating 1 µg of each of the above GST fusion proteins (GST-BMAL1, GST-c-Jun (1-79), or GST) and activated JNK3 (70 ng) in a kinase buffer (25 mM Tris-HCl, pH 7.5 / 5 mM β-glycerophosphate / 2 mM DTT / 0.1 mM Na₃VO₄ / 10 mM MgCl₂ / 10 µM ATP) containing 5 µCi of [γ-³²P] ATP (3000 Ci/mmol, NEN Life Science Products) at 30°C for 30 min. After the reaction, an equivalent amount of 2x SDS sample buffer (4% SDS / 125 mM Tris-HCl, pH 6.8 /20% glycerol / 0.01% BPB / 10% β-mercaptoethanol) was added and treated for 5 min at 100°C, and the protein was then separated by 5-20% SDS-PAGE Phosphorylated protein was detected by autoradiography using BAS2000 (Fuji Photo Film Co., Ltd.) (Figure 9B). Further, after separating each of the used GST fusion proteins (1 µg) by 5-20% SDS-PAGE, the mobility of the proteins of interest was confirmed by CBB staining (Figure 9A). In order to confirm JNK3 dose-dependency in the phosphorylation reaction of GST-BMAL1, activated JNK3 was added to the reaction system in an amount of 0 ng, 14 ng, 28 ng or 70 ng, and phosphorylated protein was then detected by a similar method (Figure 10).

Next, the phosphorylation reaction was performed by incubating 1 µg of GST-c-Jun (1-79) and activated JNK3 (70 ng) in a kinase buffer containing 100 µM of ATP at 30°C for 20 min in the presence or absence of GST or GST-BMAL1 (both consisting of 1 µg or 2 µg). After the reaction, the reaction product was treated in a sample buffer in the same manner as described above, the supernatant was separated by 10% SDS-PAGE, and phosphorylated GST-c-Jun (1-79) was detected by immunoblotting using anti-Phospho-c-Jun antibody (New England Biolabs Inc.) as the primary antibody, and HRP-conjugated anti-rabbit IgG antibody (Amersham Pharmacia Biotech, Inc.) as the secondary antibody (Figure 11). An ECL Western Blotting Detection Kit (Amersham Pharmacia Biotech, Inc.) was used for the detection. The band strength of the detected phosphorylated GST-c-Jun (1-79) was quantified using image analysis software, Intelligent Quantifier (Bio Image Inc.), and the band strengths in the presence of GST or GST-BMAL1 were shown as relative strengths when the strength in the absence thereof was taken as 100% (Table 3).

### <Results>

As shown in Figure 9B, the phosphorylation of GST-c-Jun (1-79) by JNK3 was observed. Since phosphorylation was not observed in the GST as a negative control, it was confirmed that the assay system used in this example is appropriate for measuring JNK3 activity. In this assay system, the phosphorylation of GST-BMAL1 by JNK3 was observed. Further, since the phosphorylation was dependent on the dose of JNK3, it was clarified that the phosphorylation of GST-BMAL1 was not self-phosphorylation, but was caused by JNK3 (Figure 10). It was thus determined that JNK3 and BMAL1 interact each other.

In addition, as shown in Figure 11 and Table 3, in the presence of 1µg and 2 µg of GST-BMAL1, phosphorylation of GST-c-Jun (1-79) by JNK3 decreased to as far as approximately 15% and 5%, respectively. In contrast, there was no change in the phosphorylation of GST-c-Jun (1-79) by JNK3 in the presence of GST. It was thus clarified that the phosphorylation of GST-c-Jun (1-79) by JNK3 is inhibited by BMAL1.

**Table 3**

| Added Protein | Added Amount (µg) | Relative Strength (%) |
|---|---|---|
| None | | 100 |
| GST | 1.0 | 92.7 |
| | 2.0 | 93.6 |
| GST-BMAL1 | 1.0 | 15.3 |
| | 2.0 | 4.7 |

### Example 4

### (In-silico search for proteins that interact with JNK3)

Prediction *in silico* of proteins that interact with JNK3 was conducted in the same manner as in Example 1. As a result, it was found that oligopeptides LPPSSN (SEQ ID NO: 19) and AVLCQV (SEQ ID NO: 20), which have homology to oligopeptides LPPSSS (SEQ ID NO: 17) and ANLCQV (SEQ ID NO: 18) comprising six amino acid residues derived from JNK3, are present in the amino acid sequence of BPL1, which is a holocarboxylase synthetase. Figure 12 shows the results of local alignment between JNK3 and BPL1. Eight fragments exhibiting a score of more than 25.0 were found for the local alignment between JNK3 and BPL1. It was thus predicted that JNK3 and BPL1 are interacting proteins.

### (Analysis of action of BPL1 on the phosphorylation of c-Jun by JNK3)

### <Materials>

Activated human JNK3 was prepared as a protein with an N-terminal histidine-tag (His-JNK3) in the same manner as in Example 1.

c-Jun (1-79) was prepared as an N-terminal GST fusion protein (hereunder, referred to as "GST-c-Jun (1-79)") in the same manner as in Example 1.

Human BPL1 was expressed as an N-terminal GST fusion protein (hereafter, referred to as "GST-BPL1") in *E*. *coli*, and then purified using Glutathione Sepharose 4B (Amersham Pharmacia Biotech, Inc.) in preparation for use. In this example, a peptide comprised of the amino acid sequence described in SEQ ID NO: 5 was used as the BPL1.

More specifically, after amplifying by PCR the ORF region of BPL1 from pcDNA3.1-BPL1/V5-His (Invitrogen Corp.), which is a human BPL1 expression plasmid with the C-terminal V5/His-tag added, the amplified product was inserted into pGEX-4T (Amersham Pharmacia Biotech, Inc.) to construct a GST-BPL1 expression vector for *E*. *coli.* The obtained nucleotide sequence (SEQ ID NO: 10) was confirmed by direct sequencing according to a common method.

Next, after culturing *E*. *coli* BL21 strain into which the above expression vector was introduced, at 37°C in LB medium containing 100 µg/ml of ampicillin, IPTG was added thereto to a final concentration of 0.1 mM, followed by further culturing at 25°C before recovering bacterial cells. An extract was prepared from these bacterial cells using TGEDS buffer (50 mM Tris-HCl, pH 8.0 / 0.2 mM EDTA / 1 mM DTT / 150 mM NaCl /10 % glycerol) containing a protease inhibitor cocktail, and the GST-BPL1 was purified using Glutathione Sepharose 4B. The purified GST-BPL1 was dialyzed using a TGEDS buffer in preparation for use.

### <In vitro kinase assay>

The phosphorylation reaction was performed by incubating 1 µg of each GST fusion protein (GST-BPL1, GST-c-Jun (1-79), or GST) and activated JNK3 (0, 14, 28 or 70 ng) in a kinase buffer (25 mM Tris-HCl, pH 7.5 / 5 mM glycerophosphate / 2 mM DTT / 0.1 mM Na₃VO₄ / 10 mM MgCl₂ / 10 µM ATP) containing 5 µCi of [γ-³²P] ATP (3000 Ci/mmol, NEN Life Science Products) at 30°C for 30 min. After the reaction, an equivalent amount of 2x SDS sample buffer (4% SDS / 125 mM Tris-HCl, pH 6.8 / 20% glycerol / 0.01% BPB / 10% β-mercaptoethanol) was added and treated for 5 min at 100°C, and the protein was then separated by 5-20% SDS-PAGE. Phosphorylated protein was detected by autoradiography using BAS2000 (Fuji Photo Film Co., Ltd.).

Next, the phosphorylation reaction was conducted by incubating 1 µg of GST-c-Jun (1-79) and activated JNK3 (70 ng) in a kinase buffer containing 5 µCi of [γ-³²P] ATP (3000 Ci/mmol, NEN Life Science Products) at 30°C for 30 min in the presence or absence of GST or GST-BPL1 (both consisting of 1 µg or 2 µg). After the reaction, an equivalent amount of 2x SDS sample buffer was added, and after treatment for 5 min at 100°C, the supernatant was separated by 10% SDS-PAGE, and the phosphorylated GST-c-Jun (1-79) was detected by immunoblotting using anti-Phospho-c-Jun (S63) antibody (New England Biolabs Inc.) as the primary antibody, and HRP-conjugated anti-rabbit IgG antibody (Amersham Pharmacia Biotech, Inc.) as the secondary antibody (Figure 13). An ECL Western Blotting Detection Kit (Amersham Pharmacia Biotech, Inc.) was used for the detection. The band strength of the detected phosphorylated GST-c-Jun (1-79) was quantified using image analysis software, Intelligent Quantifier (Bio Image Inc.), and the band strengths in the presence of GST or GST-BPL1 were shown as relative strengths when the strength in the absence thereof was taken as 100% (Table 3).

### <Results>

The phosphorylation of GST-BPL1 by JNK3 was observed. Since the phosphorylation was dependent on the dose of JNK3, it was clarified that the phosphorylation of GST-BPL1 was not self-phosphorylation, but was caused by JNK3. It was thus determined that JNK3 and BPL1 interact with each other.

As shown in Figure 13 and Table 4, in the presence of 1 µg and 2 µg of GST-BPL1, the phosphorylation of GST-c-Jun (1-79) by JNK3 was inhibited by approximately 20% and 40%, respectively. In contrast, in the presence of GST, there was no change in the phosphorylation of GST-c-Jun (1-79) by JNK3. It was thus clarified that the phosphorylation of GST-c-Jun (1-79) by JNK3 is dose-dependently inhibited by BPL1.

**Table 4**

| Added Protein | Added Amount (µg) | Relative Strength (%) |
|---|---|---|
| None | | 100 |
| GST | 1.0 | 92.7 |
| | 2.0 | 93.6 |
| GST-BPL1 | 1.0 | 81.6 |
| | 2.0 | 59.7 |

### POSSIBILITIES FOR INDUSTRIAL USE

The present invention provides KIAA1491 complete (SEQ ID NO:1), KIAA0596CT (SEQ ID NO:2), KIAA0596 (SEQ ID NO:3), BMAL1, and BPL1 which have a function for interacting with JNK3, and hence allows utilizing these peptides to inhibit the phosphorylation of c-Jun by JNK3, c-Jun is known to be a transactivator that has a transcription activation activity. Additionally, JNK3 is known to play a role in diseases that involve neurocyte death, such as polyglutamine disease and Alzheimer's disease. Accordingly, it is anticipated that one or more of KIAA1491 complete (SEQ ID NO:1), KIAA0596CT (SEQ ID NO:2), KIAA0596 (SEQ ID NO:3), BMAL1, and BPL1, which have a function for interacting with JNK3, can be used to inhibit the phosphorylation of c-Jun by JNK3, so that the ability to activate transcripition of c-Jun may be suppressed and further, so that a physiological phenomenon caused by the phosphorylation of c-Jun by JNK3, such as apoptosis, may be suppressed.

The present invention allows utilizing KIAA1491 complete (SEQ ID NO:1), KIAA0596CT (SEQ ID NO:2), KIAA0596 (SEQ ID NO:3), BMAL1, and BPL1, which have a function for interacting with JNK3, as well as a peptide derived therefrom to inhibit the phosphorylation and functions of c-Jun (such as transcription activation activity), resulting in suppressing apoptosis (such as preventing neurocyte apoptosis). Thus, the present invention also can provide a drug, a pharmaceutical composition and a method for use in preventing and/or treating a neurodegenerative disease (such as polyglutamine disease and Alzheimer's disease). The present invention is also very useful for researching the JNK signaling pathway and its function.

### FREE TEXT IN SEQUENCING LISTING

SEQ ID NO:11: Partial oligopeptide of JNK3 showing a high score in the local alignment between JNK3 and KIAA1491 (SEQ ID NO:6).
SEQ ID NO:12: Partial oligopeptide of KIAA1491 showing a high score in the local alignment between JNK3 and KIAA1491 (SEQ ID NO:6).
SEQ ID NO:13: Peptide comprised of 6 consecutive amino acid residues in the amino acid sequence of JNK3.
SEQ ID NO:14: Partial peptide of KIAA0596 (SEQ ID NO:3) that has high homology to a peptide comprised of the amino acid sequence represented by SEQ ID NO:9.
SEQ ID NO:15: Peptide comprised of 6 consecutive amino acid residues in the amino acid sequence of JNK3.
SEQ ID NO:16: Partial peptide of BMAL1 or BMAL2 that has high homology to a peptide comprised of the amino acid sequence represented by SEQ ID NO:11.
SEQ ID NO:17: Peptide comprised of 6 consecutive amino acid residues in the amino acid sequence of JNK3.
SEQ ID NO:18: Peptide comprised of 6 consecutive amino acid residues in the amino acid sequence of JNK3.
SEQ ID NO:19: Partial peptide of BPL1 that has a high homology to a peptide comprised of the amino acid sequence represented by SEQ ID NO: 13.
SEQ ID NO:20: Partial peptide of BPL1 that has a high homology to a peptide comprised of the amino acid sequence represented by SEQ ID NO:14.
SEQ ID NO:21: Partial oligopeptide of JNK3 showing a high score in the local alignment between JNK3 and KIAA1491 (SEQ ID NO:6).
SEQ ID NO:22: Partial oligopeptide of KIAA1491 showing a high score in the local alignment between JNK3 and KIAA1491 (SEQ ID NO:6).
SEQ ID NO:23: Partial oligopeptide of JNK3 showing a high score in the local alignment between JNK3 and KIAA1491 (SEQ ID NO:6).
SEQ ID NO:24: Partial oligopeptide of KIAA1491 showing a high score in the local alignment between JNK3 and KIAA1491 (SEQ ID NO:6).
SEQ ID NO:25: Partial sequence which is common in sequence between JNK3 and KIAA1491 (SEQ ID NO:6).
SEQ ID NO:26: Partial oligopeptide of JNK3 showing a high score in the local alignment between JNK3 and KIAA1491 (SEQ ID NO:6).
SEQ ID NO:27: Partial oligopeptide of KIAA1491 showing a high score in the local alignment between JNK3 and KIAA1491 (SEQ ID NO:6).
SEQ ID NO:28: Partial sequence which is common in sequence between JNK3 and KIAA1491 (SEQ ID NO: 6).
SEQ ID NO:29: Partial oligopeptide of JNK3 showing a high score in the local alignment between JNK3 and KIAA1491 (SEQ ID NO:6).
SEQ ID NO:30: Partial oligopeptide of KIAA1491 showing a high score in the local alignment between JNK3 and KIAA1491 (SEQ ID NO:6).
SEQ ID NO:31: Partial sequence which is common in sequence between JNK3 and KIAA1491 (SEQ ID NO:6).
SEQ ID NO:32: Partial oligopeptide of JNK3 showing a high score in the local alignment between JNK3 and KIAA1491 (SEQ ID NO:6).
SEQ ID NO:33: Partial oligopeptide of KIAA1491 showing a high score in the local alignment between JNK3 and KIAA1491 (SEQ ID NO:6).
SEQ ID NO:34: Partial oligopeptide of JNK3 showing a high score in the local alignment between JNK3 and KIAA1491 (SEQ ID NO:6).
SEQ ID NO:35: Partial oligopeptide of KIAA1491 showing high score in the local alignment between JNK3 and KIAA1491 (SEQ ID NO:6).
SEQ ID NO:36: Partial oligopeptide of JNK3 showing a high score in the local alignment between JNK3 and KIAA0596 (SEQ ED NO:3).
SEQ ID NO:37: Partial oligopeptide of KIAA0596 showing a high score in the local alignment between JNK3 and KIAAo596 (SEQ ID NO:3).
SEQ ID NO:38: Partial oligopeptide of JNK3 showing a high score in the local alignment between JNK3 and KIAA0596 (SEQ ID NO:3).
SEQ ID NO:39: Partial oligopeptide of KIAA0596 showing a high score in the local alignment between JNK3 and KIAA0596 (SEQ ID NO:3).
SEQ ID NO:40: Partial oligopeptide of JNK3 showing a high score in the local alignment between JNK3 and KIAA0596 (SEQ ID NO:3).
SEQ ID NO:41: Partial oligopeptide of KIAA0596 showing a high score in the local alignment between JNK3 and KIAA0596 (SEQ ID NO:3).
SEQ ID NO:42: Partial oligopeptide of JNK3 showing a high score in the local alignment between JNK3 and KIAA0596 (SEQ ID NO:3).
SEQ ID NO:43: Partial oligopeptide of KIAA0596 showing a high score in the local alignment between JNK3 and KIAA0596 (SEQ ID NO:3).
SEQ ID NO:44: Partial oligopeptide of JNK3 showing a high score in the local alignment between JNK3 and KIAA0596 (SEQ ID NO:3).
SEQ ID NO:45: Partial oligopeptide of KIAA0596 showing a high score in the local alignment between JNK3 and KIAA0596 (SEQ ID NO:3).
SEQ ID NO:46: Partial oligopeptide of JNK3 showing a high score in the local alignment between JNK3 and KIAA0596 (SEQ ID NO:3).
SEQ ID NO:47: Partial oligopeptide of KIAA0596 showing a high score in the local alignment between JNK3 and KIAA0596 (SEQ ID NO:3).
SEQ ID NO:48: Partial oligopeptide of JNK3 showing a high score in the local alignment between JNK3 and KIAA0596 (SEQ ID NO:3).
SEQ ID NO:49: Partial oligopeptide of KIAA0596 showing a high score in the local alignment between JNK3 and KIAA0596 (SEQ ID NO:3).
SEQ ID NO:50: Partial oligopeptide of JNK3 showing a high score in the local alignment between JNK3 and BMAL1.
SEQ ID NO:51: Partial oligopeptide of BMAL1 showing a high score in the local alignment between JNK3 and BMAL1.
SEQ ID NO:52: Partial oligopeptide of JNK3 showing a high score in the local alignment between JNK3 and BMAL1.
SEQ ID NO:53: Partial oligopeptide of BMAL1 showing a high score in the local alignment between JNK3 and BMAL1.
SEQ ID NO:54: Partial oligopeptide of JNK3 showing a high score in the local alignment between JNK3 and BMAL1.
SEQ ID NO:55: Partial oligopeptide of BMAL1 showing a high score in the local alignment between JNK3 and BMAL1.
SEQ ID NO:56: Partial oligopeptide of JNK3 showing a high score in the local alignment between JNK3 and BMAL1.
SEQ ID NO:57: Partial oligopeptide of BMAL1 showing a high score in the local alignment between JNK3 and BMAL1.
SEQ ID NO:58: Partial oligopeptide of JNK3 showing a high score in the local alignment between JNK3 and BMAL1.
SEQ ID NO:59: Partial oligopeptide of BMAL1 showing a high score in the local alignment between JNK3 and BMAL1.
SEQ ID NO:60: Partial oligopeptide of JNK3 showing a high score in the local alignment between JNK3 and BMAL1.
SEQ ID NO:61: Partial oligopeptide of BMAL1 showing a high score in the local alignment between JNK3 and BMAL1.
SEQ ID NO:62: Partial oligopeptide of JNK3 showing a high score in the local alignment between JNK3 and BMAL1.
SEQ ID NO:63: Partial oligopeptide of BMAL1 showing a high score in the local alignment between JNK3 and BMAL1.
SEQ ID NO:64: Partial oligopeptide of JNK3 showing a high score in the local alignment between JNK3 and BMAL2.
SEQ ID NO:65: Partial oligopeptide of BMAL2 showing a high score in the local alignment between JNK3 and BMAL2.
SEQ ID NO:66: Partial oligopeptide of JNK3 showing a high score in the local alignment between JNK3 and BMAL2.
SEQ ID NO:67: Partial oligopeptide of BMAL2 showing a high score in the local alignment between JNK3 and BMAL2.
SEQ ID NO:68: Partial oligopeptide of JNK3 showing a high score in the local alignment between JNK3 and BMAL2.
SEQ ID NO:69: Partial oligopeptide of BMAL2 showing a high score in the local alignment between JNK3 and BMAL2.
SEQ ID NO:70: Partial oligopeptide of JNK3 showing a high score in the local alignment between JNK3 and BMAL2.
SEQ ID NO:71: Partial oligopeptide of BMAL2 showing a high score in the local alignment between JNK3 and BMAL2.
SEQ ID NO:72: Partial oligopeptide of JNK3 showing a high score in the local alignment between JNK3 and BPL1.
SEQ ID NO:73: Partial oligopeptide of BPL1 showing a high score in the local alignment between JNK3 and BPL1.
SEQ ID NO:74: Partial oligopeptide of JNK3 showing a high score in the local alignment between JNK3 and BPL1.
SEQ ID NO:75: Partial oligopeptide of BPL1 showing a high score in the local alignment between JNK3 and BPL1.
SEQ ID NO:76: Partial oligopeptide of JNK3 showing a high score in the local alignment between JNK3 and BPL1
SEQ ID NO:77: Partial oligopeptide of BPL1 showing a high score in the local alignment between JNK3 and BPL1.
SEQ ID NO:78: Partial oligopeptide of JNK3 showing a high score in the local alignment between JNK3 and BPL1.
SEQ ID NO:79: Partial oligopeptide of BPL1 showing a high score in the local alignment between JNK3 and BPL1.
SEQ ID NO:80: Partial sequence which is common in sequence between JNK3 and BPL1.
SEQ ID NO:81: Partial oligopeptide of JNK3 showing a high score in the local alignment between JNK3 and BPL1.
SEQ ID NO:82: Partial oligopeptide of BPL1 showing a high score in the local alignment between JNK3 and BPL1.
SEQ ID NO:83: Partial sequence which is common in sequence between JNK3 and BPL1.
SEQ ID NO:84: Partial oligopeptide of JNK3 showing a high score in the local alignment between JNK3 and BPL1.
SEQ ID NO:85: Partial oligopeptide of BPL1 showing a high score in the local alignment between JNK3 and BPL1.
SEQ ID NO:86: Partial sequence which is common in sequence between JNK3 and BPL1.
SEQ ID NO:87: Partial oligopeptide of JNK3 showing a high score in the local alignment between JNK3 and BPL1.
SEQ ID NO:88: Partial oligopeptide of BPL1 showing a high score in the local alignment between JNK3 and BPL1.
SEQ ID NO:89: Partial oligopeptide of JNK3 showing a high score in the local alignment between JNK3 and BPL1.
SEQ ID NO:90: Partial oligopeptide of BPL1 showing a high score in the local alignment between JNK3 and BPL1.

## Claims

1. An agent for inhibiting phosphorylation of c-Jun, wherein the agent comprises one or more peptides selected from the following peptide group having a function for interacting with c-Jun N-terminal kinase 3 as active ingredients:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

2. The agent for inhibiting phosphorylation of c-Jun according to Claim 1, wherein the peptide group consists of (i), (ii), (iii), (iv), (x), and (xi).

3. A method for inhibiting the phosphorylation of c-Jun, wherein the method comprises putting one or more peptides selected from the following peptide group having a function for interacting with c-Jun N-terminal kinase 3 (JNK3) in coexistence with JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

4. The method for inhibiting the phosphorylation of c-Jun according to Claim 3, wherein the peptide group consists of (i), (ii), (iii), (iv), (x), or (xi).

5. An agent for inhibiting the ability of c-Jun to activate transcription, wherein the agent comprises one or more peptides selected from the following peptide group having a function for interacting with c-Jun N-terminal kinase 3 as the active ingredients:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of Lhe amino acid sequence represented by SEQ ID NO: 4 in Sequence List, or
(xi) a peptide comprised of the amino acid sequence represented by SEQ ID NO: 5 in Sequence List.

6. A method for inhibiting the ability of c-Jun to activate transcription, wherein the method comprises putting one or more peptides selected from the following peptide group having a function for interacting with c-Jun N-terminal kinase 3 in coexistence with JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

7. A pharmaceutical composition comprising an effective dose of the agent for inhibiting the phosphorylation of c-Jun according to Claim 1 or 2, or the agent for inhibiting the ability of c-Jun to activate transcription of c-Jun according to Claim 5.

8. The pharmaceutical composition according to Claim 7, wherein the pharmaceutical composition is an agent for preventing and/or treating disease(s) caused by the phosphorylation of c-Jun by c-Jun N-terminal kinase 3.

9. A pharmaceutical composition comprising an effective dose of one or more peptides selected from the following peptide group having a function for interacting with c-Jun N-terminal kinase 3, wherein the pharmaceutical composition is an agent for preventing and/or treating disease(s) caused by the phosphorylation of c-Jun by JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

10. A pharmaceutical composition comprising an effective dose of one or more polynucleotides encoding at least one peptide selected from the following peptide group having a function for interacting with c-Jun N-terminal kinase 3 (JNK3), wherein the pharmaceutical composition is an agent for preventing and/or treating disease(s) caused by the phosphorylation of c-Jun by JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

11. The pharmaceutical composition according to Claim 8, wherein the disease caused by the phosphorylation of c-Jun by c-Jun N-terminal kinase 3 is a neurodegenerative disease.

12. The pharmaceutical composition according to Claim 11, wherein the neurodegenerative disease is polyglutamine disease, Huntington's disease, spinocerebellar ataxia, bulbospinal muscular atrophy, dentatorubropallidoluysian atrophy, Alzheimer's disease, Down's disease, Parkinson's disease, Lewy body dementia, multiple system atrophy, familial amyotrophic lateral sclerosis, progressive supranuclear palsy, corticobasal degeneration, Pick's disease, familial British dementia, Creutzfeldt-Jakob disease, Gerstmann-Stranssler syndrome, mad cow disease (bovine spongiform encephalopathy) (BSE), or familial dementia accompanying neuroserpin inclusion bodies.

13. A method for preventing and/or treating disease(s) caused by the phosphorylation of c-Jun by JNK3, wherein the method comprises putting one or more peptides selected from the following peptide group having a function for interacting with c-Jun N-terminal kinase 3 (JNK3) in coexistence with JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

14. A method for preventing and/or treating disease(s) caused by the phosphorylation of c-Jun by JNK3, wherein the method comprises utilizing one or more polynucleotides encoding at least one peptide selected from the following peptide group having a function for interacting with c-Jun N-terminal kinase 3 (JNK3) to express the peptide encoded by the polynucleotides, wherein the peptide inhibits the phosphorylation of c-Jun by JNK3:
(i) BMAL1,
(ii) BPL1,
(iii) a peptide comprised of the amino acid sequence represented by SEQ ID NO: 1 in Sequence List,
(iv) a peptide comprised of the amino acid sequence represented by SEQ ID NO: 2 in Sequence List,
(v) a peptide comprised of the amino acid sequence represented by SEQ ID NO: 3 in Sequence List,
(vi) a peptide comprising at least one peptide of said (i) to (v), wherein the peptide has a function for inhibiting the phosphorylation of c-Jun by JNK3,
(vii) a peptide comprised of at least 5 consecutive amino acid residues in an amino acid sequence of at least one peptide of said (i) to (v), wherein the peptide has a function for inhibiting the phosphorylation of c-Jun by JNK3,
(viii) a peptide having mutations of one to several amino acid residues in an amino acid sequence of at least one peptide of said (i) to (v), wherein the peptide has a function for inhibiting the phosphorylation of c-Jun by JNK3,
(ix) a peptide having a homology of 70% or more to at least one peptide of said (i) to (v), wherein the peptide has a function for inhibiting the phosphorylation of c-Jun by JNK3,
(x) a peptide comprised of the amino acid sequence represented by SEQ ID NO: 4 in Sequence List, or
(xi) a peptide comprised of the amino acid sequence represented by SEQ ID NO: 5 in Sequence List.

15. A method for preventing and/or treating disease(s) caused by the phosphorylation of c-Jun by JNK3, wherein the method comprises using the pharmaceutical composition according to at least one of Claims 7 to 12.

16. The method for preventing and/or treating disease(s) according to at least one of Claims 13 to 15, wherein the disease caused by the phosphorylation of c-Jun by JNK3 is a neurodegenerative disease.

17. The method for preventing and/or treating disease(s) according to Claim 16, wherein the neurodegenerative disease is polyglutamine disease, Huntington's disease, spinocerebellar ataxia, bulbospinal muscular atrophy, dentatorubropallidoluysian atrophy, Alzheimer's disease, Down's disease, Parkinson's disease, Lewy body dementia, multiple system atrophy, familial amyotrophic lateral sclerosis, progressive supranuclear palsy, corticobasal degeneration, Pick's disease, familial British dementia, Creutzfeldt-Jakob disease, Gerstmann-Stranssler syndrome, mad cow disease (bovine spongiform encephalopathy) (BSE), or familial dementia accompanying neuroserpin inclusion bodies.

18. At least one peptide selected from the following peptide group:
(i) a peptide comprised of the amino acid sequence represented by SEQ ID NO: 1 in Sequence List,
(ii) a peptide comprising the peptide comprised of the amino acid sequence represented by SEQ ID NO: 1 in Sequence List,
(iii) a peptide comprised of at least 5 consecutive amino acid residues in the amino acid sequence represented by SEQ ID NO: 1 in Sequence List, or
(iv) a peptide having mutations of one to several amino acids in at least one peptide of (i) to (iii).

19. The peptide according to Claim 18, wherein the peptide has a function for interacting with c-Jun N-terminal kinase 3 (JNK3).

20. A polynucleotide comprising a nucleotide sequence encoding a peptide according to Claim 18 or 19, or a complementary sequence thereof.

21. A polynucleotide comprised of a nucleotide represented by SEQ ID NO: 7 in Sequence List.

22. A polynucleotide which hybridizes with a polynucleotide according to Claim 20 or 21 under a stringent conditions.

23. A recombinant vector comprising a polynucleotide according to at least one of Claims 20 to 22.

24. The recombinant vector according to Claim 23, wherein the recombinant vector is an expression recombinant vector.

25. A transformant transfected with a recombinant vector according to Claim 23 or 24.

26. A method for producing a peptide according to Claim 18 or 19, comprising a process for culturing a transformant transfected with a recombinant vector according to Claim 24.

27. An antibody which immunologically recognizes a peptide according to Claim 18 or 19.

28. A method of identifying a compound that mediates or inhibits the interaction of a peptide according to Claim 19 with c-Jun N-terminal kinase 3, wherein the method comprises using at least one selected from the peptide, a polynucleotide encoding the peptide, a recombinant vector comprising the polynucleotide, a transformant transfected with the recombinant vector, or an antibody which immunologically recognizes the peptide.

29. A method of identifying a compound that mediates or inhibits the expression of a polynucleotide encoding a peptide according to Claim 19, wherein the method comprises using at least one selected from the polynucleotide encoding the peptide, a recombinant vector comprising the polynucleotide, a transformant transfected with the recombinant vector, or an antibody which immunologically recognizes the peptide.

30. A pharmaceutical composition comprising an effective dose of at least one selected from a peptide according to Claim 19, a polynucleotide encoding the peptide, a recombinant vector comprising the polynucleotide, a transformant transfected with the recombinant vector, or an antibody which immunologically recognizes the peptide.
